(19) Europäisches Patentamt / European Patent Office / Office européen des brevets

(11) **EP 1 793 230 B1**

(12) **FASCICULE DE BREVET EUROPEEN**

(45) Date de publication et mention de la délivrance du brevet:
**12.08.2009 Bulletin 2009/33**

(51) Int Cl.:
*G01N 33/68* *(2006.01)*       *G06F 19/00* *(2006.01)*
*G01N 33/84* *(2006.01)*       *G01N 33/92* *(2006.01)*

(21) Numéro de dépôt: **06301189.4**

(22) Date de dépôt: **29.11.2006**

(54) **Procédé de détermination d'un score représentatif du stress oxydatif d'un patient, sur une échelle de 0 à 10**

Verfahren zur Bestimmung eines repräsentativen Scores des oxidativen Stresses eines Patienten auf einer Skala von 0 bis 10

Process for determining a score representing a patient's oxidative stress on a scale from 0 to 10

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **30.11.2005 FR 0512131**

(43) Date de publication de la demande:
**06.06.2007 Bulletin 2007/23**

(73) Titulaire: **Swiss Line Services Ltd.**
**Central Hong Kong**
**Hong Kong (CN)**

(72) Inventeurs:
• **Brack, Michel**
**92700 Colombes (FR)**
• **Brack, Olivier**
**60110 Esches (FR)**
• **Dreyfus, Gérard**
**91190 Gif sur Yvette (FR)**
• **Chapman, John**
**94100 Saint Maur (FR)**
• **Kontush, Anatol**
**75013 Paris (FR)**
• **Bonnefont-Rousselot, Dominique**
**75006 Paris (FR)**

(74) Mandataire: **Micheli & Cie SA**
**122, rue de Genève**
**CP 61**
**1226 Thonex-Genève (CH)**

(56) Documents cités:
WO-A-03/016527       WO-A-20/05020984
WO-A-20/05086827

• **BERGMAN B ET AL: "Clinical usefulness of serum assays of neuron-specific enolase, carcinoembryonic antigen and CA-50 antigen in the diagnosis of lung cancer" EUROPEAN JOURNAL OF CANCER PART A: GENERAL TOPICS, vol. 29, no. 2, 1993, pages 198-202, XP002388140**
• **NILSSEN O ET AL: "The 'WAM' score: Sensitivity and specificity of a user friendly biological screening test for alcohol problems in trauma patients" ADDICTION, vol. 91, no. 2, 1996, pages 255-262, XP008066022**
• **L'ITALIEN G J ET AL: "Development and validation of a Bayesian model for perioperative cardiac risk assessment in a cohort of 1,081 vascular surgical candidates" JOURNAL OF THE AMERICAN COLLEGE OF CARDIOLOGY, vol. 27, no. 4, 1996, pages 779-786, XP002388141**

**Description**

WO2005/020984 décrit un procédé de détermination d'un score représentatif du stress oxydatif d'un patient où

- on choisit une série de marqueurs biologiques liés au stress oxydatif et on évalue ces marqueurs par dosage et par comparaison avec une fenêtre pondérée obtenue d'une base de données d'un panel de personnes saines, et
- on calcule avec les valeurs obtenues un score représentatif du stress oxydatif.

La présente invention a pour objet un procédé de détermination d'un score représentatif du stress oxydatif d'un patient sur une échelle de 0 à 10.

Il est connu que l'oxygène est le carburant des cellules, et il existe dans la littérature de nombreuses publications à ce sujet.

Toutefois, certaines formes d'oxygène, notamment les radicaux libres, peuvent être fortement toxiques pour l'organisme et les cellules dans la mesure où elles catalysent des réactions d'oxydation de nature à altérer au hasard les molécules biologiques.

Il s'agit là du phénomène connu des spécialistes sous le nom de stress oxydatif.

Ce phénomène est impliqué dans une large gamme de pathologies, en particulier les hépatites, le cancer, le sida, la maladie d'Alzheimer, la maladie de Parkinson... et correspond en outre au moteur essentiel du vieillissement.

Or, le corps médical dispose aujourd'hui de moyens permettant d'intervenir sur le stress oxydatif tels qu'à titre d'exemple une amélioration de l'hygiène de vie, une prise en charge nutritionnelle (suivant les recommandations du Programme National Nutrition Santé) ou encore l'administration d'antioxydants naturels ou de synthèse.

Il serait donc souhaitable de pouvoir évaluer le stress oxydatif dans chaque cas particulier afin de prédire un risque de développement d'une pathologie et de prendre précocement, dès la détection d'une anomalie, toutes les mesures possibles pour y remédier.

L'évaluation du stress oxydatif en médecine humaine ne correspond toutefois pas à un problème aujourd'hui résolu, ce même si différents laboratoires proposent d'explorer ce stress à partir des bilans essentiellement sanguins basés sur le dosage de certains marqueurs biologiques.

Il s'agit toutefois là plus de démarches empiriques que de procédés d'évaluation réellement scientifiques, ce d'autant plus que les marqueurs utilisés varient largement d'un laboratoire à un autre.

En conséquence ces bilans ne sont pas de nature à permettre une prise en charge du stress oxydatif en médecine courante et leur interprétation qui est particulièrement complexe est l'apanage de quelques spécialistes rodés au stress oxydatif depuis plusieurs années.

La présente invention a pour objet de combler cette lacune en proposant au corps médical un outil scientifique permettant d'obtenir à partir de bilans sanguins et de dosages biologiques une évaluation du stress oxydatif d'un patient matérialisée par un score représentatif de ce stress, sur une échelle de 0 à 10 (du bilan le moins perturbé au bilan le plus perturbé).

Un tel score est donc indicatif d'un bilan normal ou plus ou moins anormal.

En présence d'un bilan anormal, le médecin doit ensuite faire un travail d'interprétation pour évaluer l'origine de cette anomalie et prescrire au patient les mesures nécessaires pour y remédier.

La pertinence de ces mesures (conseils sur l'hygiène de vie, modification du régime alimentaire, en particulier par augmentation de l'apport en fruits et légumes ou encore supplémentation antioxydante) peut être contrôlée après quelques mois grâce à une nouvelle détermination du score représentatif du stress oxydatif.

Dans ce contexte, l'invention a donc pour objet un procédé de détermination d'un score représentatif du stress oxydatif d'un patient sur une échelle de 0 à 10,

caractérisé par la succession des étapes suivantes :

- dans une étape d'étalonnage initial :

- on choisit une série de marqueurs biologiques liés au stress oxydatif et on évalue ces marqueurs biologiques par dosage, en particulier à partir de prélèvements sanguins, chez un panel de personnes saines ou présentant éventuellement différents types de pathologies et troubles cliniques connus de façon à obtenir une base de données, et
- on effectue une analyse statistique de cette base de données en s'appuyant sur des relations connues dans le domaine médical et décrites dans la littérature de façon:

- à établir pour chacun des marqueurs biologiques une échelle de gravité associée aux écarts constatés par rapport à des valeurs de référence,
- à rassembler les marqueurs biologiques en un ensemble de groupes de marqueurs biologiques exprimant respectivement des

- informations différentes pouvant être associées à des stades différents de développement du stress oxydatif, et
- à attribuer un poids d'une part à chaque groupe de marqueurs biologiques, et d'autre part à chacun des marqueurs biologiques au sein de chaque groupe, puis
- on évalue plusieurs marqueurs biologiques du patient appartenant à plusieurs groupes de marqueurs biologiques,
- on attribue à chacune des valeurs ainsi obtenues une note sur l'échelle de gravité, et
- on calcule un score de stress oxydatif Spatient spécifique au patient en utilisant la formule

$$Spatient = 10 x \frac{\sum_{groupe1}^{nb.groupe} Poids_{groupe} \; x \left( \sum_{marqueur1}^{nb.marqueurs} \left( Poids_{marqueur} x \; note_{patient} \right) \right)}{\sum_{groupe1}^{nb.groupes} Poids_{groupe} \; x \left( \sum_{marqueur4}^{nb.marqueurs} \left( Poids_{marqueur} \; x \; Max_{marqueur} \right) \right)}$$

dans laquelle $Max_{marqueur}$ représente la note maximum du marqueur biologique dans l'échelle de gravité.

Le procédé conforme à l'invention a pu être conçu à l'origine, grâce à un travail pluridisciplinaire, médical et mathématique, s'étant prolongé sur une période d'environ quatre ans.

On a ainsi effectué environ 600 « premiers » bilans sanguins de stress oxydatif sur des patients sains ou présentant différents types de pathologies et troubles cliniques, en effectuant à chaque fois le dosage de dix à vingt marqueurs biologiques.

Dans un second temps on a effectué environ 120 bilans de contrôle sur des patients ayant été traités suite à un premier bilan s'étant révélé anormal.

Ce travail a permis d'obtenir une base de données très complète qui figure en annexe 1 à titre d'exemple.

Les significations des abréviations des différents marqueurs biologiques, leurs unités et les normes sont précisées en annexe 2.

Cette base de données a été complétée par des informations se rapportant aux caractéristiques descriptives de la population suivie : sexe, âge, habitat, activité professionnelle... et à l'anamnèse du dossier médical des patients : état de santé, antécédents personnels médicaux, et chirurgicaux, antécédents de la fratrie directe, maladie évolutive, traitements, niveau de stress....

Cette base de données a été examinée par une équipe médicale pour identifier et éliminer d'éventuelles valeurs aberrantes puis soumise à une analyse statistique par une technique de modélisation de façon à pondérer les valeurs des différents marqueurs biologiques en fonction de paramètres tels que l'âge du patient et à valider celle-ci en s'appuyant sur la vérification de relations connues et évoquées dans la littérature médicale.

On a ainsi vu apparaître des corrélations entre les différents marqueurs biologiques d'une part et entre certaines anomalies de ces marqueurs biologiques et certains éléments du dossier personnel et médical des patients d'autre part.

Il est apparu que l'ensemble des marqueurs biologiques dosés ne permettait pas de reconnaître telle ou telle maladie mais que leurs anomalies renseignaient parfaitement sur l'état de santé globale des patients.

Ainsi, deux groupes remarquables par leur phénotypes extrêmes (parfaite santé et très mauvaise santé) ont été individualisés et étudiés quant à leur absence ou haut niveau de stress oxydatif.

L'étude des informations obtenues a conduit :

- à associer à chaque marqueur biologique une échelle de gravité en fonction des résultats obtenus sur l'ensemble des bilans,
- à réunir les marqueurs biologiques testés en groupes de marqueurs biologiques exprimant des informations différentes, certains exprimant plus l'état de santé des patients et d'autres étant plus liés à un risque élevé de développer telle ou telle pathologie, et
- à évaluer une grille de pondération des différents groupes de marqueurs biologiques et des différents marqueurs biologiques au sein de chaque groupe.

On a finalement conçu une échelle permettant par des algorithmes mathématiques qui tiennent compte de l'ensemble des éléments susmentionnés de calculer pour chaque patient un score de 0 à 10 représentatif de son propre niveau de stress oxydatif.

Cette échelle a permis de retrouver sur l'ensemble de la base de données les patients en parfaite santé et ceux présentant un mauvais état de santé.

Il est à noter que d'un point de vue purement statistique ces travaux ont fait appel aux techniques et méthodes suivantes:

Statistique descriptive : Test d'adéquation graphiques et numériques à des lois statistiques - Détermination d'estimateurs de niveau moyen et de dispersion,

Tests graphiques et numériques de détection de valeurs aberrantes,

Analyses d'écarts par étude des différences appariées et tests associés (paramétriques et non paramétriques),

Analyses de corrélation et modélisation en Régression linéaire simple,

Analyse en Composantes Principales,

Modélisation en Régression PLS (Partial Least Squares & Discriminant

Analysis) avec validation croisée selon le principe des blocs dits « splités ».

L'évaluation de l'échelle est actuellement réalisable de façon interactive grâce à un utilitaire développé sous Microsoft® Excel dans sa version 2003 (Professionnelle) sous la forme d'une macro-commande acceptant les bilans incomplets du fait d'une pondération ajustée en fonction des dosages dont on dispose.

Le logiciel de traitement statistique utilisé est le logiciel Jmp dans sa version 5.1.1 (A BUSINESS UNIT OF SAS - Copyright © 1989 - 2004 SAS Institute Inc).

Pour valider l'échelle ainsi conçue, on a confié un fichier aléatoire de 30 patients à trois observateurs indépendants A, B et C experts dans le domaine du stress oxydatif.

Il leur a été demandé de noter le bilan de chacun de ces patients de 0 à 10 en fonction de la gravité du stress oxydatif qu'ils imaginaient spontanément ce, sans qu'ils aient accès au dossier personnel et médical.

L'annexe 3 reproduit les diagnostics de chacun des experts, leur consensus, c'est-à-dire la moyenne des notes accordées par ces experts, le score Spatient obtenu suite à la mise en oeuvre du procédé conforme à l'invention ainsi que l'écart entre ce consensus et ce score.

Ces résultats prouvent que les trois experts ont fait des évaluations globalement équivalentes et superposables à la cotation obtenue par le procédé conforme à l'invention.

Selon une autre caractéristique de l'invention, on rassemble les marqueurs biologiques en au moins trois groupes de marqueurs biologiques d'importance croissante correspondant respectivement à des antioxydants exogènes, c'est-à-dire apportés par l'extérieur, à des antioxydants endogènes, c'est-à-dire liés à la synthèse cellulaire et à des marqueurs de dégâts oxydatifs.

Les deux premiers groupes de marqueurs biologiques renseignent sur l'état de santé des patients alors que le troisième groupe renseigne sur les risques qu'ils ont de développer certaines pathologies.

Selon l'invention, on peut avantageusement attribuer au premier groupe de marqueurs biologiques correspondant à des antioxydants exogènes un poids égal à 1, au second groupe de marqueurs biologiques correspondant à des antioxydants endogènes un poids égal à 5 et au troisième groupe de marqueurs biologiques correspondant à des marqueurs de dégâts biologiques un poids égal à 10.

Le premier groupe de marqueurs biologiques renferme de préférence au moins sept marqueurs biologiques constitués par la vitamine C, la vitamine E, le cuivre, le zinc, le sélénium, et les rapports vitamine C/vitamine E et cuivre/zinc.

Ce premier groupe peut bien entendu également renfermer d'autres marqueurs parmi lesquels on peut à titre d'exemple mentionner l'alpha tocophérol (ou vitamine E), le gamma tocophérol, le cholestérol, le rapport vitamine E/cholestérol ou encore le bêta carotène.

Le second groupe de marqueurs biologiques renferme quant à lui de préférence au moins quatre marqueurs biologiques constitués par le glutathion total GSH, le glutathion oxydé GSSG, les protéines thiols et le rapport GSH/GSSG.

Ce second groupe peut lui aussi renfermer d'autres marqueurs biologiques tels qu'à titre d'exemple la glutathion peroxydase ou encore l'acide urique.

Enfin, le troisième groupe de marqueurs biologiques renferme au moins un marqueur biologique choisi parmi les lipides basse densité oxydés (LDL Oxyd.) et le rapport 8 hydroxy 2guanosine/créatinine, ou ADN oxydé/créatinine.

Ce troisième groupe peut également renfermer lui aussi d'autres marqueurs biologiques tels qu'à titre d'exemple les peroxydes lipidiques les anticorps contre les LDL Oxyd. ou l'ADN oxydé.

Il est à noter que dans le cadre de l'invention on a obtenu des résultats globalement satisfaisants en attribuant dans chaque groupe un même poids, en particulier égal à 1 à chacun des marqueurs biologiques principaux susmentionnés.

Il est par ailleurs à noter que la note d'un marqueur biologique sur l'échelle de gravité peut selon les marqueurs varier entre de 0 à 2 et de 0 à 10.

Le procédé conforme à l'invention permet donc de fournir au médecin un score compris entre 0 et 10 donnant une indication sur l'état de stress oxydatif d'un patient, (stress oxydatif modéré ou important).

Celui-ci peut ainsi, selon que ce score doive être ou non considéré comme normal, déterminer si le patient est en bonne ou en mauvaise santé.

Dans le cas d'un stress oxydatif anormal (modéré ou important) le procédé permet d'effectuer une interprétation qualitative du score obtenu en recherchant quel est dans ce score le poids relatif des différents groupes de marqueurs biologiques.

Cette interprétation permet au médecin de déterminer si le stress oxydatif anormal qui a été révélé par le score du patient est essentiellement lié, dans l'ordre d'importance :

- à une carence relative en antioxydants exogènes : vitamine C et/ou vitamine E et/ou cuivre et/ ou zinc et/ou sélénium...,
- à une carence relative en antioxydants endogènes : glutathion, protéines thiols...,
- ou à l'objectivation de dégâts oxydatifs déjà présents : LDL Oxyd., ADN oxydé...,

A partir de cette constatation, le médecin peut décider de compléter ce bilan par des dosages auxiliaires, vérifier les apports alimentaires en fruits et légumes et/ou prescrire au patient un traitement des carences avérées grâce en particulier à l'administration de composés antioxydants.

Cette prescription sera bien entendu accompagnée d'un contrôle du bilan au bout de quelques mois, après une prise en charge adaptée.

ANNEXE 1

| Patient | Sexe | N° bilan | Date | Naissance | Vit C | Vit E | Alpha tocoph | Vit E + Alpha Toco | Gamma tocoph | Vit C/E | Chol. | Vit E/Chol | βcaro | GSH | GSSG | Ratio G/G | GPX | Prot Thiol | Cap Hydro | Cap Lipo | Acide Urique | Sel | Cu | Zn | Cu/Zn | Perox. Lipid | LDL Oxyd | Atcp/ LDL | 8ohdG/ creat | ADN OX |
|---|---|---|---|---|---|---|---|---|---|---|---|---|---|---|---|---|---|---|---|---|---|---|---|---|---|---|---|---|---|---|
| Patient n°1 | F | 1 | 12-avr-05 | 1920 | 8.60 | | 16.8 | 16.8 | 0.96 | 0.51 | 1.50 | 11.20 | 0.3 | 713.0 | 66.0 | 8.80 | | 278.0 | | 45.0 | | 94.0 | 1.53 | 0.56 | 2.73 | 1000.0 | | | | |
| Patient n°1 | F | 2 | 6-juin-05 | 1920 | 14.71 | | 16.8 | 16.8 | | 0.88 | 1.39 | 12.09 | 2.2 | 752.0 | 50.0 | 13.04 | | 297.0 | | 56.0 | | | | | | 1186.0 | | | | |
| Patient n°2 | F | 1 | 12-avr-05 | 1943 | 12.66 | | 24.4 | 24.4 | 0.61 | 0.52 | 2.07 | 11.79 | 0.3 | 745.0 | 47.0 | 13.85 | | | | 68.0 | | 123.0 | 0.93 | 0.76 | 1.22 | 340.0 | | | | |
| Patient n°3 | F | 1 | 12-mars-03 | 1952 | 17.07 | 18.0 | | 18.0 | | 0.95 | 2.30 | 7.83 | 34.4 | 1170.3 | 70.5 | 14.59 | 101.0 | 348.9 | 75.0 | 63.1 | 42.0 | 120.0 | 1.03 | 0.52 | 1.98 | | 46.7 | 2194.1 | 13.90 | 8.34 |
| Patient n°4 | F | 1 | 21-sept-05 | 1948 | 4.07 | | 10.9 | 10.9 | 0.56 | 0.37 | 2.12 | 5.14 | 0.3 | 917.0 | 134.0 | 4.84 | | 354.0 | | 65.0 | | | | | | 607.0 | | | | |
| Patient n°5 | F | 1 | 28-sept-04 | 1953 | 4.93 | 13.2 | | 13.2 | | 0.37 | 1.63 | 8.10 | 0.4 | 828.0 | 61.0 | 11.57 | | 450.0 | 470.0 | 20.7 | 36.0 | | | | | | | | 14.25 | 11.40 |
| Patient n°6 | F | 1 | 10-déc-03 | 1942 | 11.36 | 18.5 | | 18.5 | | 0.61 | 2.84 | 6.51 | 101.1 | 308.1 | 84.7 | 1.64 | 62.0 | 395.4 | 210.0 | 51.6 | 55.0 | 111.0 | 1.21 | 0.65 | 1.86 | | 144.0 | 57.3 | 12.08 | 10.87 |
| Patient n°6 | F | 2 | 31-mars-04 | 1942 | 12.32 | 20.3 | | 20.3 | | 0.61 | 3.03 | 6.70 | | 1083.3 | 82.5 | 11.14 | | 385.4 | 320.0 | 49.6 | 67.0 | | | | | | 205.6 | | | |
| Patient n°7 | F | 1 | 25-janv-05 | 1933 | 9.21 | 18.1 | | 18.1 | | 0.51 | 2.88 | 6.29 | 0.6 | 917.0 | 66.0 | 11.89 | | 361.0 | | 64.4 | 54.0 | | | | | 413.0 | | | | |
| Patient n°7 | F | 2 | 28-juin-05 | 1933 | 13.52 | | 13.5 | 13.5 | 0.98 | 1.00 | 2.45 | 5.51 | 1.2 | 931.0 | 155.0 | 4.01 | | 342.0 | | 63.0 | | | | | | 486.0 | | | | |
| Patient n°8 | M | 1 | 9-juin-04 | 1951 | 8.60 | 11.1 | | 11.1 | | 0.77 | 1.99 | 5.58 | 0.1 | 983.0 | 19.0 | 49.74 | | 369.0 | 420.0 | 26.2 | | | | | | | 74.3 | | | |
| Patient n°9 | F | 1 | 10-mai-04 | 1946 | 15.14 | 15.2 | | 15.2 | | 1.00 | 2.44 | 6.23 | | 788.6 | 113.5 | 4.95 | 57.0 | 374.0 | 205.0 | 49.6 | 42.0 | 128.0 | | | | | 142.1 | | | |
| Patient n°10 | M | 1 | 12-mai-03 | 1925 | 7.76 | 12.3 | | 12.3 | | 0.63 | 0.97 | 12.68 | 15.3 | 1126.7 | 3.4 | 330.37 | 64.0 | 379.1 | 225.0 | 55.1 | 35.0 | 95.0 | 1.06 | 0.65 | 1.63 | | 64.5 | 92.5 | 22.81 | 29.65 |
| Patient n°11 | F | 1 | 10-nov-04 | 1948 | 13.29 | 14.3 | | 14.3 | | 0.93 | 2.17 | 6.59 | 0.3 | 684.0 | 6.0 | 112.00 | | 346.0 | 575.0 | 59.0 | 53.0 | | | | | 282.0 | | | | |
| Patient n°12 | F | 1 | 1-sept-04 | 1945 | 11.71 | 17.9 | | 17.9 | | 0.65 | 2.42 | 7.40 | 0.6 | 664.0 | 21.0 | 29.62 | | 331.0 | 300.0 | 48.0 | 44.0 | | | | | | 35.0 | | | |
| Patient n°13 | F | 2 | 9-mars-05 | 1945 | 12.31 | | 17.9 | 17.9 | 1.53 | 0.69 | 2.29 | 7.82 | 0.6 | 856.0 | 21.0 | 38.76 | | | | | | | | | | 271.0 | | | | |
| Patient n°14 | F | 1 | 28-sept-04 | 1941 | 10.53 | 14.4 | | 14.4 | | 0.73 | 1.67 | 8.62 | 1.2 | 837.0 | 17.0 | 47.24 | | 362.0 | 650.0 | | 32.0 | | | | | | 44.5 | | | |
| Patient n°15 | F | 1 | 5-mai-04 | 1950 | 13.34 | 15.0 | | 15.0 | | 0.89 | 1.92 | 7.81 | | 1028.4 | 52.1 | 17.74 | 42.0 | 402.4 | 250.0 | 26.0 | 51.0 | 183.0 | | | | | 170.2 | | | |
| Patient n°16 | F | 1 | 28-avr-04 | 1957 | 12.44 | 16.6 | | 16.6 | | 0.75 | 2.83 | 5.87 | | 1016.0 | 45.3 | 20.41 | | | 235.0 | 54.4 | 43.0 | | | | | | 41.5 | | | |
| Patient n°17 | F | 1 | 26-mai-04 | 1965 | 10.82 | 11.5 | | 11.5 | | 0.94 | 1.55 | 7.42 | | 1041.1 | 10.5 | 96.78 | 42.0 | 367.8 | 555.0 | 29.8 | 63.0 | 104.0 | | | | | 33.6 | | | |
| Patient n°18 | M | 1 | 27-avr-04 | 1955 | 11.12 | 13.9 | | 13.9 | | 0.80 | 1.51 | 9.21 | | 495.5 | 17.3 | 26.57 | | 451.5 | 620.0 | 50.0 | 53.0 | | | | | | 244.7 | | | |
| Patient n°19 | F | 1 | 9-sept-03 | 1965 | 8.92 | 16.7 | | 16.7 | | 0.53 | 1.80 | 9.28 | 3.3 | 1039.9 | 136.2 | 5.63 | 52.0 | 215.7 | | 59.1 | 39.0 | 117.0 | 1.24 | 0.69 | 1.80 | | 282.9 | 176.4 | 7.98 | 4.79 |
| Patient n°20 | F | 1 | 8-sept-04 | 1951 | 10.14 | 15.6 | | 15.6 | | 0.65 | 2.38 | 6.56 | 0.5 | 801.0 | 93.0 | 6.61 | | 320.0 | 235.0 | 65.3 | 39.0 | | | | | | 52.4 | | | |
| Patient n°21 | F | 1 | 2-nov-04 | 1950 | 4.80 | 19.0 | | 19.0 | | 0.25 | 2.96 | 6.42 | 0.6 | 965.0 | 60.0 | 14.08 | | 319.0 | 600.0 | 71.0 | 45.0 | | | | | | | | 6.00 | 10.00 |

EP 1 793 230 B1

| Patient | Sexe | N° | Date | Année | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|
| Patient n°22 | F | 1 | 16-nov-04 | 1950 | 9.12 | 13.8 | | 13.8 | | 0.66 | 2.11 | 6.54 | 0.5 | 821.0 | 3.0 | 271.67 | 82.0 | 351.0 | 1090.0 | 41.0 | 84.0 | 186.0 | 1.31 | 0.78 | 1.68 | 197.0 | 45.5 | 204.9 | 9.06 | 11.78 |
| Patient n°23 | F | 1 | 15-janv-04 | 1933 | 12.92 | 17.4 | | 17.4 | | 0.74 | 2.72 | 6.40 | 0.5 | 1078.2 | 80.7 | 11.37 | 45.0 | 328.8 | 155.0 | 54.2 | 45.0 | 129.0 | 1.09 | 0.70 | 1.56 | | 55.7 | 83.5 | 21.33 | 6.40 |
| Patient n°24 | F | 1 | 25-mai-04 | 1938 | 19.95 | 28.5 | | 28.5 | | 0.70 | 2.29 | 12.45 | 0.7 | 1016.5 | 53.3 | 17.06 | 45.0 | 338.8 | 220.0 | 89.6 | 45.0 | | | | | | | | | |
| Patient n°25 | F | 1 | 1-mars-05 | 1952 | 12.95 | | 17.3 | 17.3 | 1.97 | 0.75 | 2.77 | 6.24 | 0.7 | 666.0 | 33.0 | 18.18 | | | | 62.7 | | | | | | 219.0 | | | | |
| Patient n°26 | M | 1 | 21-févr-05 | 1942 | 11.92 | 21.1 | 21.1 | 21.1 | | 0.56 | 2.12 | 9.95 | 0.3 | 686.0 | 7.0 | 96.00 | | 385.0 | | 60.0 | 55.0 | | | | | 356.0 | | | | |
| Patient n°27 | F | 1 | 9-mars-05 | 1938 | 15.47 | 15.3 | 15.3 | 15.3 | | 1.01 | 2.07 | 7.39 | 1.1 | 774.0 | 30.0 | 23.80 | | | | 48.0 | | | | | | 722.0 | 141.2 | #### | 5.51 | 8.81 |
| Patient n°27 | F | 2 | 26-juil-05 | 1938 | 14.06 | 16.8 | 16.8 | 16.8 | | 0.84 | 1.71 | 9.82 | 2.6 | 1110.0 | 19.0 | 56.42 | 46.0 | 315.0 | | 83.0 | | | | | | 589.0 | | | | |
| Patient n°28 | M | 1 | 3-févr-03 | 1965 | 9.59 | 10.4 | | 10.4 | | 0.92 | 1.71 | 6.08 | 40.7 | 430.2 | 23.9 | 16.00 | | 350.3 | 0.0 | 56.7 | 65.0 | 114.0 | 1.13 | 0.54 | 2.09 | | | | | |
| Patient n°28 | M | 2 | 14-oct-03 | 1965 | 4.00 | 10.6 | | 10.6 | | 0.38 | 1.86 | 5.70 | | 979.9 | 157.8 | 4.21 | 45.0 | 401.5 | 170.0 | 24.2 | 63.0 | | 0.92 | 0.81 | 1.14 | | 83.8 | 572.2 | 6.29 | 8.81 |
| Patient n°29 | F | 1 | 9-sept-03 | 1932 | 12.74 | 13.9 | | 13.9 | | 0.92 | 2.03 | 6.85 | 8.7 | 1119.5 | 112.9 | 7.92 | | 382.3 | | 61.1 | 52.0 | 85.0 | | | | | 334.2 | | | |
| Patient n°29 | F | 2 | 17-févr-04 | 1932 | 14.40 | 14.7 | | 14.7 | | 0.98 | 1.38 | 10.65 | | 1309.0 | 44.5 | 27.40 | 75.0 | 357.6 | 425.0 | 42.2 | 41.0 | | | | | | 110.2 | | | |
| Patient n°29 | F | 3 | 16-févr-05 | 1932 | 16.76 | | 11.2 | 11.2 | 2.43 | 1.50 | 1.83 | 6.12 | 0.6 | 1031.0 | 71.0 | 12.52 | | | | 58.0 | | 116.0 | | | | 271.0 | | | | |
| Patient n°30 | M | 1 | 12-juil-05 | 1953 | 3.41 | | 9.7 | 9.7 | 1.11 | 0.35 | 2.06 | 4.71 | 0.6 | 890.0 | 82.0 | 8.85 | | 410.0 | | 52.0 | | | | | | 223.0 | | | | |
| Patient n°31 | F | 1 | 18-mai-04 | 1964 | 9.50 | 7.6 | | 7.6 | | 1.25 | 1.53 | 4.97 | | 852.0 | 88.4 | 7.64 | 82.0 | 326.5 | 225.0 | 12.7 | 27.0 | 100.0 | | | | | 48.0 | | 15.00 | 3.00 |
| Patient n°31 | F | 2 | 26-oct-04 | 1964 | 18.98 | 8.6 | | 8.6 | | 2.21 | 1.45 | 5.93 | 1.1 | 872.0 | 43.0 | 18.28 | 65.0 | 361.0 | 295.0 | 34.0 | 30.0 | | | | | | | | | |
| Patient n°32 | M | 1 | 12-mars-03 | 1957 | 13.67 | 17.4 | | 17.4 | | 0.79 | 2.13 | 8.17 | 13.1 | 1097.1 | 138.3 | 5.94 | | 349.3 | 225.0 | 68.0 | 70.0 | 97.0 | 1.08 | 0.82 | 1.32 | | 108.5 | 604.0 | 12.40 | 23.56 |
| Patient n°33 | F | 1 | 3-févr-03 | 1961 | 12.62 | 17.2 | | 17.2 | | 0.73 | 2.25 | 7.64 | 71.6 | 994.7 | 1.1 | 919.02 | 46.0 | 408.8 | 130.0 | 66.0 | 39.0 | 98.0 | 1.07 | 0.73 | 1.47 | 488.1 | 66.0 | 592.6 | 10.27 | 16.43 |
| Patient n°34 | F | 1 | 14-avr-04 | 1961 | 9.66 | 12.0 | | 12.0 | | 0.80 | 2.08 | 5.86 | | 827.6 | 31.5 | 24.30 | 36.0 | 401.0 | 145.0 | 28.2 | 45.0 | | | | | | 106.8 | | | |
| Patient n°35 | M | 1 | 5-mai-04 | 1952 | 13.37 | 14.4 | | 14.4 | | 0.93 | 1.97 | 7.31 | | 1071.6 | 48.8 | 19.96 | 62.0 | 362.5 | 275.0 | 48.2 | 51.0 | 192.0 | 0.91 | 0.76 | 1.20 | | 143.0 | 80.0 | 14.74 | 16.21 |
| Patient n°36 | F | 1 | 21-juin-04 | 1929 | 15.09 | | 15.0 | 15.0 | | 1.01 | | 0.60 | | 815.0 | 52.0 | 13.67 | 37.0 | 382.0 | 355.0 | 13.6 | 59.0 | 117.0 | 1.01 | 0.67 | 1.51 | | 75.4 | 485.0 | 2.50 | 0.50 |
| Patient n°37 | F | 1 | 22-oct-03 | 1963 | 22.52 | 13.8 | | 13.8 | | 1.63 | 2.12 | 6.51 | 32.2 | 1061.6 | 139.2 | 5.63 | | 307.1 | 50.0 | 45.8 | 34.0 | 89.0 | 0.68 | 0.72 | 0.94 | | 349.0 | 97.0 | 6.00 | 5.60 |
| Patient n°38 | M | 1 | 12-juil-05 | 1957 | 13.48 | | 20.3 | 20.3 | 1.42 | 0.66 | 1.94 | 10.46 | 0.8 | 687.0 | 51.0 | 11.47 | 37.0 | 492.0 | | 76.0 | 51.0 | 147.0 | | | | 96.0 | 52.0 | | | |
| Patient n°39 | F | 1 | 7-avr-04 | 1954 | 7.97 | 13.4 | | 13.4 | | 0.59 | 2.10 | 6.38 | | 766.3 | 20.1 | 36.07 | | 259.1 | 15.0 | 36.4 | 29.0 | | | | | | 80.2 | | | |
| Patient n°40 | F | 2 | 9-nov-04 | 1954 | 10.82 | 13.0 | | 13.0 | | 0.83 | 1.74 | | 1.7 | 754.0 | 20.0 | 35.70 | | 359.0 | 410.0 | 52.0 | 29.0 | | | | | | | | | |
| Patient n°41 | F | 1 | 7-sept-04 | 1970 | 14.00 | 11.1 | | 11.1 | | 1.26 | 1.57 | 7.07 | 0.8 | 909.0 | 56.0 | 14.23 | | 288.0 | 330.0 | 44.0 | 45.0 | 145.0 | 1.16 | 0.72 | 1.61 | | 30.9 | | 5.00 | 2.00 |
| Patient n°41 | F | 1 | 4-mai-04 | 1951 | 10.34 | 12.5 | | 12.5 | | 0.83 | 1.94 | 6.44 | | 1196.5 | 96.4 | 10.41 | | 237.9 | 80.0 | 38.7 | 30.0 | 158.0 | | | | | 88.8 | | | |
| Patient n°42 | M | 1 | 17-nov-04 | 1933 | 6.81 | 14.1 | | 14.1 | | 0.48 | 1.86 | 7.58 | 0.4 | 748.0 | 2.0 | 372.00 | | 262.0 | | 42.0 | 77.0 | | | | | 646.0 | | | | |

7

| Patient | Sexe | N° | Date | Année | V1 | V2 | V3 | V4 | V5 | V6 | V7 | V8 | V9 | V10 | V11 | V12 | V13 | V14 | V15 | V16 | V17 | V18 | V19 | V20 | V21 | V22 | V23 | V24 | V25 |
|---|---|---|---|---|---|---|---|---|---|---|---|---|---|---|---|---|---|---|---|---|---|---|---|---|---|---|---|---|---|
| Patient n°43 | F | 1 | 21-sept-04 | 1938 | 15.08 | 14.0 | | 14.0 | 1.08 | 1.62 | 8.64 | 1.5 | 1326.0 | 56.0 | 21.68 | 53.0 | 295.0 | 400.0 | 60.0 | 37.0 | 198.0 | 1.08 | 0.91 | 1.19 | | 38.8 | 187.0 | 13.00 | 6.58 |
| Patient n°43 | F | 2 | 1-mars-05 | 1938 | 10.76 | 20.6 | 20.6 | 20.6 | 0.52 | 2.23 | | 3.1 | 873.0 | 61.0 | 12.31 | | | | 57.0 | | | | | | 436.0 | | | | |
| Patient n°44 | M | 1 | 21-sept-04 | 1939 | 8.08 | 25.6 | | 25.6 | 0.32 | 2.38 | 10.76 | 2.2 | 918.0 | 31.0 | 27.61 | 63.0 | 327.0 | 507.5 | 90.6 | 40.0 | ### | 1.30 | 0.77 | 1.69 | | 38.6 | 93.0 | 8.80 | 5.30 |
| Patient n°44 | M | 2 | 1-mars-05 | 1939 | 7.96 | 14.4 | 14.4 | 14.4 | 0.55 | 2.03 | | 1.7 | 831.0 | 31.0 | 24.81 | | | | 52.0 | | | | | | 251.0 | | | | |
| Patient n°45 | F | 1 | 23-sept-03 | 1977 | 5.28 | 17.5 | | 17.5 | 0.30 | 1.80 | 9.72 | 19.4 | 991.8 | 26.7 | 35.10 | 63.0 | 319.3 | 20.0 | 52.9 | 49.0 | 84.0 | 1.48 | 0.55 | 2.69 | | 67.8 | 1281.9 | 15.31 | 29.09 |
| Patient n°46 | F | 1 | 7-sept-04 | 1949 | 10.20 | 14.9 | | 14.9 | 0.68 | 2.08 | 7.16 | 2.3 | 951.0 | 37.0 | 23.70 | | 297.0 | 210.0 | 69.7 | 38.0 | | | | | | 28.6 | | | |
| Patient n°47 | F | 1 | 12-mai-04 | 1931 | 13.89 | 16.5 | | 16.5 | 0.84 | 2.54 | 6.50 | | 786.6 | 99.2 | 5.93 | 39.0 | 369.7 | 325.0 | 70.2 | 39.0 | 129.0 | | | | 103.0 | 211.0 | | | |
| Patient n°48 | M | 1 | 14-févr-05 | 1962 | 12.31 | 10.8 | 10.8 | 10.8 | 1.14 | 1.20 | | 0.2 | 1190.0 | 101.0 | 9.78 | | 334.0 | | 39.0 | | | | | | 414.0 | | | 8.70 | 13.00 |
| Patient n°49 | F | 1 | 29-sept-04 | 1945 | 9.20 | 12.1 | | 12.1 | 0.76 | 1.76 | 6.88 | 0.5 | 1000.0 | 109.0 | 7.17 | 50.0 | 417.0 | 515.0 | 39.0 | 42.0 | | 0.90 | 0.73 | 1.23 | | 53.0 | 1100.0 | 17.00 | 34.00 |
| Patient n°50 | M | 1 | 29-sept-04 | 1946 | 8.08 | 13.8 | | 13.8 | 0.59 | 2.14 | 6.45 | 0.5 | 861.0 | 40.0 | 19.53 | 49.0 | 381.0 | 610.0 | 50.0 | 44.0 | 133.0 | 0.93 | 0.64 | 1.45 | | 44.0 | 786.0 | 13.00 | 9.00 |
| Patient n°51 | F | 1 | 6-oct-04 | 1941 | 12.09 | 18.0 | | 18.0 | 0.67 | 2.33 | 7.68 | 0.9 | 782.0 | 55.0 | 12.22 | | 317.0 | 482.0 | 75.0 | 40.0 | 111.0 | 1.80 | 0.61 | 2.95 | | 178.8 | 105.5 | 1.00 | 0.50 |
| Patient n°52 | F | 1 | 7-oct-03 | 1928 | 6.74 | 17.6 | | 17.6 | 0.38 | 1.97 | 8.93 | 26.4 | 755.3 | 92.4 | 6.18 | | 310.2 | 25.0 | 48.2 | 34.0 | 100.0 | | | | | 169.6 | | | |
| Patient n°52 | F | 1 | 31-mars-04 | 1928 | 10.06 | 17.0 | | 17.0 | 0.59 | 2.17 | 7.93 | | 683.4 | 47.0 | 12.54 | | 302.9 | 110.0 | 43.1 | 40.0 | | | | | | | | | |
| Patient n°53 | F | 1 | 24-févr-04 | 1952 | 8.88 | 16.3 | | 16.3 | 0.54 | 2.27 | 7.18 | 89.4 | 855.0 | 45.6 | 16.74 | | 402.0 | 185.0 | 44.2 | 39.0 | | | | | | 89.7 | | | |
| Patient n°54 | F | 1 | 23-sept-03 | 1947 | 7.81 | 18.5 | | 18.5 | 0.42 | 2.07 | 8.94 | 0.3 | 772.8 | 81.6 | 7.47 | 63.0 | 413.3 | 20.0 | 55.1 | 48.0 | 107.0 | 0.84 | 0.59 | 1.42 | 220.0 | 99.5 | 394.6 | | 1.49 |
| Patient n°55 | F | 1 | 6-sept-05 | 1921 | | 17.3 | 17.3 | 17.5 | 0.00 | 2.38 | 7.27 | | 824.0 | 3.0 | 272.67 | | 383.0 | | 54.0 | | | | | | | 158.4 | | | |
| Patient n°56 | M | 1 | 27-juil-04 | 1948 | 11.84 | 17.5 | | 17.5 | 0.68 | 2.76 | 6.34 | 0.3 | 1001.0 | 50.0 | 18.02 | | 377.0 | 585.0 | 35.2 | 49.0 | | | | | | | | | |
| Patient n°57 | M | 1 | 12-oct-04 | 1945 | 10.67 | 11.8 | | 11.8 | 0.90 | 2.09 | 5.65 | 0.3 | 782.0 | 19.0 | 39.16 | | 344.0 | 565.0 | 32.0 | 63.0 | | | | | 218.0 | | | | |
| Patient n°58 | F | 1 | 13-sept-04 | 1970 | 9.56 | 10.2 | | 10.2 | 0.94 | 1.38 | 7.39 | 0.3 | 858.0 | 30.0 | 26.60 | | 292.0 | 375.0 | 52.0 | 46.0 | | | | | | 30.0 | | | |
| Patient n°59 | F | 1 | 22-sept-04 | 1970 | 12.15 | 10.9 | | 10.9 | 1.11 | 2.01 | 5.42 | 0.9 | 1011.0 | 23.0 | 41.96 | | 345.0 | 317.5 | 50.0 | 32.0 | | | | | | 51.3 | | | |
| Patient n°60 | M | 1 | 23-sept-03 | 1948 | 4.54 | 27.4 | | 27.4 | 0.17 | 2.03 | 13.50 | 55.8 | 803.4 | 60.6 | 11.27 | ### | 385.2 | 25.0 | 63.6 | 53.0 | 143.0 | 0.71 | 0.59 | 1.20 | | 96.6 | 232.8 | 11.91 | 15.48 |
| Patient n°60 | M | 2 | 7-avr-04 | 1948 | 10.12 | 25.2 | | 25.2 | 0.40 | 2.58 | 9.77 | | 1582.4 | 79.8 | 17.82 | | 356.7 | 450.0 | 59.3 | 75.0 | | | | | | | | 9.44 | 33.98 |
| Patient n°61 | F | 1 | 14-mars-05 | 1951 | 9.16 | 12.2 | 12.2 | 12.2 | 0.75 | 2.00 | | 0.6 | 649.0 | 50.0 | 10.98 | 78.0 | 323.0 | | 41.0 | | 100.0 | 0.85 | 0.79 | 1.08 | 318.0 | 47.0 | 450.0 | 6.80 | 8.80 |
| Patient n°62 | M | 1 | 13-avr-04 | 1954 | 4.99 | 13.4 | | 13.4 | 0.37 | 1.96 | 6.84 | 1.5 | 891.8 | 17.8 | 48.19 | | 450.2 | 625.0 | 30.2 | 78.0 | | | | | | 100.4 | | | |
| Patient n°63 | F | 1 | 7-sept-04 | 1956 | 14.11 | 14.2 | | 14.2 | 0.99 | 2.17 | 6.54 | 0.9 | 888.0 | 40.0 | 20.20 | 77.0 | 315.0 | 290.0 | 67.0 | 39.0 | 90.0 | 1.05 | 0.62 | 1.69 | | 71.9 | 161.0 | 21.00 | 47.12 |
| Patient n°64 | M | 1 | 23-avr-04 | 1921 | 7.05 | 18.1 | | 18.1 | 0.39 | 2.34 | 7.73 | | 918.0 | 130.1 | 5.06 | 69.0 | 310.9 | 470.0 | 40.2 | 71.0 | 133.0 | | | | | 52.0 | 209.1 | 8.29 | 9.12 |
| Patient n°65 | M | 1 | 26-févr-03 | 1951 | 11.32 | 16.1 | | 16.1 | 0.70 | 2.37 | 7.13 | 46.3 | 835.0 | 70.3 | 9.87 | 54.0 | 331.5 | 140.0 | 56.7 | 59.0 | 108.0 | 1.32 | 0.76 | 1.74 | 356.4 | 31.5 | 582.7 | 11.99 | 29.98 |

| | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|
| Patient n°65 | M | 2 | 23-juin-03 | 1951 | 39.62 | 19.3 | | 19.3 | | 2.05 | 2.34 | 8.25 | | 1065.6 | 34.3 | 29.08 | | 385.4 | 235.0 | 65.8 | 52.0 | | | | | 271.8 | 255.0 | | | |
| Patient n°66 | F | 1 | 4-mai-04 | 1949 | 14.05 | 12.9 | | 12.9 | | 1.09 | 2.41 | 5.35 | | 1176.3 | 112.0 | 8.51 | 41.0 | 329.1 | 270.0 | 31.3 | 69.0 | 125.0 | | | | 119.6 | | | | |
| Patient n°67 | F | 1 | 27-avr-05 | 1926 | 1.28 | | 16.6 | 16.6 | 0.89 | 0.08 | 3.31 | | 0.5 | 810.0 | 107.0 | 5.57 | | 261.0 | | 71.0 | | | 1.26 | 0.30 | 4.20 | 649.0 | | | | |
| Patient n°68 | F | 1 | 12-mai-03 | 1963 | 9.06 | 12.9 | | 12.9 | | 0.70 | 1.58 | 8.17 | 16.1 | 696.8 | 1.6 | 425.45 | 73.0 | 384.3 | 65.0 | 55.1 | 27.0 | 105.0 | 1.86 | 0.69 | 2.70 | 912.0 | 49.7 | 509.9 | 185.00 | 5.55 |
| | | | | | | | | | | | | | | | | | | | | | | | | | | | | | 9.77 | 19.54 |
| Patient n°69 | F | 1 | 7-avr-04 | 1958 | | 14.5 | | 14.5 | | 0.00 | 1.91 | 7.59 | | 848.2 | 56.6 | 12.98 | | 385.8 | 155.0 | 41.6 | 40.0 | | | | | | | | | |
| Patient n°69 | F | 2 | 4-nov-03 | 1958 | 15.42 | 9.3 | | 9.3 | | 1.66 | 1.63 | 5.71 | 42.5 | 876.2 | 66.1 | 11.26 | 73.0 | 459.4 | 150.0 | 34.9 | 35.0 | 81.0 | 1.71 | 0.54 | 3.17 | 125.3 | 208.5 | | 13.70 | 30.14 |
| | | | | | | | | | | | | | | | | | | | | | | | | | | | | | 7.22 | 18.78 |
| Patient n°70 | F | 1 | 21-avr-04 | 1952 | 4.67 | 13.3 | | 13.3 | | 0.35 | 2.31 | 5.76 | | 1055.3 | 6.2 | 167.13 | | 442.1 | 160.0 | 32.7 | 38.0 | | | | | 44.4 | | | | |
| Patient n°71 | F | 1 | 1-sept-04 | 1934 | 6.62 | 27.0 | | 27.0 | | 0.25 | 2.38 | 11.34 | 0.9 | 702.0 | 8.0 | 85.75 | | 272.0 | 567.5 | 47.0 | 61.0 | 97.0 | 1.07 | 0.61 | 1.75 | | | | | |
| Patient n°72 | F | 1 | 9-nov-04 | 1919 | 3.58 | 21.1 | | 21.1 | | 0.17 | 3.34 | 6.32 | 0.6 | 1193.0 | 76.0 | 13.70 | | 355.0 | 620.0 | 76.0 | 48.0 | | | | | 408.0 | | | | |
| Patient n°73 | F | 1 | 8-sept-04 | 1954 | 8.04 | 16.0 | | 16.0 | | 0.50 | 2.27 | 7.05 | 0.7 | 721.0 | 10.0 | 70.10 | | 344.0 | 360.0 | 56.4 | 58.0 | | | | | 39.6 | | | | |
| Patient n°74 | F | 1 | 4-avr-05 | 1960 | 8.73 | | 13.1 | 13.1 | 0.42 | 0.67 | 1.86 | 7.04 | 1.4 | 1125.0 | 5.0 | 223.00 | | | | 43.0 | | | | | | 587.0 | | | | |
| Patient n°75 | F | 1 | 14-déc-04 | 1938 | 11.16 | 16.5 | | 16.5 | | 0.68 | 2.30 | 7.17 | 1.1 | 967.0 | 2.0 | 481.50 | | 295.0 | | 59.5 | 50.0 | 107.0 | 1.08 | 0.63 | 1.71 | 390.0 | | | | |
| Patient n°76 | M | 1 | 27-janv-04 | 1950 | 7.72 | 13.5 | | 13.5 | | 0.57 | 2.20 | 6.14 | 0.2 | 1041.5 | 115.3 | 7.03 | 59.0 | 409.6 | 180.0 | 50.2 | 42.0 | 104.0 | 0.84 | 0.66 | 1.27 | | 97.4 | 287.1 | 9.14 | 28.34 |
| | | | | | | | | | | | | | | | | | | | | | | | | | | | | | 2.16 | 1.94 |
| Patient n°76 | M | 2 | 23-juin-04 | 1950 | 12.18 | 12.1 | | 12.1 | | 1.01 | 1.69 | | 0.4 | 970.0 | 111.0 | 6.74 | | | 365.0 | 30.0 | | | | | | | | | | |
| Patient n°77 | F | 1 | 7-déc-04 | 1922 | 14.04 | 16.2 | | 16.2 | | 0.87 | 2.51 | 6.45 | 0.9 | 909.0 | 2.0 | 452.50 | | 334.0 | | 24.2 | 62.0 | | | | | 316.0 | | | | |
| Patient n°78 | F | 1 | 26-avr-05 | 1921 | 3.20 | | 14.7 | 14.7 | 1.43 | 0.22 | 2.11 | | 0.3 | 640.0 | 7.0 | 89.43 | | 270.0 | | 44.0 | | | 1.19 | 0.45 | 2.64 | 617.0 | | | | |
| Patient n°79 | F | 1 | 3-févr-04 | 1969 | 6.96 | 14.4 | | 14.4 | | 0.48 | 2.32 | 6.21 | 0.2 | 1187.0 | 77.0 | 13.42 | 59.0 | 428.0 | 190.0 | 43.3 | 37.0 | 103.0 | 0.95 | 0.76 | 1.25 | | 47.1 | 231.7 | 12.50 | 33.74 |
| Patient n°80 | F | 1 | 6-juil-04 | 1951 | 8.04 | 14.2 | | 14.2 | | 0.57 | 1.50 | 9.47 | 0.9 | 663.0 | 71.0 | 7.34 | | 497.0 | | | 33.0 | | | | | 70.3 | | | | |
| Patient n°80 | F | 2 | 14-févr-05 | 1951 | 2.51 | | 13.5 | 13.5 | 0.19 | 0.94 | 14.36 | 2.3 | 844.0 | 125.0 | 4.75 | | 352.0 | | 41.0 | 28.0 | | | | | 208.0 | | | | |
| Patient n°81 | M | 1 | 6-juil-04 | 1947 | 4.12 | 10.4 | | 10.4 | | 0.40 | 2.12 | 4.91 | 0.5 | 905.0 | 163.0 | 3.55 | | 431.0 | | | 35.0 | | | | | 69.3 | | | | |
| Patient n°81 | M | 2 | 14-févr-05 | 1947 | 9.35 | | 10.0 | 10.0 | 0.94 | 0.82 | 12.20 | 1.2 | 902.0 | 132.0 | 4.83 | | 317.0 | | 37.8 | 15.0 | | | | | 136.0 | | | | |
| Patient n°82 | M | 1 | 10-nov-04 | 1942 | 6.85 | 14.3 | | 14.3 | | 0.48 | 2.25 | 6.36 | 0.3 | 1085.0 | 55.0 | 17.73 | | 269.0 | 360.0 | 56.0 | 49.0 | | | | | 273.0 | | | | |
| Patient n°83 | M | 1 | 27-sept-04 | 1945 | 6.70 | 12.6 | | 12.6 | | 0.53 | 1.77 | 7.12 | 0.3 | 759.0 | 27.0 | 26.11 | | 363.0 | 790.0 | 28.6 | 59.0 | | | | | 62.3 | | | | |
| | | | | | | | | | | | | | | | | | | | | | | | | | | | | | 9.50 | 3.80 |
| Patient n°84 | F | 1 | 27-sept-04 | 1944 | 15.07 | 14.0 | | 14.0 | | 1.08 | 2.05 | 6.83 | 1.1 | 822.0 | 101.0 | 6.14 | | 370.0 | 505.0 | 55.0 | 26.0 | | | | | 271.2 | | | | |
| Patient n°85 | F | 1 | 12-mai-04 | 1951 | 12.06 | 14.3 | | 14.3 | | 0.84 | 1.97 | 7.26 | | 749.7 | 71.3 | 8.51 | 54.0 | 404.0 | 450.0 | 38.9 | 66.0 | 141.0 | 1.02 | 0.82 | 1.46 | 271.2 | | | | |
| Patient n°85 | F | 2 | 13-oct-04 | 1951 | 10.70 | 14.2 | | 14.2 | | 0.75 | 0.89 | 15.96 | 1.3 | 663.0 | 22.0 | 28.14 | | 342.0 | 880.0 | 47.0 | 23.0 | 142.0 | 1.10 | 0.73 | 1.51 | 54.0 | | | | |
| Patient n°86 | F | 1 | 16-juin-04 | 1957 | 10.38 | 9.4 | | 9.4 | | 1.10 | 1.63 | 5.77 | 1.2 | 922.0 | 62.0 | 12.87 | | 553.0 | 530.0 | 26.2 | 44.0 | | | | | 56.9 | | | | |

EP 1 793 230 B1

| Patient | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|
| Patient n°87 | M | 1 | 10-mai-04 | 1926 | 20.63 | 40.1 | | 40.1 | | 0.51 | 2.60 | 15.42 | | 724.3 | 21.4 | 31.88 | 56.0 | 289.9 | 715.0 | 106.6 | 78.0 | 116.0 | 0.92 | 0.68 | 1.35 | | 198.4 | | | |
| Patient n°87 | M | 2 | 12-oct-04 | 1926 | 5.96 | 21.8 | | 21.8 | | 0.27 | 2.60 | 8.39 | 1.0 | 779.0 | 42.0 | 16.55 | | 288.0 | 1065.0 | 73.0 | 83.0 | | | | | | 86.0 | | | |
| Patient n°88 | F | 1 | 15-sept-04 | 1977 | 8.45 | 11.9 | | 11.9 | | 0.71 | 1.66 | 7.17 | 0.2 | 782.0 | 11.0 | 69.09 | | 284.0 | 275.0 | 40.0 | 33.0 | | | | | | 46.0 | | | |
| Patient n°89 | M | 1 | 6-sept-04 | 1940 | 4.11 | 12.9 | | 12.9 | | 0.32 | 2.10 | 6.14 | 0.7 | 1047.0 | 36.0 | 27.08 | | 400.0 | 340.0 | 63.0 | 43.0 | | | | | | 61.9 | | | |
| Patient n°90 | F | 1 | 1-sept-04 | 1945 | 1.14 | 14.2 | | 14.2 | | 0.08 | 1.94 | 7.32 | 0.9 | 840.0 | 14.0 | 58.00 | | 306.0 | 367.5 | 45.6 | 47.0 | | | | | | 25.2 | | | |
| Patient n°91 | M | 1 | 27-juil-04 | 1957 | 3.85 | 15.7 | | 15.7 | | 0.25 | 2.37 | 6.62 | 0.4 | 712.0 | 44.0 | 14.18 | | 334.0 | 350.0 | 48.7 | 46.0 | | | | | | 60.3 | | | |
| Patient n°91 | M | 2 | 19-janv-05 | 1957 | 4.86 | 17.5 | | 17.5 | | 0.28 | 2.57 | 6.81 | 0.7 | 864.0 | 102.0 | 6.47 | | 385.0 | | 51.1 | 50.0 | | | | | | | | | 33.20 |
| Patient n°92 | F | 1 | 20-sept-04 | 1935 | 20.40 | 7.9 | | 7.9 | | 2.58 | 2.20 | 3.59 | 0.3 | 265.0 | 44.0 | 4.02 | | 315.0 | 660.0 | 64.0 | 58.0 | | | | | | | | | 19.70 |
| Patient n°93 | F | 1 | 14-avr-04 | 1942 | 7.53 | 13.1 | | 13.1 | | 0.57 | 2.69 | 4.87 | | 924.5 | 82.2 | 9.24 | | 432.3 | 160.0 | 22.7 | 47.0 | | | | | | 117.4 | | | |
| Patient n°94 | F | 1 | 14-avr-04 | 1948 | 13.78 | 14.5 | | 14.5 | | 0.95 | 1.66 | 8.74 | | 705.8 | 62.4 | 9.30 | | 370.2 | 80.0 | 45.1 | 31.0 | | | | | | 62.3 | | | |
| Patient n°94 | F | 2 | 27-sept-04 | 1948 | 14.30 | 13.7 | | 13.7 | | 1.04 | 2.02 | 6.78 | 2.0 | 1043.0 | 54.0 | 17.31 | | 379.0 | 320.0 | 30.0 | 22.0 | | | | | | 62.3 | | | |
| Patient n°95 | F | 1 | 7-oct-03 | 1952 | 10.50 | 16.1 | | 16.1 | | 0.65 | 2.09 | 7.70 | 53.2 | 934.7 | 87.7 | 8.65 | 60.0 | 348.6 | 55.0 | 44.0 | 42.0 | 134.0 | 0.85 | 0.69 | 1.23 | | 155.1 | 241.9 | 2.50 | 0.50 |
| Patient n°96 | M | 1 | 29-oct-03 | 1983 | 10.00 | 11.4 | | 11.4 | | 0.88 | 1.38 | 8.26 | 54.2 | 798.3 | 107.9 | 5.40 | 62.0 | 417.6 | 10.0 | 57.8 | 58.0 | 84.0 | 0.65 | 0.70 | 0.93 | | 70.0 | 493.5 | 4.22 | 14.34 |
| Patient n°97 | M | 1 | 29-sept-04 | 1939 | 12.52 | 10.0 | | 10.0 | | 1.25 | 1.70 | | | 858.0 | 46.0 | 16.65 | 51.0 | 377.0 | 675.0 | | 57.0 | 91.0 | 0.70 | 0.57 | 1.23 | | 35.0 | 1100.0 | 11.50 | 23.00 |
| Patient n°98 | F | 1 | 29-sept-04 | 1945 | 11.35 | 13.7 | | 13.7 | | 0.83 | 2.25 | | 0.9 | 838.0 | 36.0 | 21.28 | | 384.0 | 377.0 | 52.0 | 40.0 | | | | | 398.0 | | | | |
| Patient n°99 | M | 1 | 13-avr-04 | 1970 | 7.32 | 12.1 | | 12.1 | | 0.60 | 1.82 | 6.65 | | 904.9 | 62.0 | 12.60 | | 483.8 | 415.0 | 28.7 | 57.0 | | | | | | 99.4 | | | |
| Patient n°100 | M | 1 | 28-sept-04 | 1956 | 7.39 | 13.8 | | 13.8 | | 0.54 | 2.58 | 5.35 | 0.3 | 822.0 | 31.0 | 24.52 | | 336.0 | 1060.0 | 39.0 | 69.0 | | | | | | 82.3 | | | |
| Patient n°101 | F | 1 | 28-sept-04 | 1932 | 6.69 | 8.4 | | 8.4 | | 0.80 | 1.12 | 7.50 | 0.3 | 487.0 | 67.0 | 5.27 | | 292.0 | 635.0 | 34.0 | 37.0 | | | | | | 34.0 | | | |
| Patient n°102 | F | 1 | 11-mai-04 | 1943 | 13.41 | 16.9 | | 16.9 | | 0.79 | 1.83 | 9.24 | | 1686.6 | 42.8 | 37.38 | 66.0 | 439.9 | 175.0 | 53.6 | 35.0 | 133.0 | | | | | 188.5 | | | |
| Patient n°103 | F | 1 | 26-mai-04 | 1968 | 11.43 | 14.5 | | 14.5 | | 0.79 | 1.71 | 8.48 | | 859.3 | 55.0 | 13.62 | 42.0 | 404.7 | 335.0 | 45.1 | 49.0 | 85.0 | 1.12 | 0.65 | 1.72 | | 35.3 | | | |
| Patient n°104 | F | 1 | 27-sept-05 | 1972 | 14.70 | | 12.2 | 12.2 | 0.86 | 1.20 | 1.60 | 7.63 | 0.7 | 1036.0 | 49.0 | 19.14 | | 372.0 | | 52.0 | | | | | | 481.0 | | | | |
| Patient n°105 | F | 1 | 7-avr-03 | 1941 | 14.24 | 15.7 | | 15.7 | | 0.91 | 2.04 | 7.70 | 116.3 | 965.0 | 16.9 | 55.17 | 43.0 | 379.8 | 125.0 | 59.5 | 29.0 | 135.0 | 1.05 | 0.70 | 1.50 | 313.3 | 97.9 | 827.0 | | |
| Patient n°106 | F | 1 | 18-mai-05 | 1936 | 13.49 | | 13.8 | 13.8 | 0.68 | 0.98 | 2.00 | 6.80 | 0.8 | 982.0 | 115.0 | 6.54 | 39.0 | 378.0 | | 55.0 | 43.0 | 123.0 | 1.10 | 0.80 | 1.30 | 305.0 | 35.0 | 576.0 | 9.00 | 3.00 |
| Patient n°107 | F | 1 | 9-mars-04 | 1949 | 13.61 | 16.7 | | 16.7 | | 0.81 | 2.09 | 7.99 | | 952.7 | 174.6 | 3.46 | | 287.4 | 85.0 | 33.6 | 39.0 | | | | | | | | 14.11 | 21.16 |
| Patient n°108 | M | 1 | 4-nov-03 | 1957 | 13.62 | 15.6 | | 15.6 | | 0.87 | 1.94 | 8.04 | 14.7 | 1348.6 | 138.4 | 7.75 | 77.0 | 483.2 | 455.0 | 50.4 | 78.0 | 80.0 | 0.93 | 0.59 | 1.58 | | 321.7 | 62.4 | 37.85 | 41.64 |
| Patient n°109 | F | 1 | 2-déc-03 | 1958 | 7.42 | 17.1 | | 17.1 | | 0.43 | 2.25 | 7.60 | 82.1 | 1182.7 | 133.0 | 6.90 | 72.0 | 381.2 | 130.0 | 28.4 | 47.0 | 86.0 | 0.76 | 0.63 | 1.21 | | 202.5 | 335.7 | 9.86 | 19.71 |
| Patient n°109 | F | 2 | 9-févr-05 | 1958 | 8.34 | | 4.5 | 4.5 | 0.85 | 1.85 | 2.05 | 2.20 | 0.9 | 949.0 | 76.0 | 10.49 | 63.0 | 303.0 | | 45.0 | 42.0 | 103.0 | 0.93 | 0.71 | 1.31 | | 18.0 | 880.0 | | 0.40 |

EP 1 793 230 B1

| | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|
| Patient n°110 | M | 2 | 28-juin-05 | 1949 | 13.47 | | 15.7 | 15.7 | 0.47 | 0.86 | 1.17 | 13.42 | 0.8 | 750.0 | 88.0 | 6.52 | | 341.0 | | 64.0 | | | | | | | 184.0 | | | |
| Patient n°111 | M | 1 | 5-mai-04 | 1953 | 5.50 | 16.6 | | 16.6 | | 0.33 | 2.54 | 6.54 | | 1098.4 | 109.4 | 8.04 | 33.0 | 436.9 | 255.0 | 41.8 | 46.0 | 162.0 | | | | | 182.8 | | | |
| Patient n°111 | M | 2 | 30-nov-04 | 1953 | 8.91 | 13.2 | | 13.2 | | 0.68 | 2.45 | 5.39 | 2.0 | 847.0 | 14.0 | 58.50 | | 362.0 | | 44.0 | 51.0 | | | | | | 53.8 | | | |
| Patient n°112 | M | 1 | 23-juin-04 | 1965 | 7.91 | 12.2 | | 12.2 | | 0.65 | 2.06 | 5.92 | 0.6 | 815.0 | 121.0 | 4.74 | | 394.0 | | 49.0 | | | | | | | 45.1 | | | |
| Patient n°113 | F | 1 | 30-nov-04 | 1947 | 11.15 | 15.5 | | 15.5 | | 0.72 | 2.39 | 6.48 | 0.7 | 1003.0 | 15.0 | 64.87 | | 313.0 | | 52.4 | 46.0 | | | | | | 297.0 | | | |
| Patient n°114 | M | 1 | 13-oct-04 | 1924 | 6.09 | 8.1 | | 8.1 | | 0.75 | 1.50 | 5.40 | 0.1 | 573.0 | 16.0 | 33.81 | 73.0 | 347.0 | 955.0 | 31.0 | 59.0 | 72.0 | 1.21 | 0.67 | 1.81 | | 45.0 | 278.0 | 18.00 | 11.00 |
| Patient n°115 | F | 1 | 1-juin-05 | 1948 | 9.28 | | 10.7 | 10.7 | 1.82 | 0.87 | 1.58 | 6.77 | 0.3 | 890.0 | 36.0 | 22.72 | | 319.0 | | 37.0 | | | | | | | 438.0 | | | |
| Patient n°116 | F | 1 | 18-oct-04 | 1937 | 15.31 | 18.2 | | 18.2 | | 0.84 | 2.14 | 8.50 | 1.3 | 1053.0 | 28.0 | 35.61 | | 291.0 | 295.0 | 71.0 | 28.0 | | | | | | 59.0 | | | |
| Patient n°116 | F | 2 | 11-mai-04 | 1937 | 15.62 | 18.0 | | 18.0 | | 0.87 | 2.07 | 8.70 | | 1817.0 | 28.7 | 61.35 | 55.0 | 393.1 | 110.0 | 42.2 | 25.0 | 128.0 | 1.03 | 0.75 | 1.37 | | 168.3 | | | |
| Patient n°117 | F | 1 | 25-nov-03 | 1947 | 7.94 | 14.5 | | 14.5 | | 0.55 | 2.51 | 5.78 | 33.4 | 816.1 | 98.6 | 6.28 | 68.0 | 383.8 | 265.0 | 26.9 | 61.0 | 80.0 | 1.04 | 0.70 | 1.49 | | 210.4 | 45.1 | 8.70 | 13.92 |
| Patient n°118 | M | 1 | 25-nov-03 | 1941 | 5.16 | 18.5 | | 18.5 | | 0.28 | 2.56 | 7.23 | 36.9 | 1109.0 | 122.9 | 7.03 | 68.0 | 345.2 | 350.0 | 30.4 | 74.0 | 97.0 | 0.91 | 0.64 | 1.42 | | 312.1 | 333.2 | 8.06 | 19.34 |
| Patient n°119 | M | 1 | 12-mars-03 | 1921 | 15.38 | 20.5 | | 20.5 | | 0.75 | 2.29 | 8.95 | 72.8 | 1137.6 | 27.2 | 39.82 | ### | 336.5 | 155.0 | 67.8 | 50.0 | 139.0 | 0.74 | 0.71 | 1.04 | 217.3 | 74.1 | #### | 10.89 | 13.07 |
| Patient n°120 | F | 1 | 18-mars-03 | 1919 | 10.58 | 16.1 | | 16.1 | | 0.66 | 2.83 | 5.69 | 127.7 | 871.0 | 2.1 | 418.79 | 70.0 | 331.3 | 280.0 | 66.2 | 74.0 | 123.0 | 1.25 | 0.64 | 1.95 | 421.2 | 76.7 | 73.2 | 12.84 | 10.27 |
| Patient n°121 | F | 1 | 11-oct-04 | 1959 | 14.44 | 11.5 | | 11.5 | | 1.26 | 1.33 | 8.65 | 2.0 | 951.0 | 40.0 | 21.78 | | 333.0 | 370.0 | 48.0 | 25.0 | | | | | | 252.0 | | | |
| Patient n°121 | F | 2 | 9-mars-05 | 1958 | 10.38 | | 9.1 | 9.1 | 0.58 | 1.14 | 0.97 | | 0.9 | 779.0 | 36.0 | 19.64 | | | | 37.0 | | | | | | 227.0 | | | |
| Patient n°122 | F | 1 | 11-oct-04 | 1933 | 7.86 | 13.6 | | 13.6 | | 0.58 | 2.27 | | 0.9 | 716.0 | 14.0 | 49.14 | | 357.0 | 475.0 | 45.0 | 43.0 | | | | | | 314.0 | | | |
| Patient n°123 | F | 1 | 27-juil-04 | 1950 | 2.55 | 20.3 | | 20.3 | | 0.13 | 2.77 | 7.33 | 1.3 | 693.0 | 78.0 | 6.88 | | 456.0 | 550.0 | 18.8 | 62.0 | | | | | | 105.3 | | | |
| Patient n°124 | F | 1 | 14-avr-04 | 1948 | 5.10 | 12.4 | | 12.4 | | 0.41 | 2.25 | 5.51 | | 953.5 | 162.7 | 3.86 | | 402.5 | 510.0 | 46.0 | 52.0 | | | | | | 145.0 | | | |
| Patient n°124 | F | 2 | 17-nov-04 | 1948 | 11.84 | 11.4 | | 11.4 | | 1.04 | 1.99 | 5.73 | 1.5 | 1200.0 | 5.0 | 238.00 | | 361.0 | 900.0 | 42.0 | 53.0 | | | | | | 126.0 | | | |
| Patient n°125 | F | 1 | 7-juin-05 | 1954 | 10.39 | | 16.5 | 16.5 | 1.19 | 0.63 | | 1.87 | 0.3 | 785.0 | 124.0 | 4.33 | | 375.0 | | 52.0 | | | | | | | 217.0 | | | |
| Patient n°126 | F | 1 | 20-oct-04 | 1950 | 4.19 | 16.1 | | 16.1 | | 0.26 | 2.85 | 5.65 | 1.1 | 918.0 | 110.0 | 6.35 | 65.0 | 374.0 | 1095.0 | 40.0 | 81.0 | 105.0 | 1.22 | 0.80 | 1.52 | | 40.0 | 119.0 | 13.00 | 4.00 |
| Patient n°127 | M | 1 | 15-sept-04 | 1950 | 6.80 | 15.6 | | 15.6 | | 0.44 | 2.00 | 7.80 | 1.2 | 676.0 | 69.0 | 7.80 | | 327.0 | 677.5 | 72.0 | 69.0 | | | | | | 55.7 | | | |
| Patient n°128 | F | 1 | 17-mars-04 | 1949 | 13.26 | 14.3 | | 14.3 | | 0.93 | 1.82 | 7.86 | 1.4 | 1055.0 | 28.9 | 34.53 | 59.0 | 273.0 | 195.0 | 30.0 | 33.0 | 112.0 | 1.13 | 0.67 | 1.69 | | 40.5 | 350.0 | 11.45 | 2.29 |
| Patient n°129 | F | 1 | 13-sept-04 | 1938 | 15.28 | 15.6 | | 15.6 | | 0.98 | 2.25 | 6.93 | 1.1 | 925.0 | 17.0 | 52.41 | 54.0 | 281.0 | 555.0 | 52.4 | 41.0 | 124.0 | 0.90 | 0.70 | 1.29 | | 56.3 | 129.0 | 24.00 | 42.00 |
| Patient n°130 | M | 1 | 13-sept-04 | 1952 | 7.28 | 22.4 | | 22.4 | | 0.33 | 1.89 | 11.85 | 0.9 | 1369.0 | 37.0 | 35.00 | | 331.0 | 730.0 | 74.6 | 27.0 | | | | | | 66.8 | | | |
| Patient n°131 | M | 1 | 21-avr-04 | 1945 | 9.24 | 12.0 | | 12.0 | | 0.77 | 1.85 | 6.49 | | 983.4 | 74.2 | 11.25 | | 411.4 | 415.0 | 29.1 | 61.0 | | | | | | 200.0 | | | |
| Patient n°131 | M | 2 | 7-sept-04 | 1945 | 10.60 | 13.4 | | 13.4 | | 0.79 | 1.97 | 6.80 | 1.9 | 1709.0 | 38.0 | 42.97 | | 320.0 | 400.0 | 54.6 | 61.0 | | | | | | 20.4 | | | |

| Patient | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|
| Patient n°131 | M | 3 | 1-juin-05 | 1945 | 7.35 | | 15.5 | 15.5 | | 0.47 | 1.86 | 8.33 | 0.6 | 990.0 | 187.0 | 3.29 | | 363.0 | | 51.0 | | | | | | | | 44.0 | | | |
| Patient n°132 | F | 1 | 21-avr-04 | 1947 | 8.71 | 18.7 | | 18.7 | | 0.47 | 2.53 | 7.39 | | 709.4 | 47.7 | 12.86 | | 442.7 | 485.0 | 53.8 | 58.0 | | | | | | | | | 13.76 | 26.14 |
| Patient n°132 | F | 2 | 7-sept-04 | 1947 | 12.70 | 18.2 | | 18.2 | | 0.70 | 2.18 | 8.30 | 1.5 | 666.0 | 23.0 | 26.96 | | 299.0 | 240.0 | 54.0 | 50.0 | | | | | | | | | | 12.70 |
| Patient n°132 | F | 3 | 1-juin-05 | 1947 | 10.40 | | 16.3 | 16.3 | | 0.64 | 2.05 | 7.95 | 0.2 | 543.0 | 67.0 | 6.10 | | 323.0 | | 53.0 | | | | | | | | | | 12.20 | 11.00 |
| Patient n°133 | F | 1 | 22-sept-04 | 1935 | 5.29 | 15.2 | | 15.2 | | 0.35 | 2.29 | 6.64 | 1.4 | 675.0 | 31.0 | 19.77 | | 371.0 | 540.0 | 71.0 | 39.0 | | | | | | 55.0 | | | | |
| Patient n°134 | M | 1 | 22-oct-03 | 1938 | 18.54 | 16.5 | | 16.5 | | 1.12 | 2.18 | 7.57 | 72.0 | 821.1 | 72.0 | 9.41 | | 327.0 | 10.0 | 49.3 | 53.0 | 91.0 | 0.73 | 0.72 | 1.01 | | 414.2 | 408.7 | | 6.18 |
| Patient n°134 | M | 2 | 7-avr-04 | 1938 | 7.34 | 15.4 | | 15.4 | | 0.48 | 2.41 | | | 880.9 | 97.2 | 7.07 | | 280.1 | 270.0 | 38.4 | 64.0 | | | | | | 156.3 | | | |
| Patient n°135 | F | 1 | 7-avr-03 | 1933 | 12.89 | 18.5 | | 18.5 | | 0.70 | 2.65 | 6.98 | 107.8 | 844.1 | 8.9 | 92.42 | 50.0 | 351.8 | 235.0 | 65.1 | 57.0 | 95.0 | 0.95 | 0.57 | 1.67 | | 152.7 | 467.5 | | 6.84 |
| Patient n°136 | M | 1 | 27-avr-04 | 1942 | 13.14 | 12.7 | | 12.7 | | 1.03 | 0.92 | 13.80 | | 1078.5 | 140.3 | 5.68 | | 394.0 | 295.0 | 48.7 | 40.0 | | | | | | 255.0 | | | |
| Patient n°136 | M | 2 | 19-oct-04 | 1942 | 6.91 | 12.8 | | 12.8 | | 0.54 | 1.99 | 6.43 | 0.4 | 653.0 | 66.0 | 7.89 | | 366.0 | 1080.0 | 34.0 | 65.0 | | | | | | 43.0 | | | |
| Patient n°137 | F | 1 | 18-juin-03 | 1971 | 9.10 | 18.0 | | 18.0 | | 0.51 | 2.20 | 8.18 | 45.8 | 1118.7 | 106.1 | 8.55 | 68.0 | 448.8 | 185.0 | 69.1 | 39.0 | 88.0 | 1.07 | 0.59 | 1.81 | | 99.6 | 510.0 | | 0.50 |
| Patient n°138 | F | 1 | 27-sept-04 | 1953 | 7.35 | 15.7 | | 15.7 | | 0.47 | 2.36 | 6.65 | 0.3 | 897.0 | 112.0 | 6.01 | | 440.0 | 535.0 | 40.6 | 40.0 | | | | | | 43.0 | | | |
| Patient n°139 | F | 2 | 4-avr-05 | 1938 | 15.31 | | 19.3 | 19.3 | | 0.79 | 2.44 | 7.91 | 1.1 | 729.0 | 23.0 | 29.70 | | 385.0 | | | | | | | | | | | | 6.00 |
| Patient n°140 | F | 1 | 10-août-04 | 1960 | 6.68 | 10.1 | | 10.1 | | 0.66 | 1.76 | 5.74 | 0.6 | 1355.0 | 135.0 | 8.04 | 71.0 | 416.0 | 252.5 | 34.1 | 32.0 | 106.0 | 0.96 | 0.62 | 1.55 | | 56.6 | 816.0 | 7.41 | 7.41 |
| Patient n°141 | F | 1 | 4-nov-03 | 1971 | 17.11 | 11.7 | | 11.7 | | 1.46 | 1.98 | 5.91 | 31.7 | 1110.3 | 109.7 | 8.12 | 73.0 | 444.6 | 210.0 | 44.9 | 42.0 | 90.0 | 1.01 | 0.68 | 1.48 | | 216.5 | 257.9 | 15.27 | 9.16 |
| Patient n°142 | M | 1 | 14-sept-04 | 1935 | 15.17 | 9.9 | | 9.9 | | 1.53 | 1.20 | 8.25 | 0.6 | 971.0 | 84.0 | 9.56 | | 262.0 | 327.5 | 31.5 | 74.0 | | | | | | 38.6 | | | |
| Patient n°143 | F | 1 | 8-sept-04 | 1945 | 14.34 | 15.2 | | 15.2 | | 0.94 | 1.78 | 8.54 | 0.3 | 1124.0 | 29.0 | 36.76 | | 317.0 | 510.0 | 52.4 | 55.0 | | | | | | 27.5 | | | |
| Patient n°144 | F | 1 | 6-janv-04 | 1966 | 16.00 | 7.6 | | 7.6 | | 2.11 | 1.32 | 5.76 | 74.3 | 1097.0 | 98.7 | 9.11 | 54.0 | 353.4 | 115.0 | 27.1 | 29.0 | 78.0 | 0.82 | 0.72 | 1.14 | | 33.7 | 74.4 | 10.44 | 8.35 |
| Patient n°145 | M | 1 | 28-avr-04 | 1937 | 11.84 | 17.0 | | 17.0 | | 0.70 | 2.34 | 7.27 | | 877.3 | 67.2 | 11.05 | | 302.4 | 305.0 | 49.1 | 72.0 | | | | | | 41.9 | | | |
| Patient n°145 | M | 2 | 11-oct-04 | 1937 | 7.45 | 17.7 | | 17.7 | | 0.42 | 2.38 | 7.44 | 1.9 | 724.0 | 73.0 | 7.92 | | 339.0 | 770.0 | 51.0 | 56.0 | | | | | | | | 11.00 | 13.00 |
| Patient n°146 | F | 1 | 25-oct-04 | 1964 | 13.11 | 9.0 | | 9.0 | | 1.46 | 2.00 | 4.50 | 0.4 | 1307.0 | 39.0 | 31.51 | | 397.0 | 545.0 | 38.0 | 49.0 | | | | | 176.0 | | | | |
| Patient n°147 | F | 1 | 25-oct-04 | 1939 | 10.28 | 11.5 | | 11.5 | | 0.89 | 2.22 | 5.18 | 0.2 | 849.0 | 52.0 | 14.33 | | 354.0 | 520.0 | 43.0 | 48.0 | | | | | 340.0 | | | | |
| Patient n°148 | M | 1 | 25-oct-04 | 1967 | 6.96 | 12.6 | | 12.6 | | 0.55 | 2.50 | 5.04 | 0.5 | 814.0 | 39.0 | 18.87 | | 371.0 | 675.0 | 49.0 | 62.0 | | | | | 50.0 | | | | |
| Patient n°149 | F | 1 | 8-sept-04 | 1944 | 8.30 | 14.2 | | 14.2 | | 0.58 | 2.33 | 6.09 | 0.8 | 995.0 | 67.0 | 12.85 | | 324.0 | 330.0 | 54.6 | 41.0 | | | | | | 37.7 | | | |
| Patient n°150 | M | 1 | 23-mars-05 | 1920 | 3.21 | | 14.8 | 14.8 | 1.11 | 0.22 | 1.74 | 8.51 | 0.5 | 1120.0 | 21.0 | 51.33 | | 276.0 | | 34.0 | | | 0.77 | 0.53 | 1.45 | 134.0 | | | | |
| Patient n°151 | F | 1 | 28-sept-04 | 1920 | 5.10 | 8.3 | | 8.3 | | 0.61 | 1.62 | 5.12 | 0.1 | 575.0 | 2.0 | 285.50 | | 234.0 | 620.0 | 33.0 | 35.0 | | | | | | 59.4 | | | |
| Patient n°152 | F | 1 | 25-oct-04 | 1938 | 14.65 | 19.9 | | 19.9 | | 0.74 | 2.43 | 8.19 | 0.6 | 661.0 | 34.0 | 17.44 | | 375.0 | 695.0 | 60.0 | 40.0 | | 1.23 | | 2.63 | 780.0 | | | 41.00 | 33.00 |

| Patient | Sexe | N | Date | Année | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|
| Patient n°153 | M | 1 | 23-mars-05 | 1915 | 0,40 | 14,4 | | 14,4 | 0,46 | 0,03 | 1,63 | 8,83 | 0,3 | 967,0 | 13,0 | 72,38 | | 234,0 | | 33,0 | | | 1,21 | 0,46 | 2,63 | 780,0 | | | | |
| Patient n°154 | F | 1 | 9-nov-04 | 1943 | 5,60 | 15,2 | | 15,2 | | 0,37 | 1,93 | 7,88 | 0,3 | 1063,0 | 2,0 | 529,50 | | 338,0 | 615,0 | 63,0 | 48,0 | | | | | 381,0 | | | | |
| Patient n°155 | M | 1 | 21-févr-05 | 1970 | 7,24 | 12,0 | 12,0 | | 0,53 | 0,60 | 1,85 | 6,49 | 0,1 | 878,0 | 24,0 | 34,58 | | 409,0 | | 37,0 | | | | | | 265,0 | | | | |
| Patient n°156 | M | 1 | 13-sept-04 | 1946 | 9,33 | 19,3 | | 19,3 | | 0,48 | 1,17 | 16,50 | 0,2 | 583,0 | 11,0 | 51,00 | 79,0 | 262,0 | 690,0 | 54,2 | 79,0 | 313,0 | 0,65 | 0,72 | 0,90 | | 32,6 | 207,0 | 28,50 | 42,80 |
| Patient n°157 | M | 1 | 17-févr-04 | 1921 | 5,81 | 18,6 | | 18,6 | | 0,31 | 1,72 | 10,81 | | 1317,6 | 149,2 | 6,83 | | 408,8 | 555,0 | 43,1 | 55,0 | 97,0 | | | | | 141,2 | 413,7 | | |
| Patient n°158 | M | 1 | 27-janv-04 | 1959 | 12,48 | 12,1 | | 12,1 | | 1,03 | 1,85 | 6,54 | 0,3 | 1181,4 | 141,4 | 6,36 | | 393,5 | 260,0 | 45,1 | 46,0 | | 0,85 | 0,69 | 1,23 | | 91,7 | 91,7 | | 28,00 |
| Patient n°158 | M | 1 | 13-juil-05 | 1959 | 6,95 | 10,5 | | 10,5 | | 0,66 | 1,60 | 6,56 | 1,0 | 1119,0 | 114,0 | 7,82 | 49,0 | 371,0 | | 31,3 | 47,0 | | | | | | 149,0 | | | 10,85 |
| Patient n°158 | F | 2 | 9-mars-05 | 1949 | 11,78 | 11,3 | 11,3 | | 0,64 | 1,04 | 2,00 | 5,65 | 0,3 | 799,0 | 9,0 | 86,78 | 86,0 | | | 34,0 | | | | | | | | | | 12,00 |
| Patient n°159 | F | 1 | 19-mai-04 | 1938 | 10,78 | 16,0 | | 16,0 | | 0,67 | 2,04 | 7,84 | 0,4 | 916,2 | 30,0 | 28,52 | | 236,5 | 130,0 | 49,3 | 31,0 | 111,0 | 1,17 | 0,70 | 1,67 | 371,0 | 39,0 | 629,2 | 11,00 | 10,00 |
| Patient n°160 | F | 1 | 6-oct-04 | 1938 | 12,56 | 17,0 | | 17,0 | | 0,74 | 2,27 | 7,49 | 0,9 | 738,0 | 24,0 | 28,75 | | 335,0 | 600,0 | 72,0 | 39,0 | | | | | | 43,0 | | | |
| Patient n°160 | M | 2 | 22-sept-04 | 1936 | 8,19 | 12,8 | | 12,8 | | 0,64 | 1,62 | 7,90 | 0,7 | 1090,0 | 12,0 | 88,83 | | 321,0 | 865,0 | 49,0 | | 70,0 | 0,56 | 0,51 | 1,10 | 217,0 | | | | |
| Patient n°161 | F | 1 | 10-nov-04 | 1939 | 14,63 | 21,8 | | 21,8 | | 0,67 | 2,22 | 9,82 | 0,6 | 899,0 | 112,0 | 6,03 | | 368,0 | 365,0 | 75,0 | 61,0 | 39,0 | | | | 919,0 | | | | |
| Patient n°162 | F | 1 | 20-sept-05 | 1962 | 9,05 | 13,3 | 13,3 | | 0,42 | 0,68 | 1,65 | 8,06 | 0,6 | 818,0 | 175,0 | 2,67 | 65,0 | 362,0 | | 70,0 | 39,0 | 95,0 | 0,79 | 0,67 | 1,18 | 145,5 | 210,3 | 584,2 | 26,76 | 50,85 |
| Patient n°163 | M | 1 | 22-oct-03 | 1943 | 25,38 | 14,2 | | 14,2 | | 1,79 | 1,46 | 9,73 | 14,1 | 858,1 | 65,4 | 11,13 | 73,0 | 374,9 | 160,0 | 49,3 | 59,0 | | | | | 205,0 | | | | 3,90 |
| Patient n°164 | M | 1 | 1-mars-05 | 1973 | 11,20 | 14,2 | 14,2 | | 1,43 | 0,79 | 1,73 | 8,21 | 1,3 | 955,0 | 87,0 | 8,98 | | | | 56,0 | | | | | | | 121,7 | | | |
| Patient n°165 | M | 1 | 6-juil-04 | 1923 | 1,19 | 18,3 | | 18,3 | | 0,07 | 1,89 | 9,63 | 0,8 | 901,0 | 6,0 | 148,17 | | 434,0 | | | 55,0 | | | | | | | | | |
| Patient n°166 | F | 1 | 7-avr-04 | 1978 | 6,30 | 11,1 | | 11,1 | 0,79 | 0,57 | 1,81 | 6,13 | | 985,5 | 15,4 | 62,03 | | 299,6 | 55,0 | 29,1 | | 55,0 | | | | | 77,7 | | | |
| Patient n°167 | F | 1 | 26-avr-05 | 1920 | 2,61 | 9,9 | 9,9 | | | 0,26 | 1,46 | | 0,2 | 862,0 | 19,0 | 43,37 | | 274,0 | 500,0 | 37,0 | 32,0 | | 1,27 | 0,66 | 1,92 | 780,0 | | | | |
| Patient n°168 | F | 1 | 27-sept-04 | 1951 | 5,66 | 12,8 | | 12,8 | 0,75 | 0,44 | 1,68 | 7,62 | 0,9 | 737,0 | 15,0 | 47,13 | | 370,0 | | 37,0 | 44,0 | 99,0 | 0,87 | 0,59 | 1,48 | | 34,9 | 157,0 | 9,40 | 17,00 |
| Patient n°169 | F | 2 | 22-mars-05 | 1951 | 9,65 | 15,0 | 15,0 | 15,0 | | 0,64 | 1,49 | | 0,5 | 614,0 | 47,0 | 11,06 | | | | 58,0 | 54,0 | | | | | | | | 10,70 | 16,00 |
| Patient n°170 | M | 1 | 13-juil-04 | 1954 | 6,05 | 15,0 | | | | 0,40 | 2,48 | 6,05 | 1,3 | 982,0 | 54,0 | 16,19 | | 367,0 | | 42,8 | | | | | | | 163,5 | | | |
| Patient n°170 | M | 2 | 8-févr-05 | 1954 | 8,59 | 15,8 | 15,8 | 15,8 | | 0,54 | 2,64 | 5,98 | 0,5 | 1094,0 | 87,0 | 10,57 | | 335,0 | | 61,0 | 59,0 | | | | | | | | | |
| Patient n°171 | F | 1 | 7-juin-05 | 1968 | 8,46 | 13,8 | 13,8 | 13,8 | 1,44 | 0,61 | 1,90 | 7,26 | 3,6 | 874,0 | 119,0 | 5,34 | | 420,0 | 430,0 | 49,0 | | 160,0 | 1,15 | 0,77 | 1,49 | 271,0 | | | | |
| Patient n°172 | F | 1 | 12-oct-04 | 1947 | 6,18 | 15,8 | | 15,8 | | 0,39 | 2,45 | 6,45 | 0,3 | 1203,0 | 2,0 | 599,50 | | 307,0 | | 58,0 | 54,0 | | | | | 183,0 | | | | |
| Patient n°173 | M | 1 | 10-mai-05 | 1948 | 9,38 | 14,3 | 14,3 | | 1,38 | 0,66 | 1,87 | 7,65 | 1,6 | 945,0 | 56,0 | 14,88 | | 295,0 | | 43,5 | 46,0 | 54,0 | | | | 194,0 | 38,8 | | | |
| Patient n°174 | F | 1 | 14-déc-04 | 1931 | 15,16 | 12,4 | | 12,4 | | 1,22 | 1,65 | 7,52 | | 842,0 | 42,0 | 18,05 | | 327,0 | | 62,4 | | 46,0 | 0,92 | 0,66 | 1,39 | 218,0 | | 448,5 | | |
| Patient n°175 | M | 1 | 22-oct-03 | 1955 | 8,70 | 8,2 | | 8,2 | | 1,06 | 1,95 | 4,20 | 11,1 | 1047,2 | 15,8 | 64,19 | 44,0 | 486,3 | 365,0 | 35,8 | 71,0 | 80,0 | | | | | 189,7 | | | 37,93 |

13

| Patient | Sexe | N° | Date | Année | V1 | V2 | V3 | V4 | V5 | V6 | V7 | V8 | V9 | V10 | V11 | V12 | V13 | V14 | V15 | V16 | V17 | V18 | V19 | V20 | V21 | V22 | V23 | V24 | V25 | V26 |
|---|---|---|---|---|---|---|---|---|---|---|---|---|---|---|---|---|---|---|---|---|---|---|---|---|---|---|---|---|---|---|
| Patient n°176 | M | - | 14-avr-04 | 1953 | 10.58 | 16.5 | | 16.5 | | 0.64 | 2.32 | 16.50 | 0.2 | 806.2 | 50.7 | 13.89 | 58.0 | 415.4 | 550.0 | 50.0 | 54.0 | 123.0 | 0.89 | 0.69 | 1.29 | | 103.9 | 101.9 | 11.62 | 33.71 |
| Patient n°177 | F | - | 13-janv-03 | 1949 | 14.73 | 8.0 | | 8.0 | | 1.84 | 1.66 | 4.82 | 55.1 | 807.5 | 55.2 | 12.63 | 89.0 | 264.2 | 4.8 | 27.1 | 37.0 | 102.0 | 0.72 | 0.58 | 1.24 | 290.3 | 121.0 | 90.5 | | 10.61 |
| Patient n°177 | F | - | 15-juil-03 | 1949 | 10.50 | 12.2 | | 12.2 | | 0.86 | 1.61 | 7.58 | | 1150.4 | 7.2 | 158.01 | | 332.4 | 110.0 | 44.2 | 30.0 | | | | | | 115.7 | | | |
| Patient n°178 | F | 2 | 12-mai-03 | 1918 | 0.65 | 15.9 | | 15.9 | 0.63 | 0.04 | 2.52 | 6.31 | 24.5 | 1225.1 | 2.9 | 420.43 | 67.0 | 396.8 | 195.0 | 64.2 | 54.0 | 117.0 | 1.70 | 0.69 | 2.46 | 647.7 | 102.9 | 918.2 | | 13.05 |
| Patient n°179 | M | - | 29-sept-04 | 1929 | 9.14 | 13.7 | | 13.7 | | 0.67 | 2.21 | 6.20 | 1.0 | 626.0 | 55.0 | 9.38 | | 341.0 | 595.0 | 46.0 | 51.0 | | | | | 317.0 | | | | |
| Patient n°179 | M | 2 | 28-juin-05 | 1929 | 7.83 | | 15.6 | | | 0.50 | 0.90 | 17.33 | 1.8 | 597.0 | 47.0 | 10.70 | | 272.0 | | 63.0 | | | | | | 198.0 | | | | |
| Patient n°180 | M | - | 25-août-03 | 1943 | 7.76 | 21.1 | | 21.1 | | 0.37 | 2.60 | 8.11 | 91.7 | 1025.8 | 159.2 | 4.44 | 51.0 | 453.0 | 210.0 | 58.9 | 60.0 | 113.0 | 0.62 | 0.63 | 0.98 | | 279.0 | 164.1 | | |
| Patient n°180 | M | 2 | 3-oct-03 | 1943 | 0.12 | 29.5 | | 29.5 | | 0.00 | 3.58 | 8.24 | | 1190.0 | 138.9 | 6.57 | 41.0 | 436.3 | 345.0 | 66.0 | 52.0 | | | | | | 157.6 | | | 4.39 |
| Patient n°180 | M | 3 | 23-juin-04 | 1943 | 4.78 | 24.2 | | 24.2 | | 0.20 | 2.98 | 8.12 | 0.6 | 780.0 | 46.0 | 14.96 | 38.0 | 366.0 | | | 58.0 | | | | | | 81.9 | | 18.64 | |
| Patient n°181 | M | - | 17-févr-04 | 1941 | 2.22 | 15.0 | | 15.0 | | 0.15 | 1.56 | 9.61 | 0.1 | 1320.7 | 109.7 | 10.04 | 55.0 | 444.6 | 425.0 | 35.1 | 53.0 | 106.0 | 0.94 | 0.73 | 1.29 | | 89.5 | 147.9 | | 13.05 |
| Patient n°182 | M | - | 9-juin-04 | 1939 | 12.79 | 11.2 | | 11.2 | | 1.14 | 1.89 | 5.93 | 0.5 | 988.0 | 5.0 | 195.60 | | 370.0 | 345.0 | 13.8 | | | | | | | 79.7 | | | |
| Patient n°183 | F | - | 21-janv-04 | 1940 | 14.02 | 20.6 | | 20.6 | | 0.68 | 2.92 | 6.26 | 0.4 | 808.8 | 16.4 | 47.44 | 41.0 | 353.4 | 415.0 | 55.2 | 56.0 | 106.0 | 1.02 | 0.57 | 1.79 | | 108.5 | 153.2 | 11.49 | 18.39 |
| Patient n°184 | F | - | 4-mai-04 | 1949 | 11.32 | 15.2 | | 15.2 | | 0.74 | 2.43 | 6.86 | | 937.6 | 47.4 | 17.78 | 38.0 | 356.3 | 95.0 | 47.4 | 24.0 | 166.0 | | | | | 131.4 | | | |
| Patient n°184 | F | 2 | 10-nov-04 | 1949 | 7.27 | 15.1 | | 15.1 | | 0.48 | 2.20 | 12.49 | 3.2 | 940.0 | 67.0 | 12.03 | 46.0 | 345.0 | 495.0 | 67.0 | 30.0 | | | | | | 43.0 | | | |
| Patient n°185 | M | - | 27-juil-04 | 1936 | 4.06 | 27.6 | | 27.6 | | 0.15 | 2.21 | 7.78 | 0.5 | 958.0 | 101.0 | 7.49 | 48.0 | 359.0 | 390.0 | 47.6 | 42.0 | 169.0 | 1.00 | 0.80 | 1.25 | | 61.8 | #### | | |
| Patient n°185 | M | 2 | 20-oct-04 | 1936 | 10.99 | 13.0 | 16.4 | 13.0 | | 0.85 | 1.67 | 11.44 | 1.3 | 1004.0 | 76.0 | 11.21 | 64.0 | 310.0 | 540.0 | 40.0 | 39.0 | 123.0 | 0.80 | 0.63 | 1.27 | | 41.0 | #### | 6.79 | 7.47 |
| Patient n°186 | F | - | 17-févr-04 | 1947 | 14.66 | 13.5 | | 13.5 | | 1.09 | 1.18 | 6.44 | 0.2 | 1001.1 | 65.8 | 13.23 | 49.0 | 303.0 | 320.0 | 37.6 | 36.0 | 72.0 | 0.92 | 0.52 | -1.00 | | 47.7 | #### | 12.00 | 23.00 |
| Patient n°187 | F | - | 27-juil-04 | 1937 | 7.62 | 15.4 | | 15.4 | | 0.49 | 2.39 | 5.97 | 0.7 | 803.0 | 38.0 | 19.13 | 65.0 | 377.0 | 525.0 | 30.1 | 63.0 | 79.0 | 0.77 | 0.52 | 1.43 | | 72.4 | #### | 13.89 | 12.50 |
| Patient n°188 | F | - | 25-mai-04 | 1956 | 9.48 | 14.1 | | 14.1 | | 0.67 | 2.36 | 8.97 | | 1074.3 | 47.7 | 20.51 | | 399.4 | 225.0 | 34.7 | 43.0 | 103.0 | | | | | 56.2 | | 14.58 | 23.33 |
| Patient n°188 | F | 2 | 14-déc-04 | 1956 | 12.76 | 14.8 | | 14.8 | | 0.86 | 1.65 | 6.20 | 0.3 | 814.0 | 10.0 | 79.40 | | 440.0 | | 48.0 | 36.0 | | | | | | | | 18.33 | 29.33 |
| Patient n°189 | M | - | 1-sept-04 | 1944 | 7.41 | 12.4 | | 12.4 | 0.76 | 0.60 | 2.00 | 8.28 | 0.8 | 766.0 | 15.0 | 49.07 | 83.0 | 337.0 | 480.0 | 47.4 | 66.0 | | | | | | 32.4 | | | 14.00 |
| Patient n°190 | F | - | 23-mars-05 | 1921 | 1.20 | | | | | 0.07 | 1.98 | 7.10 | 0.2 | 782.0 | 2.0 | 389.00 | | 256.0 | | 40.0 | | | 0.97 | 0.46 | 2.11 | 501.0 | | | | |
| Patient n°191 | F | - | 31-août-04 | 1950 | 6.50 | 13.2 | | 13.2 | | 0.49 | 1.86 | 13.56 | 0.7 | 1017.0 | 26.0 | 37.12 | | 269.0 | 342.5 | 47.5 | | | | | | | 33.4 | | | |
| Patient n°192 | M | - | 5-mai-04 | 1947 | 7.12 | 33.9 | | 33.9 | | 0.21 | 2.50 | 12.01 | | 717.2 | 76.2 | 7.41 | | 414.9 | 230.0 | | 43.0 | 163.0 | 0.99 | 0.76 | 1.30 | | 404.1 | | | |
| Patient n°192 | M | 2 | 14-juin-05 | 1947 | 6.01 | | 37.0 | | | 0.16 | 3.08 | | 0.3 | 369.0 | 30.0 | 10.30 | | 432.0 | | 66.0 | | | | | | | 81.0 | | | |
| Patient n°193 | F | - | 8-févr-05 | 1951 | 10.89 | 11.7 | 11.7 | 11.7 | | 0.93 | 1.80 | 6.50 | 0.8 | 897.0 | 140.0 | 4.41 | | 366.0 | | 52.0 | 55.0 | | | | | 199.0 | | | | |
| Patient n°194 | F | - | 9-févr-05 | 1939 | 11.57 | 20.3 | 20.3 | 20.3 | 1.11 | 0.57 | 2.44 | | 1.0 | 977.0 | 57.0 | 15.14 | | | | 68.4 | | | | | | 87.0 | | | | 5.60 |

| Patient | Sex | N° | Date | Year | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|
| Patient n°194 | F | 2 | 5-juil-05 | 1939 | 10.51 | | 19.4 | 19.4 | | 0.54 | 2.34 | 8.29 | 2.7 | 897.0 | 16.0 | 54.06 | | 347.0 | | 70.0 | | | | | | 341.0 | | | | |
| Patient n°195 | M | 1 | 9-mars-05 | 1967 | 8.01 | | 10.9 | 10.9 | 0.58 | 0.73 | 1.76 | 6.19 | 0.1 | 966.0 | 17.0 | 54.82 | | | | 34.0 | | | | | | 114.0 | | | | |
| Patient n°196 | F | 1 | 23-nov-04 | 1943 | 14.32 | 12.5 | | 12.5 | | 1.15 | 2.09 | 5.98 | 0.4 | 1131.0 | 13.0 | 85.00 | | 333.0 | | 57.0 | 31.0 | | | | | 377.0 | | | | |
| Patient n°197 | M | 1 | 11-janv-05 | 1964 | 9.15 | 16.0 | | 16.0 | | 0.57 | 1.41 | 11.35 | 0.7 | 813.0 | 28.0 | 27.04 | 47.0 | 556.0 | | 52.4 | 35.0 | 119.0 | 0.71 | 0.54 | 1.32 | | 27.9 | | | 7.22 |
| Patient n°198 | M | 1 | 22-déc-04 | 1974 | 7.91 | 9.9 | | 9.9 | | 0.80 | 1.46 | 6.78 | 0.2 | 770.0 | 17.0 | 43.29 | 49.0 | 417.0 | | 28.2 | 63.0 | 92.0 | 1.20 | 0.76 | 1.58 | | 43.3 | 306.0 | | 19.22 |
| Patient n°199 | F | 1 | 12-mai-04 | 1946 | 13.09 | 12.2 | | 12.2 | | 1.07 | 1.47 | 8.30 | | 960.4 | 85.8 | 9.19 | 54.0 | 374.1 | 310.0 | 42.4 | 48.0 | 127.0 | | | | | 164.3 | | | |
| Patient n°200 | M | 1 | 19-mai-04 | 1946 | 9.72 | 12.6 | | 12.6 | | 0.77 | 1.78 | 7.08 | | 913.1 | 9.2 | 97.57 | 49.0 | 364.3 | 200.0 | 29.8 | 49.0 | 118.0 | | | | | 47.4 | | | |
| Patient n°201 | F | 1 | 19-oct-04 | 1939 | 10.60 | 11.3 | | 11.3 | | 0.94 | 2.16 | 5.23 | 1.7 | 981.0 | 114.0 | 6.61 | 44.0 | 312.0 | 910.0 | 35.0 | 60.0 | 89.0 | 1.11 | 0.59 | 1.88 | | 69.0 | 67.0 | | 9.00 |
| Patient n°201 | F | 2 | 29-mars-05 | 1939 | 9.64 | | 15.6 | 15.6 | 0.97 | 0.62 | 1.96 | | 0.8 | 1258.0 | 92.0 | 11.67 | 76.0 | 278.0 | | 47.0 | 60.0 | 93.0 | 0.70 | 0.46 | 1.52 | 376.0 | 55.0 | 220.0 | 12.00 | 18.00 |
| Patient n°202 | F | 1 | 27-oct-04 | 1948 | 16.30 | 13.2 | | 13.2 | | 1.23 | 2.26 | 5.84 | 0.4 | 925.0 | 52.0 | 15.79 | | 357.0 | 315.0 | 48.0 | 32.0 | | | | | 529.0 | | | | |
| Patient n°203 | M | 1 | 9-juin-04 | 1950 | 6.74 | 15.6 | | 15.6 | | 0.43 | 2.34 | 6.67 | 0.2 | 931.0 | 10.0 | 91.10 | | 360.0 | 615.0 | 20.7 | | | | | | | 120.2 | | | |
| Patient n°204 | F | 1 | 9-juin-04 | 1954 | 7.17 | 20.8 | | 20.8 | | 0.34 | 2.76 | 7.54 | 0.4 | 894.0 | 5.0 | 176.80 | 61.0 | 377.0 | 465.0 | 73.8 | 54.0 | 123.0 | 0.93 | 0.77 | 1.21 | | 117.3 | 222.0 | 4.20 | 1.68 |
| Patient n°205 | F | 1 | 6-déc-04 | 1931 | 12.70 | 15.6 | | 15.6 | | 0.81 | 1.86 | 8.39 | 0.3 | 308.0 | 65.0 | 2.74 | | 299.0 | | 60.0 | 34.0 | | | | | 417.0 | | | | |
| Patient n°206 | M | 1 | 4-janv-05 | 1931 | 7.90 | 15.0 | | 15.0 | | 0.53 | 1.87 | 8.02 | 0.2 | 1229.0 | 2.0 | 612.50 | | 305.0 | | 57.3 | 71.0 | | | | | 218.0 | | | | |
| Patient n°207 | M | 1 | 19-mai-04 | 1928 | 7.19 | 13.3 | | 13.3 | | 0.54 | 1.38 | 9.64 | | 1429.5 | 108.5 | 11.17 | 56.0 | 351.7 | 325.0 | 31.8 | 45.0 | 101.0 | | | | | 51.1 | | | |
| Patient n°208 | F | 1 | 27-avr-04 | 1954 | 8.72 | 19.6 | | 19.6 | | 0.44 | 1.77 | 11.07 | 0.3 | 840.4 | 140.0 | 4.00 | 67.0 | 449.7 | 215.0 | 74.4 | 29.0 | 138.0 | 0.97 | 0.85 | 1.14 | | 325.2 | 143.0 | 19.76 | 59.29 |
| Patient n°209 | F | 1 | 11-oct-04 | 1952 | 9.36 | 13.1 | | 13.1 | | 0.71 | 2.23 | 5.87 | 1.5 | 760.0 | 26.0 | 27.23 | | 371.0 | 330.0 | | 33.0 | | | | | | | | 8.00 | 12.00 |
| Patient n°210 | F | 1 | 23-mars-05 | 1926 | 10.58 | | 13.3 | 13.3 | 0.79 | 0.80 | 1.24 | 10.73 | 0.5 | 622.0 | 6.0 | 101.67 | | 304.0 | | 34.0 | | | 0.89 | 0.43 | 2.07 | 341.0 | | | | |
| Patient n°211 | F | 1 | 6-juil-04 | 1989 | 10.38 | 14.4 | | 14.4 | | 0.72 | 2.33 | 6.18 | 0.5 | 810.0 | 100.0 | 6.10 | 69.0 | 468.0 | | | 46.0 | 103.0 | 0.91 | 0.65 | 1.40 | | 84.4 | 827.0 | 11.90 | 22.62 |
| Patient n°212 | F | 2 | 21-déc-04 | 1989 | 12.28 | 17.6 | | 17.6 | | 0.70 | 2.14 | 8.22 | 1.8 | 891.0 | 9.0 | 97.00 | | 277.0 | | 37.1 | 52.0 | | | | | | | | | 16.70 |
| Patient n°213 | M | 1 | 5-oct-04 | 1947 | 8.78 | 16.3 | | 16.3 | | 0.54 | 2.02 | 8.07 | 0.6 | 868.0 | 57.0 | 13.23 | 45.0 | 329.0 | 990.0 | 58.0 | 40.0 | 140.0 | 0.97 | 0.86 | 1.13 | | 64.0 | 174.0 | 15.30 | 23.00 |
| Patient n°214 | M | 1 | 12-nov-03 | 1950 | 10.61 | 8.6 | | 8.6 | | 1.23 | 1.60 | 5.38 | 61.9 | 1091.6 | 67.7 | 14.11 | 66.0 | | 160.0 | | 46.0 | 89.0 | 0.80 | 0.71 | 1.13 | | 156.3 | 55.5 | 8.31 | 6.65 |
| Patient n°214 | M | 2 | 1-juin-04 | 1950 | 8.23 | 10.8 | | 10.8 | | 0.76 | 1.69 | 6.39 | | 1124.0 | 39.1 | 26.74 | | 522.0 | 290.0 | 32.7 | 45.0 | | | | | | 54.3 | | | |
| Patient n°215 | F | 1 | 21-avr-04 | 1930 | 8.57 | 12.2 | | 12.2 | | 0.70 | 2.32 | 5.26 | | 650.7 | 8.4 | 75.10 | | 409.0 | 555.0 | 28.2 | 63.0 | | | | | | 397.3 | | | |
| Patient n°215 | F | 2 | 7-sept-04 | 1930 | 12.72 | 13.0 | | 13.0 | | 0.98 | 2.19 | 5.90 | 0.9 | 999.0 | 34.0 | 27.38 | | 257.0 | 475.0 | 66.0 | 66.0 | | | | | | 54.7 | | | |
| Patient n°216 | F | 1 | 7-sept-04 | 1947 | 2.23 | 20.8 | | 20.8 | | 0.11 | 2.10 | 10.00 | 0.2 | 1109.0 | 25.0 | 42.36 | | 256.0 | 445.0 | 71.0 | 54.0 | | | | | | 88.0 | | | |
| Patient n°217 | F | 1 | 31-mars-04 | 1946 | 7.31 | 17.1 | | 17.1 | | 0.43 | 2.51 | 6.81 | 0.4 | 882.6 | 64.5 | 11.69 | 62.0 | 440.8 | 145.0 | 42.0 | 52.0 | 102.0 | 1.03 | 0.59 | 1.75 | | 112.9 | 86.0 | 8.60 | 17.13 |

| Patient | | | Date | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|
| Patient n°217 | F | 2 | 13-sept-04 | 1946 | 6,53 | 17,8 | | 17,8 | | 0,37 | 2,15 | 8,28 | 0,6 | 685,0 | 22,0 | 29,14 | | 395,0 | 435,0 | 66,2 | 45,0 | | | 0,63 | | | 51,5 | | | |
| Patient n°218 | F | 1 | 23-mai-03 | 1944 | 9,78 | 10,5 | | 10,5 | | 0,93 | 1,71 | 6,14 | 44,9 | 1633,8 | 52,9 | 28,90 | 42,0 | 432,2 | 195,0 | 66,7 | 43,0 | 98,0 | 1,29 | 0,70 | 1,84 | | 138,0 | 338,4 | | |
| Patient n°219 | F | 1 | 18-mai-05 | 1972 | 10,22 | | 13,3 | 13,3 | 0,68 | 0,77 | 1,69 | 7,90 | 1,3 | 863,0 | 132,0 | 4,54 | | 389,0 | | 40,0 | | | | | | 394,0 | | | |
| Patient n°220 | M | 1 | 28-juin-05 | 1973 | 10,31 | | 8,6 | 8,6 | 0,50 | 1,20 | 1,76 | 4,89 | 0,3 | 822,0 | 183,0 | 2,49 | | 383,0 | | 40,0 | | | | | | 166,0 | | | |
| Patient n°221 | F | 1 | 15-sept-04 | 1943 | 11,78 | 17,5 | | 17,5 | | 0,67 | 2,02 | 8,66 | 1,4 | 735,0 | 54,0 | 11,61 | 50,0 | 278,0 | 322,5 | 69,3 | 41,0 | 73,0 | 0,75 | 0,64 | 1,17 | | 26,5 | 398,0 | 19,00 | 11,35 |
| Patient n°221 | F | 2 | 8-févr-05 | 1943 | 12,65 | | 15,1 | 15,1 | 0,84 | 2,02 | 7,47 | 1,9 | 954,0 | 103,0 | 7,26 | | 373,0 | | 54,0 | | | | | | 47,0 | | | 0,50 |
| Patient n°222 | M | 1 | 6-oct-04 | 1941 | 10,09 | 12,3 | | 12,3 | | 0,82 | 2,13 | 5,78 | 0,6 | 835,0 | 102,0 | 6,68 | | 316,0 | 240,0 | 60,0 | 41,0 | | | | | | 348,0 | | | |
| Patient n°222 | M | 2 | 22-mars-05 | 1941 | 9,68 | | 14,7 | 14,7 | 0,97 | 0,66 | 1,89 | 7,78 | 1,6 | 773,0 | 8,0 | 94,63 | | | | 36,0 | | | | | | 367,0 | | | |
| Patient n°223 | F | 1 | 26-oct-04 | 1935 | 13,53 | 15,9 | | 15,9 | | 0,85 | 2,04 | 7,79 | 0,5 | 955,0 | 135,0 | 5,07 | | | 555,0 | 65,0 | 47,0 | | | | | | 373,0 | | | |
| Patient n°224 | M | 1 | 21-sept-04 | 1960 | 12,07 | 12,1 | | 12,1 | | 1,00 | 1,96 | 6,17 | 0,9 | 685,0 | 53,0 | 10,92 | | 347,0 | 535,0 | 51,1 | 54,0 | | | | | | 32,4 | | | |
| Patient n°225 | F | 1 | 21-sept-04 | 1946 | 15,74 | 15,8 | | 15,8 | | 1,00 | 2,02 | 7,82 | 0,8 | 1138,0 | 67,0 | 14,99 | | 324,0 | 270,0 | 65,7 | 40,0 | 122,0 | 0,99 | 0,82 | 1,21 | | 35,4 | | | |
| Patient n°226 | F | 1 | 1-sept-04 | 1963 | 4,51 | 11,3 | | 11,3 | | 0,40 | 2,05 | 5,51 | 0,7 | 925,0 | 65,0 | 12,23 | | 391,0 | 420,0 | 37,7 | 36,0 | | | | | | 25,7 | | | |
| Patient n°227 | M | 1 | 23-juin-03 | 1930 | 1,12 | 19,4 | | 19,4 | | 0,06 | 2,42 | 8,02 | 82,8 | 1033,5 | 84,9 | 10,18 | 54,0 | 304,9 | 255,0 | 62,9 | 61,0 | 100,0 | 0,92 | 0,61 | 1,51 | | 896,5 | 465,7 | 11,86 | 15,42 |
| Patient n°228 | M | 1 | 14-sept-04 | 1943 | 7,37 | 12,5 | | 12,5 | | 0,59 | 1,70 | 7,35 | 0,5 | 778,0 | 44,0 | 15,68 | | 278,0 | 382,5 | 40,8 | 54,0 | | | | | | 28,3 | | | |
| Patient n°229 | F | 1 | 14-sept-04 | 1946 | 11,50 | 14,8 | | 14,8 | | 0,78 | 2,00 | 7,40 | 0,7 | 732,0 | 23,0 | 29,83 | | 308,0 | 492,5 | 64,9 | 28,0 | | | | | | 40,7 | | | |
| Patient n°230 | F | 1 | 4-janv-05 | 1946 | 8,86 | 15,2 | | 15,2 | | 0,58 | 2,40 | 6,33 | 0,2 | 710,0 | 11,0 | 62,55 | | 327,0 | | 61,3 | 57,0 | | | | | | | | | 25,80 |
| Patient n°231 | M | 1 | 7-déc-04 | 1925 | 14,71 | 13,5 | | 13,5 | | 1,09 | 2,34 | | 0,5 | 1038,0 | 2,0 | 517,00 | | 338,0 | | 29,3 | 43,0 | 90,0 | 1,07 | 0,63 | 1,70 | | 429,0 | | | |
| Patient n°231 | M | 2 | 3-mai-05 | 1925 | 11,65 | | 16,1 | 16,1 | 2,28 | 0,72 | 2,39 | | 2,4 | 1123,0 | 111,0 | 8,12 | | | | 68,0 | | | | | | 281,0 | | | |
| Patient n°232 | F | 1 | 27-avr-04 | 1964 | 3,42 | 8,9 | | 8,9 | | 0,38 | 1,54 | 5,78 | | 1178,8 | 127,5 | 7,25 | | 462,3 | 200,0 | 24,2 | 39,0 | | | | | | 174,0 | | | |
| Patient n°233 | M | 1 | 14-avr-04 | 1931 | 3,79 | 13,1 | | 13,1 | | 0,29 | 2,15 | 6,09 | 0,3 | 1029,0 | 95,3 | 8,80 | 32,0 | 316,3 | 605,0 | 30,4 | 50,0 | 98,0 | 0,92 | 0,68 | 1,35 | | 99,6 | 454,3 | 11,10 | 9,99 |
| Patient n°233 | M | 2 | 29-sept-04 | 1931 | 6,39 | 12,3 | | 12,3 | | 0,52 | 2,14 | 5,75 | 0,5 | 1239,0 | 84,0 | 12,75 | | 341,0 | 900,0 | 49,0 | 43,0 | | | | | | 85,0 | | | |
| Patient n°234 | F | 1 | 29-sept-04 | 1938 | 6,31 | 19,9 | | 19,9 | | 0,32 | 2,99 | 6,66 | 0,5 | 769,0 | 122,0 | 4,30 | | 385,0 | 790,0 | 75,0 | 64,0 | | | | | | 419,0 | | | |
| Patient n°235 | F | 1 | 10-août-04 | 1943 | 9,54 | 15,4 | | 15,4 | | 0,62 | 2,15 | 7,16 | 0,4 | 895,0 | 64,0 | 11,98 | 76,0 | 376,0 | 452,5 | 45,0 | 49,0 | 129,0 | 1,42 | 0,82 | 1,73 | | 106,1 | 185,0 | 9,25 | 6,48 |
| Patient n°236 | F | 1 | 26-mai-04 | 1963 | 9,41 | 12,1 | | 12,1 | | 0,78 | 2,43 | 4,98 | | 1255,3 | 61,3 | 18,47 | 77,0 | 406,2 | 205,0 | 65,7 | 36,0 | 113,0 | 0,85 | 0,72 | 1,18 | | 51,5 | | | |
| Patient n°236 | F | 2 | 14-déc-04 | 1963 | 13,31 | 11,1 | | 11,1 | | 1,20 | 2,21 | 5,02 | 1,4 | 1045,0 | 22,0 | 45,50 | | 395,0 | | 47,0 | 40,0 | | | | | | 265,0 | | | |
| Patient n°237 | F | 1 | 3-janv-03 | 1970 | 8,23 | 13,1 | | 13,1 | | 0,63 | 2,10 | 6,24 | 36,2 | 1275,5 | 86,3 | 12,78 | 60,0 | 430,7 | 3,4 | 46,7 | 39,0 | 113,0 | 0,82 | 0,55 | 1,49 | | 217,8 | 100,9 | 9,53 | 11,44 |
| Patient n°238 | M | 1 | 15-sept-04 | 1931 | 10,05 | 13,2 | | 13,2 | | 0,76 | 1,62 | 8,15 | 0,6 | 709,0 | 127,0 | 3,58 | 55,0 | 284,0 | 740,0 | 53,7 | 50,0 | 83,0 | 0,68 | 0,57 | 1,19 | | 34,0 | 1112,0 | 14,60 | 13,17 |

| Patient | Sexe | N° | Date | Année | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|
| Patient n°238 | M | 2 | 9-févr-05 | 1931 | 10.91 | | 14.5 | 14.5 | 0.54 | 0.75 | 1.60 | 9.06 | 0.6 | 1145.0 | 123.0 | 7.31 | | | | 46.0 | | | | | | 84.0 | | | | |
| Patient n°239 | F | 1 | 14-juin-05 | 1946 | 7.44 | | 10.9 | 10.9 | 0.74 | 0.68 | 1.93 | 5.65 | 0.5 | 649.0 | 12.0 | 52.08 | | 328.0 | | 36.0 | | | | | | 425.0 | | | | |
| Patient n°240 | M | 1 | 5-juil-05 | 1947 | 10.53 | | 17.8 | 17.8 | 0.53 | 0.59 | 1.93 | 9.22 | 0.8 | 815.0 | 54.0 | 13.09 | 35.0 | 380.0 | | 59.0 | 84.0 | 170.0 | 1.05 | 0.66 | 1.59 | 291.0 | 43.0 | 105.0 | 3.00 | 5.00 |
| Patient n°241 | F | 1 | 21-avr-04 | 1944 | 9.57 | 12.1 | | 12.1 | | 0.79 | 1.95 | 6.20 | 0.4 | 976.8 | 51.8 | 16.85 | 54.0 | 444.6 | 245.0 | 28.7 | 39.0 | 23.4 | 0.45 | 0.38 | 1.18 | | 296.0 | 153.7 | 6.78 | 4.07 |
| Patient n°241 | F | 2 | 5-oct-04 | 1944 | 3.98 | 12.7 | | 12.7 | | 0.31 | 0.83 | 15.30 | 0.5 | 697.0 | 44.0 | 13.84 | | 361.0 | 640.0 | 56.0 | 15.0 | | | | | | 38.0 | | | |
| Patient n°242 | M | 1 | 15-sept-04 | 1956 | 13.65 | 11.5 | | 11.5 | | 1.19 | 2.09 | 5.50 | 1.0 | 821.0 | 40.0 | 18.53 | 28.0 | 259.0 | 502.5 | 33.3 | 43.0 | 86.0 | 0.82 | 0.58 | 1.41 | | 35.8 | 262.0 | 19.70 | 45.30 |
| Patient n°243 | M | 1 | 27-avr-04 | 1939 | 11.56 | 17.3 | | 17.3 | | 0.67 | 1.98 | 8.74 | | 899.1 | 91.7 | 7.81 | | 384.6 | 780.0 | 66.7 | 76.0 | | | | | | 368.9 | | | |
| Patient n°244 | F | 1 | 20-déc-03 | 1921 | 7.96 | 12.9 | | 12.9 | | 0.62 | 1.87 | 6.90 | 153.2 | 1027.7 | 13.8 | 72.53 | 47.0 | 475.6 | 8.8 | 52.0 | 55.0 | 73.0 | 1.18 | 0.62 | 1.90 | | 34.8 | 171.5 | 11.71 | 26.93 |
| Patient n°245 | F | 1 | 25-août-03 | 1950 | 8.03 | 14.4 | | 14.4 | | 0.56 | 1.66 | 8.68 | 38.0 | 811.8 | 111.5 | 5.28 | 42.0 | 431.5 | 211.0 | 50.0 | 40.0 | 78.0 | 1.05 | 0.47 | 2.23 | 79.4 | 586.9 | 4.47 | 5.81 | |
| Patient n°246 | F | 1 | 29-nov-04 | 1951 | 8.65 | 16.3 | | 16.3 | | 0.53 | 2.78 | | 1.7 | 916.0 | 23.0 | 37.83 | | 340.0 | | 54.0 | 25.0 | | | | | | 351.0 | | | |
| Patient n°246 | F | 2 | 17-mai-05 | 1951 | 10.30 | | 19.4 | 19.4 | | 0.53 | 2.48 | 7.82 | 2.2 | 912.0 | 56.0 | 14.29 | | 372.0 | | 64.0 | | | | | | | 378.0 | | | |
| Patient n°247 | M | 1 | 7-sept-04 | 1953 | 8.11 | 17.0 | | 17.0 | | 0.48 | 2.10 | 8.09 | 0.9 | 1349.0 | 58.0 | 21.26 | | 326.0 | 330.0 | 56.0 | 50.0 | | | | | | 79.8 | | | |
| Patient n°248 | F | 1 | 6-déc-04 | 1931 | 6.82 | 17.2 | | 17.2 | | 0.40 | 1.80 | | 0.7 | 802.0 | 2.0 | 399.00 | | 428.0 | | 36.0 | | | | | | | 626.0 | | | |
| Patient n°249 | M | 1 | 5-oct-04 | 1931 | 14.23 | 39.6 | | 39.6 | | 0.36 | 2.36 | 16.78 | 0.7 | 779.0 | 29.0 | 24.86 | 49.0 | 289.0 | 1065.0 | 92.0 | 48.0 | 196.0 | 0.98 | 0.60 | 1.63 | | 73.0 | 111.0 | 23.00 | 14.00 |
| Patient n°250 | F | 1 | 5-oct-04 | 1953 | 11.91 | 16.0 | | 16.0 | | 0.74 | 1.94 | 16.00 | 0.9 | 795.0 | 58.0 | 11.71 | | 350.0 | 385.0 | 70.0 | 19.0 | | | | | | 289.0 | | | |
| Patient n°251 | M | 1 | 22-mai-03 | 1957 | 9.27 | 12.9 | | 12.9 | | 0.72 | 2.21 | 5.84 | 156.5 | 1229.5 | 61.3 | 18.07 | 65.0 | 342.0 | 385.0 | 71.6 | 64.0 | 115.0 | 1.29 | 0.64 | 2.02 | | 152.7 | 26.8 | 27.00 | 21.60 |
| Patient n°251 | M | 2 | 14-oct-03 | 1957 | 11.96 | 14.6 | | 14.6 | | 0.82 | 1.74 | 8.39 | | 737.8 | 112.5 | 4.56 | | 400.7 | 300.0 | 39.8 | 77.0 | | | | | | 183.6 | | 48.88 | 171.08 |
| Patient n°251 | M | 3 | 9-mars-04 | 1957 | 13.21 | 10.4 | | 10.4 | | 1.27 | 1.72 | 6.05 | | 822.0 | 121.2 | 4.78 | | 229.9 | 580.0 | 16.7 | 65.0 | | | | | | 87.5 | | 28.50 | 39.92 |
| Patient n°251 | M | 4 | 25-oct-04 | 1957 | 8.44 | 11.5 | | 11.5 | | 0.73 | 1.79 | 6.43 | 1.2 | 1088.0 | 36.0 | 28.22 | | 364.0 | 1240.0 | 45.0 | 93.0 | | | | | | | | 36.00 | 71.00 |
| Patient n°252 | M | 1 | 1-sept-04 | 1965 | 7.29 | 13.8 | | 13.8 | | 0.53 | 1.83 | 7.54 | 1.1 | 957.0 | 53.0 | 16.06 | | 361.0 | 257.5 | 47.8 | 29.0 | | | | | | 24.7 | | | |
| Patient n°253 | F | 1 | 12-mai-03 | 1958 | 11.09 | 22.9 | | 22.9 | | 0.48 | 1.20 | 19.08 | 133.2 | 1059.4 | 2.5 | 415.10 | 60.0 | 438.4 | 160.0 | 72.0 | 19.0 | 129.0 | 1.60 | 0.61 | 2.62 | 584.3 | 67.9 | 863.3 | 16.53 | 4.96 |
| Patient n°254 | F | 1 | 13-avr-04 | 1958 | 9.58 | 14.0 | | 14.0 | | 0.68 | 1.79 | 7.93 | | 940.4 | 138.2 | 4.81 | | 477.3 | 55.0 | 34.7 | 31.0 | | | | | | 80.0 | | | |
| Patient n°255 | F | 1 | 21-sept-04 | 1940 | 7.10 | 21.8 | | 21.8 | | 0.33 | 1.47 | 14.83 | 0.9 | 757.0 | 75.0 | 8.09 | | 307.0 | 375.0 | 26.0 | 26.0 | | | | | | | | 15.00 | 12.28 |
| Patient n°256 | M | 1 | 6-oct-04 | 1946 | 6.46 | 15.2 | | 15.2 | | 0.43 | 2.16 | 7.04 | 0.1 | 757.0 | 10.0 | 73.70 | 31.0 | 344.0 | 1115.0 | 63.0 | 73.0 | 110.0 | 0.74 | 0.69 | 1.07 | | 70.0 | 315.0 | | 11.00 |
| Patient n°257 | F | 1 | 25-juil-05 | 1946 | 13.71 | | 15.0 | 15.0 | 0.94 | 0.91 | 1.39 | 10.79 | 1.1 | 651.0 | 32.0 | 18.34 | | 301.0 | | 63.0 | | | | | | 222.0 | | | | |
| Patient n°258 | F | 1 | 10-mai-04 | 1929 | 11.49 | 19.1 | | 19.1 | | 0.60 | 2.40 | 7.96 | | 1129.7 | 99.3 | 9.38 | 45.0 | 384.9 | 330.0 | 50.7 | 51.0 | 106.0 | | | | | 159.6 | | | |
| Patient n°259 | F | 1 | 13-sept-04 | 1960 | 9.77 | 13.9 | | 13.9 | | 0.70 | 2.59 | 5.37 | 0.7 | 858.0 | 63.0 | 11.62 | | 292.0 | 150.0 | 59.0 | 80.0 | 107.0 | 0.68 | 0.57 | 1.19 | | 47.5 | | | |

EP 1 793 230 B1

| Patient | Sexe | N° | Date | Année | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|
| Patient n°260 | F | 1 | 4-nov-03 | 1951 | 13.88 | 14.6 | | 14.6 | | 0.95 | 2.07 | 7.05 | 36.0 | 802.2 | 121.6 | 4.60 | 69.0 | 414.9 | 250.0 | 48.4 | 45.0 | 108.0 | 0.95 | 0.75 | 1.27 | | 371.7 | 955.0 | 14.46 | 14.46 |
| Patient n°260 | F | 2 | 7-avr-04 | 1951 | 10.06 | 16.4 | | 16.4 | | 0.61 | 2.05 | 8.00 | | 914.7 | 89.2 | 8.25 | | 269.5 | 515.0 | 47.1 | 48.0 | | | | | | 101.6 | | | |
| Patient n°261 | F | 1 | 4-mai-04 | 1929 | 11.71 | 19.8 | | 19.8 | | 0.59 | 2.57 | 7.70 | | 866.9 | 19.0 | 43.55 | 32.0 | 282.9 | 255.0 | 64.7 | 37.0 | 133.0 | | | | | 137.8 | | | |
| Patient n°262 | F | 1 | 18-juin-03 | 1941 | 10.86 | 10.5 | | 10.5 | | 1.03 | 1.49 | 7.05 | | 1187.0 | 133.1 | 6.92 | | | 495.0 | 61.1 | | 83.0 | 0.81 | 0.68 | 1.19 | 206.1 | 304.1 | 1179.2 | | |
| Patient n°262 | F | 2 | 4-nov-03 | 1941 | 16.13 | 11.6 | | 11.6 | | 1.39 | 1.64 | 7.07 | | 662.4 | 9.7 | 66.36 | | 368.8 | 485.0 | 50.4 | 73.0 | | | | | | 457.0 | | | |
| Patient n°262 | F | 3 | 19-mai-04 | 1941 | 10.03 | 18.5 | | 18.5 | | 0.54 | 1.44 | 12.85 | | 906.0 | 12.1 | 72.81 | 80.0 | 332.2 | 605.0 | 55.3 | 55.0 | 134.0 | | | | | 85.9 | | | |
| Patient n°263 | F | 1 | 26-févr-03 | 1943 | 13.30 | 25.3 | | 25.3 | | 0.53 | 1.87 | 13.53 | 81.6 | 975.9 | 32.3 | 28.22 | 68.0 | 383.0 | 155.0 | 68.7 | 47.0 | 186.0 | 1.42 | 0.95 | 1.49 | 261.9 | 23.6 | 693.8 | 6.30 | 11.97 |
| Patient n°264 | F | 1 | 23-mars-05 | 1900 | 4.76 | | 11.7 | 11.7 | 0.54 | 0.41 | 1.52 | 7.70 | 0.3 | 818.0 | 5.0 | 161.60 | | 267.0 | | 27.0 | | | 0.81 | 0.49 | 1.65 | 373.0 | | | | |
| Patient n°265 | F | 1 | 19-avr-05 | 1936 | 16.65 | | 15.5 | 15.5 | 0.77 | 1.07 | 2.17 | 7.14 | 0.2 | 944.0 | 35.0 | 24.97 | | | | 30.0 | | 91.0 | 0.95 | 0.61 | 1.56 | 421.0 | | | | |
| Patient n°266 | F | 1 | 21-juin-05 | 1959 | 11.27 | | 13.2 | 13.2 | 1.01 | 0.85 | 1.86 | 7.10 | 0.8 | 733.0 | 42.0 | 15.45 | | 328.0 | | 37.0 | | | | | | 485.0 | | | | |
| Patient n°267 | F | 1 | 12-mai-03 | 1955 | 12.45 | 18.1 | | 18.1 | | 0.69 | 2.21 | 8.19 | | 1311.0 | 2.6 | 508.10 | | | 240.0 | 61.6 | | 128.0 | 1.19 | 0.91 | 1.31 | 396.7 | 52.4 | 918.2 | | |
| Patient n°268 | M | 1 | 1-mars-05 | 1946 | 5.07 | | 10.6 | 10.6 | 1.19 | 0.48 | 1.41 | 7.52 | 0.2 | 759.0 | 16.0 | 45.44 | | | | | | 94.0 | | | | 389.0 | | | | |
| Patient n°269 | F | 1 | 6-sept-04 | 1937 | 19.15 | 12.0 | | 12.0 | | 1.60 | 2.03 | 5.91 | 1.1 | 1426.0 | 104.0 | 11.71 | | 304.0 | 582.5 | 42.2 | 56.0 | | | | | | 72.6 | | | |
| Patient n°270 | M | 1 | 12-oct-04 | 1934 | 8.41 | 15.8 | | 15.8 | | 0.53 | 2.18 | 7.25 | 0.4 | 1035.0 | 88.0 | 9.76 | | 358.0 | 1230.0 | 60.0 | 94.0 | | | | | 94.0 | | | | |
| Patient n°271 | F | 1 | 13-avr-04 | 1937 | 9.94 | 12.9 | | 12.9 | | 0.77 | 3.10 | 4.16 | | 837.8 | 57.3 | 12.62 | | 485.4 | 170.0 | 37.6 | 35.0 | | | | | | 152.7 | | | |
| Patient n°272 | M | 1 | 25-août-03 | 1971 | | 11.5 | | 11.5 | | 0.00 | 2.26 | 5.09 | 9.9 | 1195.5 | 65.1 | 16.37 | 17.0 | 217.5 | 440.0 | 42.4 | 78.0 | 53.0 | 1.02 | 0.56 | 1.82 | | 192.7 | 71.5 | 26.44 | 37.02 |
| Patient n°273 | F | 1 | 25-août-03 | 1959 | 25.82 | 18.4 | | 18.4 | | 1.40 | 2.48 | 7.42 | 55.6 | 1123.7 | 13.6 | 80.57 | 41.0 | 453.1 | 100.0 | 50.4 | 54.0 | 70.0 | 1.00 | 0.69 | 1.45 | | 229.3 | 524.0 | 3.42 | 2.05 |
| Patient n°274 | F | 1 | 20-sept-04 | 1950 | 13.81 | 16.7 | | 16.7 | | 0.83 | 2.35 | 7.11 | 1.1 | 639.0 | 20.0 | 29.95 | 63.0 | 292.0 | 510.0 | 64.0 | 41.0 | 132.0 | 1.29 | 0.62 | 2.08 | | 49.3 | 309.0 | 9.20 | 4.60 |
| Patient n°275 | M | 1 | 16-juin-04 | 1938 | 7.52 | 19.9 | | 19.9 | | 0.38 | 2.08 | 9.57 | 0.3 | 655.0 | 57.0 | 9.49 | | 340.0 | 825.0 | 44.4 | 88.0 | | | | | | 55.2 | | | |
| Patient n°276 | F | 1 | 16-juin-04 | 1945 | 6.74 | 19.1 | | 19.1 | | 0.35 | 2.62 | 7.29 | 0.8 | 848.0 | 26.0 | 30.62 | | 501.0 | 245.0 | 43.1 | 40.0 | | | | | | 76.9 | | | |
| Patient n°277 | M | 1 | 6-déc-02 | 1955 | 7.96 | 7.7 | | 7.7 | | 1.03 | 1.81 | 4.25 | | 1048.2 | 3.2 | 323.52 | | | 7.3 | 51.1 | | 103.0 | | | | | 200.0 | 308.6 | | |
| Patient n°277 | M | 2 | 12-mai-03 | 1955 | 7.41 | 11.9 | | 11.9 | | 0.62 | 1.49 | 7.99 | | 1027.6 | 1.2 | 833.41 | | 451.2 | 240.0 | 58.0 | 40.0 | | | | | | 54.9 | | | |
| Patient n°278 | F | 1 | 2-nov-04 | 1947 | 9.54 | 12.8 | | 12.8 | | 0.75 | 1.84 | | 0.4 | 1117.0 | 112.0 | 7.97 | | 322.0 | 400.0 | 51.0 | 34.0 | | | | | 858.0 | | | | |
| Patient n°279 | M | 1 | 9-mars-05 | 1954 | 8.95 | | 9.1 | 9.1 | 0.91 | 0.98 | 1.84 | 4.95 | 0.3 | 938.0 | 77.0 | 10.18 | 49.0 | 359.0 | | 32.0 | 46.0 | 92.0 | 0.74 | 0.59 | 1.25 | 306.0 | 29.0 | 886.0 | | 12.00 |
| Patient n°280 | M | 1 | 14-oct-03 | 1928 | 12.53 | 12.4 | | 12.4 | | 1.01 | 2.24 | 5.54 | 38.4 | 901.2 | 116.3 | 5.75 | 62.0 | 421.3 | 110.0 | 39.3 | 52.0 | 63.0 | 1.27 | 0.64 | 1.98 | | 243.0 | 115.9 | 12.34 | 23.44 |
| Patient n°281 | F | 1 | 6-janv-04 | 1956 | 17.73 | 22.9 | | 22.9 | | 0.77 | 2.69 | 8.51 | 82.3 | 952.2 | 115.4 | 6.25 | 44.0 | 288.5 | 220.0 | 51.1 | 38.0 | 116.0 | 1.35 | 0.94 | 1.44 | | 61.2 | 259.5 | 12.00 | 27.60 |
| Patient n°282 | F | 1 | 18-juin-03 | 1956 | 14.88 | 8.5 | | 8.5 | | 1.75 | 1.75 | 4.86 | | 1058.5 | 4.5 | 233.75 | | | 210.0 | 59.8 | 39.0 | | | | | | 151.0 | 649.8 | | |

EP 1 793 230 B1

| Patient | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|
| Patient n°283 | F | I | 13-avr-04 | 1925 | 8.08 | 18.4 | | 18.4 | | 0.44 | 2.43 | 7.57 | | 962.4 | 62.4 | 13.43 | | 402.5 | 350.0 | 34.2 | 61.0 | | | | | | 119.9 | | | |
| Patient n°284 | F | I | 22-déc-04 | 1981 | 6.37 | 9.4 | | 9.4 | | 0.68 | 2.02 | | 0.2 | 829.0 | 10.0 | 80.90 | | 366.0 | | 21.8 | 31.0 | | | | | | 1122.0 | | | |
| Patient n°285 | F | I | 23-févr-05 | 1956 | 14.63 | | 14.3 | 14.3 | 0.65 | 1.02 | 1.61 | | 0.4 | 686.0 | 2.0 | 341.00 | | | | 55.1 | | | | | | | 565.0 | | | |
| Patient n°286 | F | I | 25-janv-05 | 1964 | 14.24 | 18.1 | | 18.1 | | 0.79 | 2.45 | 7.39 | 0.7 | 871.0 | 5.0 | 172.20 | | 320.0 | 55.6 | | 45.0 | | | | | | 219.0 | | | |
| Patient n°287 | F | I | 25-janv-05 | 1934 | 11.76 | 14.4 | | 14.4 | | 0.82 | 2.30 | 6.26 | 0.7 | 810.0 | 127.0 | 4.38 | | 322.0 | | 60.4 | 38.0 | | | | | | 200.0 | | | |
| Patient n°287 | F | 2 | 21-juin-05 | 1934 | 11.32 | | 15.4 | 15.4 | 1.16 | 0.74 | 2.43 | 6.34 | 0.8 | 744.0 | 84.0 | 6.86 | | 310.0 | | 52.0 | | | | | | | 345.0 | | | |
| Patient n°288 | F | I | 15-janv-04 | 1941 | 14.74 | 15.1 | | 15.1 | | 0.98 | 2.12 | 7.12 | 0.2 | 1058.4 | 43.2 | 22.49 | 69.0 | 390.9 | 185.0 | 46.7 | 43.0 | 87.0 | 0.85 | 0.86 | 0.99 | | 73.6 | 176.6 | 7.17 | 10.75 |
| Patient n°289 | F | I | 12-mai-04 | 1961 | 13.13 | 13.7 | | 13.7 | | 0.96 | 1.53 | 8.95 | | 719.9 | 86.1 | 6.36 | 41.0 | 401.4 | 215.0 | 32.9 | 29.0 | 114.0 | | | | | 101.2 | | | |
| Patient n°290 | M | I | 29-juin-04 | 1940 | 7.58 | 14.7 | | 14.7 | | 0.52 | 2.30 | 6.39 | 0.5 | 1000.0 | 96.0 | 8.42 | 60.0 | 409.0 | | | 55.0 | 84.0 | 0.87 | 0.59 | 1.48 | | 98.2 | 1005.0 | | 12.49 |
| Patient n°291 | F | I | 15-sept-04 | 1949 | 1.67 | 7.9 | | 7.9 | | 0.21 | 1.54 | 5.13 | 0.4 | 792.0 | 10.0 | 77.20 | | 353.0 | 395.0 | 28.4 | 38.0 | | | | | | 32.0 | | | |
| Patient n°292 | M | I | 23-mars-05 | 1922 | 3.86 | | 14.3 | 14.3 | 0.48 | 0.27 | 2.13 | 6.71 | 0.2 | 835.0 | 1.0 | 833.00 | | 285.0 | | 57.0 | | | 0.61 | 0.32 | 1.91 | 444.0 | | | | |
| Patient n°293 | F | I | 28-sept-04 | 1935 | 8.78 | 15.9 | | 15.9 | | 0.55 | 2.42 | 6.57 | 1.8 | 828.0 | 40.0 | 18.70 | | 397.0 | 665.0 | 52.0 | 43.0 | | | | | | 57.4 | | | |
| Patient n°294 | F | I | 11-oct-04 | 1959 | 10.14 | 16.0 | | 16.0 | | 0.63 | 2.02 | 7.92 | 0.5 | 724.0 | 7.0 | 101.43 | 44.0 | 340.0 | 305.0 | | 26.0 | 99.0 | 0.96 | 0.65 | 1.48 | | 55.0 | | | 24.00 |
| Patient n°295 | F | I | 28-avr-04 | 1929 | 13.33 | 14.8 | | 14.8 | | 0.90 | 1.95 | 7.59 | | 781.3 | 25.9 | 28.13 | | 334.0 | 505.0 | 54.0 | 55.0 | | | | | | 44.3 | | | |
| Patient n°295 | F | 2 | 11-oct-04 | 1929 | 8.64 | 14.9 | | 14.9 | | 0.58 | 2.17 | 6.87 | 1.3 | 713.0 | 46.0 | 13.50 | | 303.0 | 620.0 | 41.0 | 56.0 | | | | | | | | 12.00 | 14.00 |
| Patient n°296 | F | I | 27-sept-04 | 1974 | 4.80 | 9.2 | | 9.2 | | 0.52 | 1.44 | 6.39 | 0.2 | 953.0 | 139.0 | 4.86 | | 314.0 | 260.0 | | 38.0 | | | | | | | | 8.80 | 6.20 |
| Patient n°297 | F | I | 11-mai-04 | 1962 | 13.86 | 11.6 | | 11.6 | | 1.19 | 1.15 | 10.09 | | 1056.9 | 50.1 | 19.11 | 36.0 | 446.9 | 130.0 | 35.3 | 27.0 | 132.0 | | | | | 165.5 | | | |
| Patient n°297 | F | 2 | 23-nov-04 | 1962 | 12.82 | 8.4 | | 8.4 | | 1.53 | 1.36 | | 1.4 | 966.0 | 22.0 | 41.91 | | 272.0 | | 44.0 | 32.0 | | | | | 486.0 | 102.0 | | | |
| Patient n°298 | M | I | 29-oct-03 | 1951 | 5.31 | 18.7 | | 18.7 | | 0.28 | 2.10 | 8.91 | 19.1 | 782.3 | 29.2 | 24.79 | 42.0 | 439.0 | 85.0 | 50.0 | 58.0 | 87.0 | 0.92 | 0.65 | 1.42 | | 258.7 | 92.6 | 5.82 | 7.56 |
| Patient n°299 | F | I | 9-sept-03 | 1957 | 10.74 | 26.7 | | 26.7 | | 0.40 | 2.43 | 10.99 | 12.0 | 887.0 | 193.0 | 2.59 | 68.0 | 440.4 | 150.0 | 64.9 | 72.0 | 111.0 | 1.08 | 0.69 | 1.57 | | 438.5 | 108.7 | 5.51 | 4.96 |
| Patient n°300 | F | I | 2-mars-04 | 1968 | 15.70 | 18.4 | | 18.4 | | 0.85 | 2.43 | 7.57 | | 949.3 | 68.6 | 11.84 | | 266.1 | 260.0 | 39.6 | 39.0 | | | | | | 291.2 | | | |
| Patient n°300 | F | 2 | 26-oct-04 | 1968 | 18.99 | 17.8 | | 17.8 | | 1.07 | 2.46 | 7.24 | 1.2 | 1110.0 | 25.0 | 42.40 | | 362.0 | 635.0 | 62.0 | 42.0 | | | | | | 35.0 | | | |
| Patient n°301 | F | I | 15-juil-03 | 1935 | 10.46 | 16.9 | | 16.9 | | 0.62 | 1.35 | 12.52 | | 1040.0 | 12.2 | 83.60 | | 407.8 | 345.0 | 55.6 | 31.0 | | | | | | 159.5 | 459.0 | | |
| Patient n°302 | F | I | 14-avr-04 | 1933 | 8.16 | 10.5 | | 10.5 | | 0.78 | 1.47 | 7.14 | | 660.9 | 43.8 | 13.08 | | 391.3 | 440.0 | 23.3 | 65.0 | | | | | | 40.1 | | | |
| Patient n°302 | F | 2 | 17-nov-04 | 1933 | 11.86 | 13.1 | | 13.1 | | 0.91 | 1.64 | 7.99 | 1.0 | 778.0 | 3.0 | 257.33 | | 274.0 | 870.0 | 42.0 | 61.0 | | | | | | 690.0 | | | |
| Patient n°303 | F | I | 27-juil-04 | 1948 | 13.40 | 8.5 | | 8.5 | | 1.58 | 1.37 | 6.20 | 0.3 | 760.0 | 53.0 | 12.34 | | 405.0 | 440.0 | 35.0 | 57.0 | | | | | | 65.0 | | | |
| Patient n°304 | F | I | 23-févr-05 | 1949 | 17.27 | | 11.9 | 11.9 | | 1.45 | 1.87 | 6.36 | 0.5 | 886.0 | 38.0 | 21.32 | | 402.0 | | 56.4 | 34.0 | | | | | | 531.0 | | | 18.90 |

EP 1 793 230 B1

| Patient | Sexe | N | Date | Année | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|
| Patient n°305 | F | 1 | 1-mars-05 | 1975 | 12,21 | | 11,3 | 11,3 | 1,20 | 1,08 | 1,84 | 6,14 | 1,9 | 797,0 | 6,0 | 130,83 | | | | 1,3 | | | | | | | 773,0 | | | |
| Patient n°306 | F | 1 | 14-avr-04 | 1943 | 7,46 | 18,8 | | 18,8 | | 0,40 | 2,98 | 6,31 | | 1102,0 | 110,4 | 7,98 | | 334,6 | 160,0 | 58,9 | 46,0 | | | | | | 225,1 | | | |
| Patient n°307 | F | 1 | 21-déc-04 | 1981 | 11,57 | 12,6 | | 12,6 | | 0,92 | 1,70 | 7,41 | 0,5 | 1177,0 | 27,0 | 41,59 | | 324,0 | | 32,2 | 40,0 | | | | | | | | | 11,35 |
| Patient n°308 | F | 1 | 13-avr-04 | 1941 | 6,80 | 21,5 | | 21,5 | | 0,32 | 2,77 | 7,76 | 1,1 | 771,1 | 66,3 | 9,63 | 88,0 | 402,5 | 510,0 | 46,0 | 67,0 | 168,0 | 1,04 | 0,67 | 1,55 | | 107,6 | 424,8 | 9,45 | 10,40 |
| Patient n°308 | F | 2 | 5-oct-04 | 1941 | 6,74 | 18,1 | | 18,1 | | 0,37 | 1,25 | 14,48 | 2,0 | 827,0 | 74,0 | 9,18 | | 295,0 | 1055,0 | 65,0 | 28,0 | | | | | | 64,0 | | | |
| Patient n°309 | M | 1 | 31-août-04 | 1937 | 11,08 | 14,4 | | 14,4 | | 0,77 | 1,88 | 7,66 | 1,1 | 968,0 | 50,0 | 17,36 | | 403,0 | 267,5 | 47,6 | 47,0 | 108,0 | 0,77 | 0,68 | 1,13 | | 24,0 | | | |
| Patient n°310 | F | 1 | 26-sept-05 | 1961 | | | 16,9 | 16,9 | 1,13 | 0,00 | 2,10 | 8,05 | 1,6 | 816,0 | 4,0 | 202,00 | | 357,0 | | 53,0 | | | | | | | 267,0 | | | |
| Patient n°311 | F | 1 | 6-juil-04 | 1965 | 6,13 | 10,3 | | 10,3 | | 0,60 | 1,25 | 8,24 | 0,6 | 750,0 | 23,0 | 30,61 | | 542,0 | | | 26,0 | | | | | | 77,8 | | | |
| Patient n°312 | M | 1 | 19-mai-04 | 1947 | 2,58 | 13,8 | | 13,8 | | 0,19 | 2,35 | 5,87 | 0,3 | 1146,6 | 10,4 | 108,78 | 85,0 | 240,8 | 360,0 | 39,1 | 60,0 | 109,0 | 0,88 | 0,73 | 1,20 | | 60,0 | 1295,0 | | |
| Patient n°313 | M | 1 | 21-févr-05 | 1963 | 9,99 | | 13,5 | 13,5 | 1,15 | 0,74 | 1,60 | 8,44 | 0,5 | 847,0 | 28,0 | 28,25 | 71,0 | 360,0 | | 40,0 | 54,0 | 108,0 | 0,97 | 0,63 | 1,54 | 290,0 | 22,0 | 642,0 | | 8,00 |
| Patient n°314 | F | 1 | 13-avr-04 | 1955 | 11,09 | 11,6 | | 11,6 | | 0,96 | 1,80 | 6,44 | | 766,3 | 27,8 | 25,55 | | 493,5 | 170,0 | 23,8 | 28,0 | | | | | | 65,6 | | | |
| Patient n°315 | F | 1 | 21-avr-04 | 1960 | 10,95 | 18,6 | | 18,6 | | 0,59 | 2,07 | 8,99 | 0,8 | 825,9 | 40,4 | 18,43 | 41,0 | 432,5 | 370,0 | 57,3 | 64,0 | 72,0 | 0,75 | 0,53 | 1,42 | | 210,7 | #### | 8,60 | 7,74 |
| Patient n°315 | F | 2 | 26-oct-04 | 1960 | 13,90 | 12,9 | | 12,9 | | 1,08 | 1,78 | 7,25 | 1,0 | 1302,0 | 99,0 | 11,15 | | 347,0 | 840,0 | 54,0 | 65,0 | | | | | | 32,0 | | | |
| Patient n°316 | M | 1 | 21-avr-04 | 1952 | 7,90 | 13,5 | | 13,5 | | 0,59 | 2,24 | 6,03 | | 1153,5 | 150,4 | 5,67 | | 476,2 | 410,0 | 34,0 | 62,0 | | | | | | 447,9 | | | |
| Patient n°316 | M | 2 | 26-oct-04 | 1952 | 8,91 | 13,1 | | 13,1 | | 0,68 | 2,48 | 5,28 | 0,9 | 1317,0 | 6,0 | 217,50 | | 359,0 | 905,0 | 51,0 | 67,0 | | | | | | 55,0 | | | |
| Patient n°317 | M | 1 | 6-déc-04 | 1941 | 8,10 | 19,9 | | 19,9 | | 0,41 | 2,27 | | 0,1 | 754,0 | 12,0 | 60,83 | 43,0 | 353,0 | | 34,7 | 77,0 | 146,0 | 0,86 | 0,69 | 1,25 | | 61,1 | #### | 10,50 | 12,60 |
| Patient n°317 | M | 2 | 24-mai-05 | 1941 | 6,51 | | 12,9 | 12,9 | | 0,50 | 1,50 | 8,54 | 0,2 | 826,0 | 87,0 | 7,49 | | 344,0 | | | | | | | | | 357,0 | #### | | |
| Patient n°318 | F | 1 | 4-mai-04 | 1953 | 12,55 | 14,9 | | 14,9 | | 0,84 | 2,11 | 7,06 | | 1020,4 | 108,3 | 7,42 | 33,0 | 365,9 | 215,0 | 40,0 | 40,0 | 201,0 | | | | | 149,7 | | | |
| Patient n°319 | F | 1 | 12-janv-05 | 1949 | 7,40 | 15,9 | | 15,9 | | 0,47 | 2,23 | 7,13 | 0,5 | 796,0 | 55,0 | 12,47 | 49,0 | 404,0 | | 58,7 | 38,0 | 126,0 | 1,08 | 0,70 | 1,54 | | 37,6 | #### | | 3,10 |
| Patient n°320 | M | 1 | 5-janv-05 | 1921 | 10,70 | 12,1 | | 12,1 | | 0,88 | 2,10 | 5,76 | 1,9 | 1064,0 | 60,0 | 15,73 | 55,0 | 356,0 | | 58,7 | 32,0 | 98,0 | 0,83 | 0,64 | 1,30 | | 37,6 | 1013,0 | | 3,11 |
| Patient n°321 | F | 1 | 19-mai-04 | 1939 | 6,98 | 12,6 | | 12,6 | | 0,55 | 2,13 | 5,92 | 1,1 | 1268,8 | 18,5 | 66,55 | 67,0 | 337,6 | 370,0 | 35,6 | 43,0 | 102,0 | 1,20 | 0,62 | 1,93 | | 68,9 | 736,3 | 5,20 | 4,67 |
| Patient n°321 | F | 2 | 12-avr-05 | 1939 | 10,42 | | 19,3 | 19,3 | 1,13 | 0,54 | 2,09 | | 1,1 | 810,0 | 42,0 | 17,29 | | 351,0 | | 61,0 | | | | | | | 357,0 | | | |
| Patient n°322 | M | 1 | 1-juin-05 | 1950 | 11,18 | | 12,5 | 12,5 | 0,63 | 0,89 | 2,27 | 5,51 | 0,5 | 789,0 | 50,0 | 13,78 | 45,0 | 302,0 | | 55,0 | | 138,0 | 0,89 | 0,65 | 1,37 | 167,0 | 52,0 | 282,0 | | 22,00 |
| Patient n°323 | M | 1 | 9-mars-05 | 1929 | 13,73 | | 16,6 | 16,6 | 0,99 | 0,83 | 1,90 | 8,74 | 2,1 | 934,0 | 78,0 | 10,62 | | | | 45,0 | | | | | | | 405,0 | | | 10,00 |
| Patient n°324 | F | 1 | 28-avr-04 | 1970 | 11,89 | 10,7 | | 10,7 | | 1,11 | 2,03 | 5,27 | | 963,3 | 82,3 | 9,71 | | 430,4 | 165,0 | 24,0 | 31,0 | | | | | | 27,0 | | | |
| Patient n°324 | F | 2 | 19-oct-04 | 1970 | 10,38 | 10,3 | | 10,3 | | 1,01 | 1,71 | 6,02 | 1,4 | 630,0 | 85,0 | 5,41 | | 348,0 | 500,0 | 14,0 | 32,0 | | | | | | | | 13,00 | 26,00 |
| Patient n°325 | M | 1 | 6-sept-05 | 1940 | 9,10 | | 19,1 | 19,1 | 1,20 | 0,48 | 2,46 | 7,76 | 0,3 | 958,0 | 54,0 | 15,74 | | 386,0 | | 63,0 | | | | | | | 116,0 | | | |

| | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|
| Patient n°326 | F | 1 | 1-févr-05 | 1938 | 8.10 | | 17.6 | 17.6 | | 0.46 | 2.36 | 7.46 | 0.5 | 834.0 | 73.0 | 9.42 | | 358.0 | | 51.1 | 45.0 | | | | | 831.0 | | | | |
| Patient n°326 | F | 2 | 6-sept-05 | 1938 | 8.03 | | 18.4 | 18.4 | | 0.44 | 2.71 | 6.79 | 1.5 | 1040.0 | 60.0 | 15.33 | | 336.0 | | 60.0 | | | | | | 412.0 | | | | |
| Patient n°327 | M | 1 | 23-mars-05 | 1914 | 2.68 | | 12.2 | 12.2 | 0.44 | 0.22 | 1.73 | 7.05 | 0.2 | 838.0 | 10.0 | 81.80 | | 278.0 | | 37.0 | | | 1.07 | 0.45 | 2.38 | 466.0 | | | | |
| Patient n°328 | F | 1 | 10-août-04 | 1952 | 5.78 | 11.4 | | 11.4 | | 0.51 | 1.76 | 6.48 | 0.7 | 1377.0 | 103.0 | 11.37 | | 369.0 | 547.5 | 41.9 | 56.0 | | | | | 79.0 | | | | |
| Patient n°328 | F | 2 | 31-mai-05 | 1952 | 19.19 | | 13.9 | 13.9 | | 1.38 | 1.83 | 7.60 | 1.2 | 1132.0 | 3.0 | 375.33 | | 287.0 | | 53.0 | | | | | | 347.0 | | | | |
| Patient n°329 | M | 1 | 16-nov-04 | 1947 | 7.71 | 20.6 | | 20.6 | | 0.37 | 2.67 | 7.71 | 0.2 | 1026.0 | 37.0 | 25.73 | | 366.0 | 1085.0 | 43.0 | 51.0 | | | | | 293.0 | | | | |
| Patient n°330 | F | 1 | 1-févr-05 | 1980 | 4.33 | | 9.2 | 9.2 | | 0.47 | 1.84 | 5.00 | 0.2 | 1525.0 | 89.0 | 15.13 | | 383.0 | | 36.4 | 52.0 | | | | | 304.0 | | | | |
| Patient n°331 | F | 1 | 6-juil-04 | 1966 | 8.22 | 11.2 | | 11.2 | | 0.73 | 1.81 | 6.19 | 0.8 | 743.0 | 38.0 | 17.55 | 48.0 | 448.0 | | | 28.0 | 122.0 | 0.88 | 0.59 | 1.49 | | 119.7 | 107.0 | 7.77 | 13.20 |
| Patient n°332 | F | 1 | 23-mars-05 | 1920 | 3.55 | | 24.6 | 24.6 | 1.39 | 0.14 | 2.62 | 9.39 | 0.1 | 1192.0 | 15.0 | 77.47 | | 234.0 | | 55.0 | | | 0.77 | 0.38 | 2.03 | 435.0 | | | | |
| Patient n°333 | F | 1 | 6-sept-05 | 1964 | 14.00 | | 16.5 | 16.5 | 1.01 | 0.85 | 2.05 | 8.05 | 0.9 | 857.0 | 11.0 | 75.91 | | 389.0 | | 60.0 | | | | | | 340.0 | | | | |
| Patient n°334 | F | 1 | 17-mai-05 | 1931 | 13.69 | | 14.9 | 14.9 | | 0.92 | 2.00 | 7.45 | 0.3 | 761.0 | 117.0 | 4.50 | | 311.0 | | 63.0 | | | | | | 454.0 | | | 9.00 | 15.00 |
| Patient n°335 | F | 1 | 8-févr-05 | 1967 | 17.63 | | 8.4 | 8.4 | | 2.10 | 2.00 | 4.20 | 0.2 | 757.0 | 123.0 | 4.15 | 54.0 | 311.0 | | 30.0 | 52.0 | 69.0 | 1.19 | 0.49 | 2.43 | | 30.0 | 440.0 | | 0.70 |
| Patient n°336 | F | 1 | 30-nov-04 | 1944 | 10.20 | 11.0 | | 11.0 | | 0.93 | 1.87 | 5.88 | 0.6 | 1021.0 | 7.0 | 143.86 | | 290.0 | | 33.8 | 60.0 | | | | | | | | | 19.40 |
| Patient n°337 | F | 1 | 25-mai-04 | 1954 | 12.00 | 15.9 | | 15.9 | | 0.75 | 2.13 | 7.46 | 0.7 | 1282.1 | 38.0 | 31.78 | 56.0 | 405.2 | 235.0 | 42.4 | 44.0 | 140.0 | 1.49 | 0.76 | 1.96 | | 37.4 | 93.2 | 11.98 | 13.18 |
| Patient n°338 | M | 1 | 24-févr-04 | 1950 | 8.39 | 16.3 | | 16.3 | | 0.51 | 2.30 | 7.09 | | 1012.0 | 112.2 | 7.02 | | 308.0 | 640.0 | 41.1 | 62.0 | | | | | 66.9 | | | | |
| Patient n°339 | F | 1 | 14-sept-04 | 1947 | 11.60 | 14.5 | | 14.5 | | 0.80 | 2.38 | 6.09 | 1.3 | 739.0 | 13.0 | 54.85 | | 307.0 | 497.5 | 52.0 | 54.0 | | | | | 47.9 | | | | |
| Patient n°340 | F | 1 | 12-juil-05 | 1980 | 15.85 | | 7.8 | 7.8 | 0.55 | 2.03 | 1.32 | 5.91 | 0.5 | 695.0 | 19.0 | 34.58 | 42.0 | 352.0 | | 40.0 | 41.0 | 88.0 | 1.06 | 0.61 | 1.74 | 625.0 | 27.0 | 386.0 | 3.00 | 1.20 |
| Patient n°341 | M | 1 | 28-avr-04 | 1955 | 5.50 | 14.1 | | 14.1 | | 0.39 | 2.46 | 5.73 | | 807.2 | 57.3 | 12.10 | | | 535.0 | 37.1 | 79.0 | | | | | 107.1 | | | | |
| Patient n°342 | M | 1 | 26-avr-05 | 1914 | 4.28 | | 20.8 | 20.8 | 1.73 | 0.21 | 3.07 | | 0.3 | 933.0 | 154.0 | 4.06 | | 307.0 | | 82.0 | | | 1.18 | 0.58 | 2.03 | 497.0 | | | | |
| Patient n°343 | F | 1 | 6-juil-04 | 1968 | 10.00 | 14.5 | | 14.5 | | 0.69 | 1.52 | 9.54 | 1.1 | 694.0 | 13.0 | 51.38 | 72.0 | 515.0 | | | 23.0 | 131.0 | 0.67 | 0.69 | 0.97 | 72.6 | 386.0 | | | 6.22 |
| Patient n°344 | F | 1 | 31-août-04 | 1942 | 9.73 | 14.1 | | 14.1 | | 0.69 | 2.19 | 6.44 | 0.5 | 699.0 | 50.0 | 11.98 | | 407.0 | 565.0 | 47.9 | 59.0 | | | | | 31.8 | | | | |
| Patient n°345 | M | 1 | 21-sept-04 | 1944 | 7.02 | 17.6 | | 17.6 | | 0.40 | 2.16 | 8.15 | 0.3 | 1254.0 | 130.0 | 7.65 | | 303.0 | 435.0 | 63.0 | | | | | | 61.8 | | | | |
| Patient n°345 | M | 2 | 7-juin-05 | 1944 | 7.57 | | 12.5 | 12.5 | | 0.61 | 1.80 | 6.94 | 0.9 | 1111.0 | 189.0 | 3.88 | | 363.0 | | 48.0 | | | | | | 45.0 | | | | |
| Patient n°346 | M | 1 | 31-août-04 | 1947 | 8.17 | 15.4 | | 15.4 | | 0.53 | 2.07 | 7.44 | 0.2 | 837.0 | 35.0 | 21.91 | | 416.0 | 450.0 | 47.7 | 66.0 | | | | | 39.5 | | | | |
| Patient n°347 | F | 1 | 1-sept-04 | 1939 | 10.02 | 13.1 | | 13.1 | | 0.76 | 2.20 | 5.95 | 1.7 | 964.0 | 15.0 | 62.27 | | 298.0 | 90.0 | 47.0 | 18.0 | | | | | 39.0 | | | | |
| Patient n°348 | F | 1 | 8-sept-04 | 1943 | 8.53 | 17.4 | | 17.4 | | 0.49 | 1.97 | 8.83 | 0.7 | 819.0 | 121.0 | 4.77 | | 320.0 | 417.5 | 51.5 | 42.0 | | | | | 41.5 | | | | |
| Patient n°349 | F | 1 | 14-avr-04 | 1956 | 11.86 | 14.4 | | 14.4 | | 0.82 | 2.07 | 6.96 | | 1009.8 | 183.2 | 3.51 | | 407.0 | 135.0 | 43.6 | 42.0 | | | | | 68.2 | | | | |

EP 1 793 230 B1

| Patient | Sexe | N° | Date | Année | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|
| Patient n°349 | F | 2 | 9-févr-05 | 1956 | 14.86 | | 16.5 | 16.5 | 2.13 | 0.90 | 2.16 | 7.64 | 0.5 | 1055.0 | 26.0 | 38.58 | 30.0 | 259.3 | 315.0 | 57.3 | 48.0 | 98.0 | 0.97 | 0.83 | 1.17 | 292.0 | 535.0 | 179.5 | 12.75 | 17.85 |
| Patient n°350 | M | 1 | 2-mars-04 | 1955 | 8.38 | | | 16.1 | | 0.52 | 2.56 | 6.29 | 0.3 | 808.0 | 18.3 | 42.13 | | 377.1 | 285.0 | 35.1 | 43.0 | | | | | | | | | |
| Patient n°351 | F | 1 | 27-avr-04 | 1960 | 5.90 | | | 15.3 | | 0.39 | 2.04 | 7.50 | | 854.9 | 85.4 | 8.01 | | 394.0 | | 52.2 | 46.0 | | | | | | 537.3 | | | |
| Patient n°351 | F | 2 | 18-janv-05 | 1960 | 10.56 | | | 15.6 | | 0.68 | 2.24 | 6.96 | 1.9 | 822.0 | 6.0 | 135.00 | 52.0 | 434.0 | | 52.2 | 53.0 | 104.0 | 1.04 | 0.75 | 1.39 | | 32.1 | | | |
| Patient n°352 | M | 1 | 4-mai-04 | 1959 | 7.92 | | | 10.4 | | 0.76 | 1.66 | 6.27 | | 714.6 | 127.6 | 3.60 | | 388.0 | 255.0 | 16.0 | 71.0 | 104.0 | 0.69 | 0.58 | 1.19 | | 126.5 | | | |
| Patient n°353 | M | 2 | 25-janv-05 | 1959 | 6.11 | | | 7.7 | | 0.79 | 0.98 | 7.86 | 0.8 | 668.0 | 55.0 | 10.15 | | 301.0 | | 22.2 | 44.0 | | | | | | 32.1 | | | |
| Patient n°354 | F | 1 | 6-sept-04 | 1943 | 9.29 | | | 14.8 | | 0.63 | 2.35 | 6.30 | 1.0 | 782.0 | 29.0 | 24.97 | | 337.0 | 375.0 | 47.0 | 78.0 | | | | | | 71.7 | | | |
| Patient n°355 | M | 1 | 6-oct-04 | 1932 | 5.14 | | | 12.4 | | 0.41 | 1.65 | 7.52 | 0.3 | 1156.0 | 71.0 | 14.28 | | 281.0 | 1240.0 | 56.0 | 32.0 | | | | 97.0 | | | | |
| Patient n°356 | F | 1 | 20-sept-04 | 1960 | 7.46 | | | 15.2 | | 0.49 | 2.14 | 7.10 | 0.5 | 549.0 | 15.0 | 34.60 | | 341.0 | 370.0 | 68.4 | 51.0 | | | | | | 50.2 | | | |
| Patient n°357 | M | 1 | 7-sept-04 | 1961 | 12.76 | | | 11.0 | | 1.16 | 1.80 | 6.11 | 1.4 | 1073.0 | 31.0 | 32.61 | 57.0 | 303.0 | 350.0 | 59.0 | 49.0 | 99.0 | 1.17 | 0.79 | 1.48 | | 65.4 | 110.4 | | |
| Patient n°358 | F | 1 | 6-sept-04 | 1950 | 10.42 | | | 14.2 | | 0.73 | 1.91 | 7.44 | 1.2 | 981.0 | 25.0 | 37.24 | | | 430.0 | 44.2 | 34.0 | | | | | | 47.0 | | | |
| Patient n°359 | F | 1 | 28-avr-04 | 1954 | 15.20 | | | 16.3 | | 0.93 | 2.16 | 7.55 | | 894.5 | 133.2 | 4.72 | | 346.0 | | 55.6 | 34.0 | | | | | | 33.8 | | | |
| Patient n°359 | F | 2 | 30-nov-04 | 1954 | 12.57 | | | 16.3 | | 0.77 | 2.16 | 7.55 | | 934.0 | 24.0 | 36.92 | 55.0 | 396.2 | 160.0 | 57.3 | 31.0 | | | | | | | | 10.25 | 9.70 |
| Patient n°360 | F | 1 | 15-janv-04 | 1934 | 6.25 | | | 15.0 | | 0.42 | 2.07 | 7.34 | 0.4 | 1343.8 | 126.9 | 8.59 | | | 110.0 | 48.9 | | 111.0 | 0.82 | 0.53 | 1.55 | | 59.2 | | | 11.27 |
| Patient n°361 | F | 1 | 19-avr-05 | 1946 | 12.03 | | 9.1 | 9.1 | | 1.32 | 1.46 | 6.23 | 2.8 | 673.0 | 24.0 | 26.04 | 44.0 | 451.5 | 285.0 | 38.0 | 48.0 | 119.0 | 1.07 | 0.73 | 1.47 | 398.0 | 30.7 | | | |
| Patient n°362 | F | 1 | 28-avr-04 | 1919 | 3.71 | | | 22.4 | | 0.17 | 2.53 | 8.85 | 0.2 | 1063.6 | 86.5 | 10.30 | | 351.0 | | 48.7 | 40.0 | | | | | | 35.7 | 703.7 | 28.60 | 14.30 |
| Patient n°362 | F | 2 | 18-oct-04 | 1919 | 10.18 | | | 18.5 | | 0.55 | 2.19 | 8.45 | 1.0 | 899.0 | 111.0 | 6.10 | 44.0 | 414.0 | 580.0 | 57.0 | 29.0 | 130.0 | 1.10 | 0.92 | 1.20 | | | | 15.00 | 12.00 |
| Patient n°363 | F | 1 | 9-juin-04 | 1949 | 12.45 | | | 13.4 | | 0.93 | 2.23 | | 0.8 | 1262.0 | 73.0 | 15.29 | | 397.0 | 265.0 | 46.0 | 27.0 | 121.0 | 1.02 | 0.78 | 1.31 | | 77.3 | 176.0 | 2.50 | 0.50 |
| Patient n°363 | F | 2 | 21-déc-04 | 1949 | 7.37 | | | 14.4 | | 0.51 | 1.99 | 7.07 | 0.9 | 1060.0 | 3.0 | 351.33 | | 356.0 | | 28.0 | | | | | | | | | | |
| Patient n°364 | M | 1 | 23-juin-04 | 1947 | 10.73 | | | 16.2 | | 0.66 | 2.29 | 10.35 | 0.2 | 1161.0 | 63.0 | 16.43 | | 388.0 | | | 64.0 | | | | | 428.0 | 48.0 | 226.0 | | 1.76 |
| Patient n°364 | M | 2 | 13-déc-04 | 1947 | 8.98 | | | 20.8 | | 0.43 | 2.01 | 7.20 | 0.3 | 992.0 | 18.0 | 53.11 | | 504.0 | | 64.0 | 48.0 | | | | | | | | | |
| Patient n°365 | F | 1 | 9-juin-04 | 1957 | 9.95 | | | 13.9 | | 0.72 | 1.93 | 4.70 | 0.2 | 1088.0 | 17.0 | 62.00 | 39.0 | 426.5 | 325.0 | 30.7 | 36.0 | 97.0 | 1.19 | 0.62 | 1.92 | | 63.2 | 370.0 | 7.30 | 10.95 |
| Patient n°366 | M | 1 | 28-avr-04 | 1978 | 12.73 | | | 9.4 | | 1.35 | 2.00 | 7.06 | 1.5 | 940.0 | 66.5 | 12.13 | 41.0 | 386.6 | 95.0 | 18.0 | 20.0 | 89.0 | 1.74 | 0.71 | 2.45 | | 30.9 | 1117.7 | 18.47 | 11.08 |
| Patient n°367 | M | 1 | 28-avr-04 | 1918 | 4.19 | | | 13.0 | | 0.32 | 1.87 | 7.46 | 0.4 | 970.4 | 82.7 | 9.73 | 61.0 | 315.0 | 385.0 | 35.8 | 58.0 | 129.0 | 1.02 | 0.73 | 1.40 | | 46.1 | 157.2 | 12.60 | 12.60 |
| Patient n°367 | M | 2 | 18-oct-04 | 1918 | 10.68 | | | 13.2 | | 0.81 | 1.77 | 5.55 | | 825.0 | 40.0 | 18.63 | | | | 52.0 | 53.0 | | | | | 225.0 | | | 14.00 | |
| Patient n°367 | M | 3 | 2-mai-05 | 1918 | 5.58 | | 7.1 | 7.1 | | 0.79 | 1.28 | 7.99 | | 975.0 | 70.0 | 11.93 | | 369.5 | 915.0 | 26.0 | | | | | | | | | | 18.00 |
| Patient n°368 | M | 1 | 31-mars-04 | 1919 | 10.43 | | 14.7 | 14.7 | 0.07 | 0.71 | 1.84 | | 0.4 | 694.8 | 58.7 | 9.84 | 55.0 | | 290.0 | 40.4 | 65.0 | 68.0 | 1.07 | 0.58 | 1.85 | | 77.5 | 218.6 | 14.75 | 10.33 |

22

| Patient | Sexe | Gr | Date | Année | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|
| Patient n°369 | F | 1 | 8-sept-04 | 1945 | 14,36 | 20,8 | | 20,8 | | 0,69 | 3,33 | 6,25 | 1,2 | 1158,0 | 6,0 | 191,00 | | 332,0 | 447,0 | 67,5 | 57,0 | 139,0 | 1,27 | 0,87 | 1,46 | | 113,0 | | | |
| Patient n°369 | F | 2 | 4-avr-05 | 1945 | 19,63 | 21,1 | 21,1 | | | 0,93 | 2,03 | 10,39 | 0,4 | 1058,0 | 59,0 | 15,93 | 56,0 | 362,0 | | | | | | | | | | | | 12,00 |
| Patient n°370 | F | 1 | 18-mai-04 | 1935 | 7,97 | 14,0 | | 14,0 | | 0,57 | 2,54 | 5,51 | | 1157,5 | 93,1 | 10,43 | | 303,2 | 880,0 | 32,2 | 64,0 | 122,0 | | | | | 54,8 | | | |
| Patient n°371 | F | 1 | 4-nov-03 | 1954 | 21,47 | 12,3 | | 12,3 | | 1,75 | 1,69 | 7,28 | | 1067,8 | 141,0 | 5,57 | | 392,2 | 270,0 | 43,6 | 40,0 | | | | | | 234,8 | | | |
| Patient n°372 | F | 1 | 12-mai-04 | 1947 | 15,26 | 14,9 | | 14,9 | | 1,02 | 1,99 | 7,49 | 0,5 | 1104,8 | 77,7 | 12,22 | 42,0 | 414,4 | 475,0 | 48,7 | 33,0 | 146,0 | | | | 202,0 | 210,0 | | | |
| Patient n°373 | F | 1 | 6-sept-05 | 1961 | 14,00 | 12,4 | 12,4 | | 2,13 | 1,13 | 1,74 | 7,13 | | 776,0 | 11,0 | 68,55 | | 332,0 | | 45,0 | | | | | | 202,0 | | | | |
| Patient n°374 | F | 1 | 9-nov-04 | 1944 | 10,12 | 12,0 | | 12,0 | | 0,84 | 1,70 | 7,06 | 0,9 | 929,0 | 43,0 | 19,60 | | 346,0 | 580,0 | 64,0 | 37,0 | | | | | 387,0 | | | | |
| Patient n°375 | F | 1 | 4-janv-05 | 1951 | 18,14 | 13,2 | | 13,2 | | 1,37 | 1,50 | 8,80 | 0,1 | 783,0 | 42,0 | 16,64 | 46,0 | 329,0 | | 51,6 | 41,0 | 112,0 | 0,97 | 0,57 | 1,70 | | 20,1 | 119,0 | 11,47 | 108,03 |
| Patient n°376 | F | 1 | 31-août-04 | 1967 | 11,33 | 10,8 | | 10,8 | | 1,05 | 1,59 | 6,79 | 1,1 | 710,0 | 42,0 | 14,90 | 41,0 | 462,0 | 180,0 | 36,2 | 33,0 | 79,0 | 1,15 | 0,61 | 1,89 | | 24,0 | 388,0 | | 37,58 |
| Patient n°377 | M | 1 | 26-avr-05 | 1917 | 3,66 | 12,0 | 12,0 | | 1,19 | 0,31 | 1,81 | | 0,5 | 746,0 | 11,0 | 65,82 | | 292,0 | | 47,0 | | | 1,16 | 0,66 | 1,76 | 386,0 | | 70,0 | X | PI |
| Patient n°378 | M | 1 | 9-sept-03 | 1951 | 9,06 | 25,4 | | 25,4 | | 0,36 | 1,83 | 13,88 | 1,8 | 1179,8 | 99,9 | 9,82 | 49,0 | 460,7 | nd | 64,2 | 39,0 | 72,0 | 0,63 | 0,63 | 1,00 | | 294,5 | | | |
| Patient n°379 | F | 1 | 11-mai-04 | 1940 | 4,34 | 16,0 | | 16,0 | | 0,27 | 2,21 | 7,24 | 0,3 | 542,5 | 36,9 | 12,70 | 40,0 | 421,6 | 260,0 | 23,6 | 72,0 | 108,0 | | | | | 89,7 | 1611,0 | 9,94 | 14,91 |
| Patient n°380 | M | 1 | 11-mai-04 | 1941 | 6,40 | 11,0 | | 11,0 | | 0,58 | 1,88 | 5,96 | 0,4 | 828,4 | 69,2 | 9,97 | 54,0 | 412,2 | 425,0 | 36,2 | 59,0 | 124,0 | | | | | 258,5 | 468,0 | | 10,50 |
| Patient n°381 | M | 1 | 31-août-04 | 1921 | 13,29 | 22,3 | | 22,3 | | 0,60 | 1,81 | 12,32 | 1,1 | 766,0 | 11,0 | 67,64 | | 354,0 | 610,0 | 47,9 | 67,0 | | | | | | 32,4 | | | |
| Patient n°381 | M | 2 | 3-mai-05 | 1921 | 3,60 | 15,3 | 15,3 | | 0,66 | 0,24 | 1,87 | | 0,7 | 1183,0 | 66,0 | 15,92 | | | | 48,0 | | | | | | 134,0 | | | | |
| Patient n°382 | F | 1 | 6-sept-04 | 1957 | 11,45 | 10,7 | | 10,7 | | 1,07 | 1,75 | 6,11 | 0,3 | 926,0 | 25,0 | 35,04 | 57,0 | 332,0 | 610,0 | 47,9 | 61,0 | 83,0 | 1,13 | 0,60 | 1,88 | | 60,8 | | | |
| Patient n°383 | F | 1 | 9-nov-04 | 1956 | 11,29 | 13,4 | | 13,4 | | 0,84 | 2,78 | 4,82 | 0,4 | 1215,0 | 125,0 | 7,72 | 69,0 | 372,0 | 720,0 | 67,0 | 51,0 | 120,0 | 1,01 | 0,84 | 1,20 | | 36,0 | | | |
| Patient n°384 | F | 1 | 6-sept-04 | 1943 | 10,96 | 18,3 | | 18,3 | | 0,60 | 2,45 | 7,47 | 0,6 | 1062,0 | 46,0 | 21,09 | | 300,0 | 395,0 | 45,7 | 61,0 | | | | | | 100,8 | 200,0 | 8,24 | 23,90 |
| Patient n°385 | M | 1 | 31-mars-04 | 1954 | 9,34 | 18,9 | | 18,9 | | 0,49 | 2,72 | 6,95 | 0,1 | 1139,0 | 104,5 | 8,90 | 46,0 | 401,3 | 230,0 | 47,8 | 54,0 | 85,0 | 1,09 | 0,69 | 1,58 | | 157,5 | | | |
| Patient n°386 | F | 1 | 27-avr-04 | 1938 | 7,56 | 17,1 | | 17,1 | | 0,44 | 2,12 | 8,07 | | 796,7 | 89,9 | 6,86 | | 372,7 | 525,0 | 64,0 | 58,0 | | | | | | 218,9 | | | |
| Patient n°386 | F | 2 | 5-oct-04 | 1938 | 8,35 | 20,3 | | 20,3 | | 0,41 | 1,12 | 18,13 | 3,0 | 618,0 | 40,0 | 13,45 | | 345,0 | 812,0 | 70,0 | 20,0 | | | | | | 32,0 | | | |
| Patient n°387 | M | 1 | 2-nov-04 | 1937 | 4,60 | 16,4 | | 16,4 | | 0,28 | 1,99 | 8,24 | 0,3 | 1468,0 | 95,0 | 13,45 | | 324,0 | 620,0 | 69,0 | 48,0 | | | | | | 193,0 | | | |
| Patient n°387 | M | 2 | 25-mai-05 | 1937 | 8,00 | 13,3 | 13,3 | | | 0,60 | 1,91 | 7,00 | 0,1 | 882,0 | 126,0 | 5,00 | | 306,0 | 20,0 | | | | | | | 300,0 | | | | |
| Patient n°388 | F | 1 | 29-oct-03 | 1961 | 13,28 | 15,1 | | 15,1 | | 0,88 | 2,19 | 6,90 | 38,1 | 860,2 | 128,9 | 4,67 | 55,0 | 469,9 | | 48,7 | 39,0 | 101,0 | 0,90 | 0,68 | 1,32 | | 116,5 | 99,9 | 6,53 | 1,96 |
| Patient n°388 | F | 2 | 1-févr-05 | 1961 | 10,41 | 14,6 | 14,6 | | | 0,71 | 2,10 | 6,95 | 0,6 | 958,0 | 9,0 | 104,44 | | 372,0 | | 52,0 | 44,0 | | | | | | 25,2 | | | |
| Patient n°389 | M | 1 | 13-avr-04 | 1932 | 8,95 | 17,3 | | 17,3 | | 0,52 | 2,18 | 7,94 | | 715,4 | 47,5 | 13,07 | | 423,5 | 610,0 | 39,1 | 67,0 | | | | | | 66,9 | | | |
| Patient n°389 | M | 2 | 5-oct-04 | 1932 | 10,07 | 13,9 | | 13,9 | | 0,72 | 0,77 | 18,05 | 0,9 | 751,0 | 63,0 | 9,92 | | 328,0 | 910,0 | 50,0 | 26,0 | | | | | | | | 17,50 | 14,00 |

EP 1 793 230 B1

| Patient | Sexe | N° | Date | Année | V1 | V2 | V3 | V4 | V5 | V6 | V7 | V8 | V9 | V10 | V11 | V12 | V13 | V14 | V15 | V16 | V17 | V18 | V19 | V20 | V21 | V22 | V23 | V24 | V25 | V26 |
|---|---|---|---|---|---|---|---|---|---|---|---|---|---|---|---|---|---|---|---|---|---|---|---|---|---|---|---|---|---|---|
| Patient n°390 | F | 1 | 9-mars-05 | 1934 | 9,70 | | 21,4 | 21,4 | 1,48 | 0,45 | 2,73 | 7,84 | 0,9 | 626,0 | 10,0 | 60,60 | | | | 60,0 | | | | | | 429,0 | | | | |
| Patient n°391 | F | 1 | 7-avr-03 | 1964 | 17,55 | 16,3 | | 16,3 | | 1,08 | 1,55 | 10,52 | 193,3 | 1122,8 | 3,2 | 348,87 | 48,0 | 379,6 | 200,0 | 52,2 | 39,0 | 144,0 | 0,99 | 0,73 | 1,36 | 290,5 | 59,7 | 398,4 | 12,94 | 16,82 |
| Patient n°391 | F | 2 | 7-oct-03 | 1964 | 12,78 | 13,4 | | 13,4 | | 0,95 | 1,27 | 10,55 | | 1141,7 | 176,9 | 4,45 | | 288,8 | 75,0 | 35,8 | 35,0 | | | | | 75,0 | | | |
| Patient n°391 | F | 3 | 9-juin-04 | 1964 | 11,15 | 13,6 | | 13,6 | | 0,82 | 1,20 | 11,33 | 0,6 | 935,0 | 93,0 | 8,05 | | 356,0 | 340,0 | 21,8 | | | | | | 43,0 | | | |
| Patient n°392 | M | 1 | 14-sept-04 | 1952 | 3,60 | 11,2 | | 11,2 | | 0,32 | 1,64 | 6,83 | 0,5 | 1040,0 | 100,0 | 8,40 | | 322,0 | 430,0 | 31,1 | 51,0 | | 1,28 | 0,87 | 1,47 | | 36,2 | | | |
| Patient n°393 | F | 1 | 14-févr-05 | 1948 | 8,04 | | 14,6 | 14,6 | 1,46 | 0,55 | 2,09 | 6,99 | 1,9 | 690,0 | 100,0 | 4,90 | | 301,0 | | 57,3 | | | | | 195,0 | | | | |
| Patient n°394 | M | 1 | 7-oct-03 | 1932 | 8,55 | 13,3 | | 13,3 | | 0,64 | 1,96 | 6,79 | | 836,6 | 53,5 | 13,65 | | | 190,0 | 45,3 | | 94,0 | 1,10 | 0,59 | 1,86 | 263,9 | 317,3 | 187,2 | | |
| Patient n°394 | M | 2 | 17-mars-04 | 1932 | 10,07 | 15,7 | | 15,7 | | 0,64 | 2,03 | 7,73 | | 1082,0 | 18,1 | 57,71 | | 287,1 | 430,0 | 37,3 | 66,0 | | | | | 38,3 | | | |
| Patient n°395 | F | 1 | 23-sept-03 | 1933 | 5,95 | 24,7 | | 24,7 | | 0,24 | 2,29 | 10,79 | 193,3 | 861,2 | 39,2 | 19,97 | 35,0 | 389,9 | | 56,9 | 39,0 | 124,0 | 1,45 | 0,62 | 2,34 | | 164,1 | 550,0 | | 17,26 |
| Patient n°395 | F | 2 | 9-mars-04 | 1933 | 13,03 | 18,3 | | 18,3 | | 0,71 | 2,14 | 8,55 | | 952,4 | 60,0 | 13,88 | | 267,5 | 105,0 | 37,3 | 34,0 | | | | | 106,7 | | | |
| Patient n°395 | F | 3 | 19-avr-05 | 1933 | 12,33 | | 19,3 | 19,3 | | 0,64 | 2,03 | 9,51 | 0,7 | 948,0 | 64,0 | 12,81 | | 358,0 | | | | | | | | 35,0 | | | |
| Patient n°396 | F | 1 | 10-août-04 | 1979 | 9,26 | 11,0 | | 11,0 | | 0,84 | 1,64 | 6,71 | 0,6 | 1484,0 | 63,0 | 21,56 | | 405,0 | 580,0 | 37,0 | 46,0 | | | | | 34,2 | | | |
| Patient n°397 | F | 1 | 6-juil-04 | 1983 | 8,94 | 13,1 | | 13,1 | | 0,68 | 1,17 | 11,20 | 1,1 | 891,0 | 93,0 | 7,58 | | 472,0 | | | 39,0 | | | | | 83,5 | | | |
| Patient n°398 | M | 1 | 17-févr-04 | 1949 | 12,47 | 15,8 | | 15,8 | | 0,79 | 1,55 | 10,19 | 0,8 | 1242,0 | 138,3 | 6,98 | 53,0 | 409,8 | 330,0 | 37,6 | 45,0 | 87,0 | 0,79 | 0,61 | 1,30 | | 65,2 | 302,8 | 22,16 | 31,03 |
| Patient n°399 | F | 1 | 29-juin-04 | 1955 | 9,72 | 12,4 | | 12,4 | | 0,78 | 1,88 | 6,60 | 0,5 | 880,0 | 82,0 | 8,73 | | 459,0 | | 37,0 | | | | | | 63,5 | | | |
| Patient n°400 | M | 1 | 13-sept-04 | 1949 | 5,90 | 17,1 | | 17,1 | | 0,35 | 2,12 | 8,07 | 0,2 | 845,0 | 34,0 | 22,85 | | 320,0 | 455,0 | 67,1 | 64,0 | | | | | 49,0 | | | |
| Patient n°401 | F | 1 | 18-juin-03 | 1939 | 8,79 | 17,7 | | 17,7 | | 0,50 | 2,58 | 6,86 | | 910,7 | 2,9 | 310,94 | | | 300,0 | 70,0 | | 95,0 | 0,97 | 0,65 | 1,49 | 397,2 | 154,1 | #### | | |
| Patient n°402 | F | 1 | 27-avr-05 | 1913 | 2,51 | | 13,3 | 13,3 | 0,69 | 0,19 | 2,20 | | | 855,0 | 135,0 | 4,33 | | 309,0 | | 53,0 | | | 1,29 | 0,72 | 1,79 | 203,0 | | | | |
| Patient n°403 | M | 1 | 12-oct-04 | 1934 | 9,78 | 15,5 | | 15,5 | | 0,63 | 1,94 | 7,99 | 0,6 | 629,0 | 45,0 | 11,98 | 42,0 | 345,0 | 970,0 | 38,0 | 70,0 | 126,0 | 0,83 | 0,60 | 1,38 | | 48,0 | 418,0 | 7,00 | 5,00 |
| Patient n°404 | M | 1 | 23-juin-03 | 1963 | 2,74 | 16,9 | | 16,9 | | 0,16 | 2,26 | 7,48 | | 1148,0 | 104,2 | 9,02 | | 420,1 | 240,0 | 57,8 | 51,0 | | | | | 337,7 | | | |
| Patient n°405 | F | 1 | 4-mai-04 | 1951 | 14,92 | 17,0 | | 17,0 | | 0,88 | 2,50 | 6,88 | | 904,2 | 75,2 | 10,03 | 58,0 | 390,4 | 370,0 | 52,4 | 57,0 | 105,0 | | | | 187,5 | | | |
| Patient n°406 | F | 1 | 25-oct-04 | 1948 | 11,99 | 17,6 | | 17,6 | | 0,68 | 2,40 | 7,33 | 1,2 | 915,0 | 7,0 | 128,71 | | 309,0 | 750,0 | 65,0 | 52,0 | | | | | 245,0 | | | |
| Patient n°407 | F | 1 | 28-sept-04 | 1965 | 13,65 | 17,4 | | 17,4 | | 0,78 | 2,31 | 7,53 | 0,3 | 745,0 | 4,0 | 184,25 | | 500,0 | 715,0 | 74,0 | 41,0 | | | | | 50,5 | | | |
| Patient n°408 | M | 1 | 31-août-04 | 1958 | 10,40 | 22,2 | | 22,2 | | 0,47 | 2,04 | 10,88 | 0,5 | 1003,0 | 46,0 | 19,80 | | 358,0 | 787,5 | 48,0 | 57,0 | | | | | 35,0 | | | |
| Patient n°409 | M | 1 | 17-févr-04 | 1966 | 0,10 | 11,3 | | 11,3 | | 0,01 | 1,52 | 7,43 | 0,4 | 1345,7 | 88,6 | 13,19 | 49,0 | 406,5 | 245,0 | 34,9 | 48,0 | 82,0 | 0,75 | 0,79 | 0,95 | | 55,4 | 297,4 | 9,23 | 19,38 |
| Patient n°409 | M | 2 | 27-mai-04 | 1966 | 5,54 | 14,0 | | 14,0 | | 0,40 | 2,13 | 6,57 | 0,4 | 1285,2 | 143,9 | 6,93 | 48,0 | 397,2 | 330,0 | 41,3 | 40,0 | 126,0 | 0,70 | 0,75 | 0,93 | | 54,5 | 949,6 | 12,61 | 35,32 |
| Patient n°410 | F | 1 | 18-nov-03 | 1951 | 8,45 | 10,9 | | 10,9 | | 0,78 | 1,87 | 5,83 | | 1218,3 | 97,5 | 10,50 | | 366,8 | 250,0 | 39,3 | 38,0 | | | | | 180,3 | | | |

| Patient | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|
| Patient n°411 | F | 1 | 24-déc-03 | 1955 | 16.51 | 14.1 | | 14.1 | | 1.17 | 2.53 | 5.57 | | 573.1 | 47.2 | 10.15 | 60.0 | 335.3 | 370.0 | 41.3 | 55.0 | 141.0 | 1.09 | 0.60 | 1.82 | | 18.0 | 201.4 | 14.49 | 14.49 |
| Patient n°412 | F | 1 | 19-déc-02 | 1946 | 9.54 | 12.2 | | 12.2 | | 0.78 | 2.22 | 5.50 | 51.2 | 815.6 | 4.7 | 173.03 | 62.0 | 427.1 | 7.6 | 52.0 | 61.0 | 116.0 | 1.19 | 0.81 | 1.47 | | 40.6 | 85.1 | 10.06 | 5.03 |
| Patient n°413 | F | 1 | 14-janv-03 | 1971 | 10.49 | 11.7 | | 11.7 | | 0.90 | 1.61 | 7.27 | 48.5 | 2178.9 | 3.1 | 710.04 | 58.0 | 393.1 | 3.3 | 43.6 | 34.0 | 113.0 | 1.19 | 0.71 | 1.68 | | 119.0 | 469.5 | 17.51 | 21.00 |
| Patient n°414 | M | 1 | 26-juil-05 | 1964 | 10.64 | | 7.1 | 7.1 | 0.47 | 1.50 | 1.02 | 6.96 | 0.3 | 539.0 | 30.0 | 15.97 | 50.0 | 268.0 | | 43.0 | 57.0 | 73.0 | 1.15 | 0.50 | 2.30 | 524.0 | 28.0 | 299.0 | 21.00 | 25.00 |
| Patient n°415 | F | 1 | 5-avr-05 | 1971 | 10.91 | | 14.6 | 14.6 | 0.76 | 0.75 | 1.79 | 8.16 | 0.3 | 948.0 | 103.0 | 7.20 | | | | 48.0 | | 99.0 | 0.68 | 0.61 | 1.11 | 290.0 | | | | |
| Patient n°416 | F | 1 | 10-août-04 | 1951 | 11.82 | 13.4 | | 13.4 | | 0.88 | 1.77 | 7.57 | 0.5 | 1412.0 | 65.0 | 19.72 | | 372.0 | 182.5 | 41.6 | 20.0 | | | | | | 61.6 | | | |
| Patient n°416 | F | 2 | 25-janv-05 | 1951 | 24.77 | 18.4 | | 18.4 | | 1.35 | 2.07 | 8.89 | 1.4 | 868.0 | 113.0 | 5.68 | | 349.0 | | 63.5 | 21.0 | | | | | 243.0 | | | | |
| Patient n°417 | F | 1 | 7-avr-03 | 1962 | 14.80 | 14.9 | | 14.9 | | 0.99 | 2.31 | 6.45 | | 1132.7 | 115.0 | 7.85 | | 366.2 | 120.0 | 57.1 | 25.0 | | | | | | 77.3 | 460.3 | | |
| Patient n°417 | F | 2 | 23-juin-04 | 1962 | 10.25 | 14.9 | | 14.9 | | 0.69 | 1.98 | 7.53 | 0.3 | 939.0 | 30.0 | 29.30 | | 374.0 | | | 28.0 | | | | | | 44.6 | | | |
| Patient n°418 | M | 1 | 13-janv-05 | 1961 | 12.05 | | 19.4 | 19.4 | | 0.62 | 1.97 | 9.85 | 0.6 | 1058.0 | 139.0 | 5.61 | | 423.0 | | | 65.0 | | | | | | 57.0 | | | |
| Patient n°419 | F | 1 | 22-sept-04 | 1975 | 8.75 | 9.0 | | 9.0 | | 0.97 | 1.61 | 5.59 | 0.1 | 706.0 | 52.0 | 11.58 | | 325.0 | 600.0 | 52.0 | 52.0 | | | | | | 44.0 | | | |
| Patient n°420 | F | 1 | 12-nov-03 | 1944 | 12.69 | 16.9 | | 16.9 | | 0.75 | 2.61 | 6.47 | 25.4 | 1363.4 | 49.5 | 25.55 | 82.0 | 349.0 | 240.0 | 52.9 | 55.0 | 166.0 | 1.07 | 0.63 | 1.70 | | 491.4 | 100.4 | | 5.22 |
| Patient n°421 | M | 1 | 27-avr-04 | 1951 | 12.99 | 12.6 | | 12.6 | | 1.03 | 1.56 | 8.08 | | 933.8 | 101.8 | 7.17 | | 397.9 | 395.0 | 38.4 | 47.0 | | | | | | 387.3 | | | |
| Patient n°422 | M | 1 | 5-déc-02 | 1925 | 1.56 | 11.4 | | 11.4 | | 0.14 | 2.16 | 5.28 | 12.0 | 887.3 | 9.3 | 93.51 | 43.0 | 354.9 | 11.4 | 60.2 | 36.0 | 121.0 | 1.33 | 0.69 | 1.93 | | 200.0 | 105.9 | 16.67 | 30.00 |
| Patient n°422 | M | 2 | 7-avr-03 | 1925 | 5.57 | 14.9 | | 14.9 | | 0.37 | 1.85 | 8.05 | | 987.0 | 0.9 | 1094.62 | | 320.7 | 260.0 | 56.2 | 42.0 | | | | | | 122.9 | | 20.84 | 29.17 |
| Patient n°423 | F | 1 | 23-mars-05 | 1913 | 1.05 | | 13.0 | 13.0 | 0.47 | 0.08 | 1.15 | 11.30 | 0.1 | 903.0 | 16.0 | 54.44 | | 263.0 | | 31.0 | | | 0.87 | 0.48 | 1.81 | 355.0 | | | | |
| Patient n°424 | F | 1 | 19-oct-04 | 1950 | 7.46 | 12.6 | | 12.6 | | 0.59 | 2.01 | 6.27 | 1.3 | 610.0 | 113.0 | 3.40 | | 316.0 | 640.0 | 25.0 | 42.0 | | | | | 504.0 | | | | |
| Patient n°425 | F | 1 | 20-sept-04 | 1960 | 7.04 | 13.1 | | 13.1 | | 0.54 | 2.09 | 6.27 | 0.7 | 772.0 | 65.0 | 9.88 | | 325.0 | 680.0 | 42.2 | 42.0 | | | | | | 37.9 | | | |
| Patient n°426 | F | 1 | 29-sept-04 | 1952 | 9.78 | 11.9 | | 11.9 | | 0.82 | 1.71 | 6.96 | 0.8 | 1004.0 | 49.0 | 18.49 | | 432.0 | 455.0 | | 33.0 | | | | | | | | 12.60 | 29.00 |
| Patient n°426 | F | 2 | 23-févr-05 | 1952 | 10.35 | | 11.2 | 11.2 | | 0.92 | 1.45 | 7.72 | 0.6 | 905.0 | 15.0 | 58.33 | | 421.0 | | 41.3 | 34.0 | | | | | | | | | 31.60 |
| Patient n°427 | F | 1 | 21-sept-04 | 1952 | 2.79 | 17.6 | | 17.6 | | 0.16 | 2.19 | 8.04 | 0.7 | 1031.0 | 117.0 | 6.81 | | 349.0 | 457.5 | 32.8 | 59.0 | | | | | | 42.9 | | | |
| Patient n°428 | F | 2 | 18-nov-03 | 1952 | 13.46 | 13.0 | | 13.0 | | 1.04 | 2.25 | 5.78 | | 947.3 | 134.3 | 5.05 | | 358.2 | 230.0 | 51.3 | 42.0 | | | | | | 112.8 | | | |
| Patient n°429 | F | 1 | 31-août-04 | 1932 | 9.61 | 13.6 | | 13.6 | | 0.71 | 2.35 | 5.79 | 0.2 | 862.0 | 52.0 | 14.58 | 35.0 | 345.0 | 345.0 | 47.2 | 46.0 | 90.0 | 1.35 | 0.64 | 2.11 | | 29.5 | 80.0 | 17.63 | 21.16 |
| Patient n°430 | F | 2 | 18-janv-05 | 1932 | 12.43 | 17.7 | | 17.7 | | 0.70 | 2.45 | 7.22 | 0.5 | 1040.0 | 104.0 | 8.00 | | 337.0 | | 45.8 | 46.0 | | | | | | | | | 19.21 |
| Patient n°431 | M | 1 | 18-juin-03 | 1954 | 6.46 | 8.9 | | 8.9 | | 0.73 | 1.79 | 4.97 | | 924.4 | 106.9 | 6.64 | | 331.1 | 325.0 | 61.3 | 58.0 | | | | | | 82.4 | | | |
| Patient n°432 | F | 1 | 12-avr-05 | 1962 | 8.44 | | 15.6 | 15.6 | 0.37 | 0.54 | 1.83 | 8.52 | 0.4 | 883.0 | 46.0 | 17.20 | | 344.0 | | 64.0 | | | | | | 395.0 | | | | |
| Patient n°433 | M | 1 | 18-mai-04 | 1928 | 8.61 | 13.3 | | 13.3 | | 0.65 | 1.44 | 9.24 | | 1057.0 | 78.1 | 11.54 | 59.0 | 314.4 | 485.0 | 26.9 | 56.0 | 97.0 | | | | | 54.1 | | | |

| Patient | Sexe | N | Date | Année | c1 | c2 | c3 | c4 | c5 | c6 | c7 | c8 | c9 | c10 | c11 | c12 | c13 | c14 | c15 | c16 | c17 | c18 | c19 | c20 | c21 | c22 | c23 | c24 | c25 | c26 |
|---|---|---|---|---|---|---|---|---|---|---|---|---|---|---|---|---|---|---|---|---|---|---|---|---|---|---|---|---|---|---|
| n°434 | F | 1 | 18-mai-04 | 1939 | 11.13 | 16.0 | | 16.0 | | 0.70 | 2.66 | 6.02 | | 1179.1 | 19.4 | 58.75 | | 269.6 | 405.0 | 45.1 | 40.0 | 148.0 | 1.11 | 0.69 | 1.61 | | 68.3 | | | |
| n°435 | M | 1 | 26-févr-03 | 1942 | 17.30 | 13.6 | | 13.6 | | 1.27 | 2.20 | 7.27 | 32.0 | 1181.2 | 116.5 | 8.14 | 77.0 | 344.0 | 400.0 | 60.0 | 68.0 | 113.0 | 1.20 | 0.94 | 1.28 | 26.2 | 26.4 | 79.9 | 3.10 | 9.01 |
| n°436 | F | 1 | 25-août-03 | 1936 | 10.81 | 18.1 | | 18.1 | | 0.60 | 2.47 | 7.33 | 118.5 | 954.9 | 26.8 | 33.58 | 68.0 | 412.1 | 105.0 | 52.4 | 56.0 | 83.0 | 1.00 | 0.66 | 1.52 | | 130.7 | 96.2 | | 1.86 |
| n°437 | F | 1 | 27-sept-04 | 1966 | 8.21 | 12.8 | | 12.8 | | 0.64 | 1.83 | 6.99 | 1.0 | 922.0 | 25.0 | 34.88 | | 339.0 | 377.5 | 54.5 | 31.0 | 82.0 | 0.85 | 0.51 | 1.67 | | 58.0 | | | |
| n°438 | F | 1 | 13-avr-04 | 1943 | 6.46 | 13.6 | | 13.6 | | 0.48 | 2.02 | 6.73 | | 699.0 | 26.3 | 24.61 | | 427.9 | 105.0 | 31.6 | 44.0 | | | | | | 97.5 | | 8.90 | 16.00 |
| n°439 | F | 1 | 28-sept-04 | 1968 | 10.08 | 11.1 | | 11.1 | | 0.91 | 1.45 | 7.66 | 0.3 | 890.0 | 25.0 | 33.60 | | 464.0 | 720.0 | 25.0 | 46.0 | | | | | | | | | |
| n°440 | F | 1 | 13-sept-04 | 1959 | 16.78 | 13.5 | | 13.5 | | 1.24 | 1.64 | 8.23 | 1.3 | 944.0 | 53.0 | 15.81 | | 257.0 | 285.0 | 63.1 | 33.0 | | | | | | 47.7 | | | |
| n°441 | F | 1 | 27-avr-05 | 1920 | 4.63 | 11.2 | 11.2 | 11.2 | 0.85 | 0.41 | 1.64 | | 0.2 | 824.0 | 141.0 | 3.84 | | 290.0 | | 49.0 | 51.0 | | 1.12 | 0.63 | 1.78 | 412.0 | | | | |
| n°442 | M | 1 | 23-juin-03 | 1963 | 0.69 | 21.9 | | 21.9 | | 0.03 | 2.50 | 8.76 | | 1285.3 | 156.7 | 6.20 | | 389.6 | 275.0 | 68.4 | | | | | | | 394.9 | | | |
| n°443 | F | 1 | 27-avr-04 | 1950 | 14.24 | 15.9 | | 15.9 | | 0.90 | 2.31 | 6.88 | | 880.8 | 75.3 | 9.69 | | 389.6 | 235.0 | 52.9 | 35.0 | | | | | | 412.0 | | | |
| n°444 | M | 1 | 5-févr-03 | 1945 | 6.32 | 24.2 | | 24.2 | | 0.26 | 2.85 | 8.49 | 14.4 | 730.3 | 0.5 | 1402.35 | 47.0 | 419.3 | 225.0 | 73.3 | 59.0 | 140.0 | 1.03 | 0.77 | 1.34 | 229.0 | 196.8 | 231.9 | 7.44 | 9.67 |
| n°445 | F | 1 | 7-avr-03 | 1945 | 15.52 | 18.0 | | 18.0 | | 0.86 | 2.22 | 8.11 | | 938.4 | 23.2 | 38.41 | | 438.2 | 515.0 | 65.8 | 66.0 | | | | | | 109.8 | 172.9 | | |
| n°446 | F | 1 | 23-mars-05 | 1916 | 0.82 | 10.7 | 10.7 | 10.7 | 0.25 | 0.08 | 0.81 | 13.21 | 0.2 | 651.0 | 4.0 | 160.75 | 112.0 | | | 27.0 | | | | | | 290.0 | | | | |
| n°447 | M | 1 | 23-juin-04 | 1951 | 9.07 | 13.1 | | 13.1 | | 0.69 | 2.41 | 5.44 | 0.3 | 888.0 | 18.0 | 47.33 | 84.0 | 309.0 | 175.0 | 23.8 | 64.0 | 108.0 | 1.11 | 0.73 | 1.52 | | 63.1 | 193.0 | 6.69 | 14.05 |
| n°448 | F | 1 | 19-mai-04 | 1969 | 4.48 | 9.8 | | 9.8 | | 0.46 | 1.41 | 6.95 | | 842.5 | 47.0 | 15.92 | | 372.4 | 250.0 | 42.9 | 25.0 | 96.0 | 1.02 | 0.69 | 1.48 | | 48.0 | | | |
| n°449 | F | 1 | 11-mai-04 | 1966 | 13.45 | 15.1 | | 15.1 | | 0.89 | 2.02 | 7.47 | 35.0 | 1244.6 | 59.6 | 18.89 | 35.0 | 448.2 | | | 40.0 | 130.0 | | | | | 91.5 | | | |
| n°450 | F | 1 | 21-juin-05 | 1955 | 9.25 | 13.3 | 13.3 | 13.3 | 1.45 | 0.70 | 2.27 | 5.86 | 0.9 | 926.0 | 63.0 | 12.70 | | 257.0 | | 50.0 | | | | | | 142.0 | | | | |
| n°451 | F | 1 | 1-sept-04 | 1943 | 12.13 | 20.7 | | 20.7 | | 0.59 | 2.66 | 7.78 | 0.8 | 653.0 | 28.0 | 21.32 | | 282.0 | 615.0 | 47.7 | 60.0 | 149.0 | 1.09 | 0.74 | 1.47 | | 42.9 | | | |
| n°452 | F | 1 | 25-mai-04 | 1951 | 10.40 | 28.8 | | 28.8 | | 0.36 | 2.39 | 12.05 | | 763.5 | 31.4 | 22.28 | 56.0 | 430.2 | 240.0 | 101.1 | 41.0 | 225.0 | | | | | 68.6 | | | |
| n°453 | M | 1 | 22-déc-04 | 1972 | 5.06 | 8.5 | | 8.5 | | 0.60 | 1.43 | 5.94 | 0.2 | 902.0 | 3.0 | 298.67 | 57.0 | 364.0 | | 20.2 | 65.0 | 92.0 | 1.50 | 0.58 | 1.81 | | 45.5 | 89.0 | | 23.00 |
| n°454 | M | 1 | 27-avr-05 | 1914 | 2.15 | 13.6 | 13.6 | 13.6 | 1.49 | 0.16 | 1.92 | | 0.3 | 1008.0 | 121.0 | 6.33 | | 303.0 | | 60.0 | | | 0.81 | 0.68 | 1.19 | 268.0 | | | | |
| n°455 | M | 1 | 21-janv-04 | 1939 | 10.04 | 11.8 | | 11.8 | | 0.85 | 21.80 | 5.41 | 0.7 | 938.4 | 103.7 | 7.05 | 57.0 | 327.1 | 390.0 | 39.1 | 61.0 | 87.0 | 0.58 | 0.57 | 1.02 | | 65.4 | 47.1 | 10.78 | 25.87 |
| n°456 | F | 2 | 1-sept-04 | 1954 | 14.53 | 12.0 | | 12.0 | | 1.21 | 1.82 | 6.59 | 1.5 | 883.0 | 33.0 | 24.76 | | 272.0 | 152.5 | 46.0 | 26.0 | | | | | 227.0 | | | 7.00 | 15.30 |
| n°457 | M | 1 | 14-mars-05 | 1954 | 17.52 | 14.1 | 14.1 | 14.1 | 1.12 | 1.24 | 2.03 | 6.95 | 1.2 | 837.0 | 99.0 | 6.45 | | 44.0 | 440.0 | 22.7 | 49.0 | 84.0 | 0.82 | 0.72 | 1.14 | | 47.7 | 64.1 | 11.11 | 19.00 |
| n°458 | F | 1 | 17-mars-04 | 1937 | 7.13 | 17.3 | | 17.3 | | 0.41 | 1.67 | 7.86 | 0.3 | 1179.0 | 51.8 | 20.77 | | 362.7 | | | 34.0 | | | | | | 41.3 | | | 23.34 |
| n°459 | M | 1 | 14-sept-04 | 1951 | 11.66 | 16.1 | | 16.1 | | 0.72 | | 9.64 | 0.4 | 1044.0 | 33.0 | 29.64 | | 266.0 | 147.5 | 48.4 | | | | | | | | | | |
| n°460 | M | 1 | 18-nov-03 | 1974 | 8.19 | 16.7 | | 16.7 | | 0.49 | 2.29 | 7.29 | 31.0 | 999.1 | 108.8 | 7.18 | 31.0 | 229.9 | 260.0 | 48.2 | 50.0 | 90.0 | 0.81 | 0.78 | 1.04 | | 302.4 | 222.7 | 7.29 | 9.48 |

EP 1 793 230 B1

| Patient | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|
| Patient n°460 | M | 1 | 5-mai-04 | 1925 | 4.49 | 15.1 | | 15.1 | | 0.30 | 2.26 | 6.68 | 0.5 | 1036.5 | 125.7 | 6.25 | 30.0 | 353.8 | 330.0 | 40.0 | 49.0 | 84.0 | 0.98 | 0.68 | 1.44 | | 101.2 | 237.6 | 18.89 | 39.67 |
| Patient n°460 | M | 2 | 9-nov-04 | 1925 | 6.07 | 14.9 | | 14.9 | | 0.41 | 2.23 | 6.68 | 1.3 | 989.0 | 11.0 | 87.91 | | 317.0 | 920.0 | 51.0 | 49.0 | | | | | | 34.0 | | | |
| Patient n°461 | F | 1 | 26-avr-05 | 1948 | 8.33 | | 10.1 | 10.1 | 0.79 | 0.82 | 1.71 | | 0.8 | 742.0 | 21.0 | 33.33 | | 363.0 | | 31.0 | | | | | | 453.0 | | | | |
| Patient n°462 | F | 1 | 20-déc-04 | 1949 | 11.14 | 13.3 | | 13.3 | | 0.84 | 1.86 | 7.15 | 0.7 | 1019.0 | 22.0 | 44.32 | | 402.0 | | 34.4 | 33.0 | | | | | 317.0 | | | | |
| Patient n°463 | F | 1 | 19-mai-04 | 1950 | 6.28 | 16.7 | | 16.7 | | 0.38 | 2.55 | 6.55 | | 1228.5 | 18.9 | 63.03 | 51.0 | 407.9 | 290.0 | 28.7 | 45.0 | 109.0 | 1.07 | 0.75 | 1.43 | | 73.7 | | | |
| Patient n°463 | F | 2 | 26-oct-04 | 1950 | 6.98 | 19.0 | | 19.0 | | 0.37 | 2.52 | 7.54 | 1.3 | 713.0 | 28.0 | 23.46 | | 386.0 | 370.0 | 75.0 | 36.0 | | | | | | 44.0 | | | |
| Patient n°464 | M | 1 | 5-juil-05 | 1957 | 7.24 | | 14.6 | 14.6 | 0.34 | 0.50 | 1.51 | 9.67 | 0.6 | 843.0 | 43.0 | 17.60 | 54.0 | 403.0 | | 58.0 | 44.0 | 134.0 | 0.80 | 0.82 | 0.98 | 214.0 | 20.0 | #### | 9.50 | 19.00 |
| Patient n°465 | F | 1 | 24-déc-03 | 1951 | 11.67 | 18.0 | | 18.0 | | 0.65 | 2.14 | 8.41 | 157.3 | 503.0 | 16.9 | 27.71 | 56.0 | 406.0 | 335.0 | 40.3 | 56.0 | 133.0 | 1.20 | 0.79 | 1.52 | | 27.0 | 38.5 | 16.93 | 11.85 |
| Patient n°466 | F | 1 | 21-juin-05 | 1984 | 7.50 | | 11.9 | 11.9 | 0.53 | 0.63 | 2.14 | 5.56 | 0.2 | 687.0 | 20.0 | 32.35 | 79.0 | 387.0 | | 47.0 | 61.0 | 94.0 | 1.71 | 0.63 | 2.71 | 1007.0 | 66.0 | 194.0 | 10.00 | 23.00 |
| Patient n°467 | M | 1 | 26-avr-05 | 1920 | 5.74 | | 10.1 | 10.1 | 0.65 | 0.57 | 1.57 | | 0.1 | 855.0 | 4.0 | 211.75 | | 311.0 | | 41.0 | | | 0.97 | 0.65 | 1.49 | 311.0 | | | | |
| Patient n°468 | F | 1 | 8-sept-04 | 1940 | 11.60 | 11.3 | | 11.3 | | 1.03 | 2.01 | 5.62 | 1.0 | 1034.0 | 73.0 | 12.16 | | 281.0 | 400.0 | 63.0 | 50.0 | | | | | | 75.0 | | | |
| Patient n°469 | F | 1 | 18-oct-04 | 1958 | 10.67 | 13.4 | | 13.4 | | 0.80 | 1.76 | 7.61 | 1.4 | 911.0 | 59.0 | 13.44 | | 372.0 | 640.0 | 54.0 | 54.0 | | | | | 132.0 | | | | |
| Patient n°470 | M | 1 | 16-juin-04 | 1951 | 2.15 | 13.8 | | 13.8 | | 0.16 | 1.66 | 8.31 | 0.3 | 864.0 | 14.0 | 59.71 | 37.0 | 443.0 | 350.0 | 8.7 | 48.0 | 95.0 | 0.85 | 0.75 | 1.13 | | 53.4 | 577.0 | 16.71 | 35.10 |
| Patient n°471 | F | 1 | 11-mai-04 | 1957 | 18.13 | 34.2 | | 34.2 | | 0.53 | 2.12 | 16.13 | | 1295.4 | 60.8 | 19.29 | 50.0 | 386.9 | 270.0 | 106.2 | 45.0 | 167.0 | | | | | 263.8 | | | |
| Patient n°471 | F | 2 | 18-oct-04 | 1957 | 14.73 | 26.9 | | 26.9 | | 0.55 | 2.28 | 11.80 | 4.6 | 885.0 | 58.0 | 13.26 | | 348.0 | 575.0 | 76.0 | 39.0 | | | | | | 61.0 | | | |
| Patient n°472 | F | 1 | 18-janv-05 | 1957 | 22.37 | 11.3 | | 11.3 | | 1.98 | 1.62 | 6.97 | 0.8 | 1082.0 | 68.0 | 13.91 | ### | 349.0 | | 46.2 | 36.0 | 210.0 | 1.03 | 0.62 | 1.66 | | 26.6 | 21.0 | | 31.41 |
| Patient n°473 | F | 1 | 7-avr-04 | 1966 | 0.00 | 11.6 | | 11.6 | | 0.00 | 1.60 | 7.25 | 0.4 | 1475.0 | 54.0 | 25.31 | 73.0 | 380.2 | 175.0 | 35.8 | 41.0 | 116.0 | 1.15 | 0.61 | 1.89 | | 116.0 | #### | 11.00 | 23.12 |
| Patient n°474 | F | 1 | 29-mars-05 | 1966 | 8.47 | | 14.6 | 14.6 | 0.58 | 2.05 | 7.12 | 0.8 | 1114.0 | 140.0 | 5.96 | | | | 41.0 | | | | | | 358.0 | | | | |
| Patient n°475 | F | 1 | 15-juin-04 | 1940 | 16.15 | 17.7 | | 17.7 | | 0.91 | 2.84 | 6.23 | 1.1 | 752.0 | 39.0 | 17.28 | | 387.0 | 385.0 | 26.2 | 42.0 | | | | | | 107.8 | | | |
| Patient n°476 | F | 1 | 20-sept-05 | 1989 | 7.26 | | 10.0 | 10.0 | 0.42 | 0.72 | 1.69 | 6.09 | 1.9 | 707.0 | 127.0 | 3.57 | 36.0 | 380.0 | | 68.0 | 25.0 | 94.0 | 0.85 | 0.69 | 1.23 | 341.0 | 51.0 | | 23.00 | 2.30 |
| Patient n°477 | F | 1 | 29-juin-04 | 1944 | 12.09 | 28.0 | | 28.0 | | 0.43 | 1.89 | 14.81 | 0.3 | 691.0 | 20.0 | 32.55 | | 580.0 | | | 55.0 | | | | | | 85.1 | | | |
| Patient n°478 | F | 1 | 13-sept-04 | 1941 | 12.22 | 16.1 | | 16.1 | | 0.76 | 2.40 | 6.71 | 0.6 | 652.0 | 72.0 | 7.06 | | 284.0 | 300.0 | 58.2 | 38.0 | | | | | | 40.4 | | | |
| Patient n°479 | M | 1 | 13-sept-04 | 1937 | 6.53 | 16.3 | | 16.3 | | 0.40 | 1.96 | 8.32 | 0.8 | 848.0 | 49.0 | 15.31 | | 308.0 | 515.0 | 50.0 | 58.0 | | | | | | 66.4 | | | |
| Patient n°480 | F | 1 | 31-août-04 | 1934 | 8.83 | 19.3 | | 19.3 | | 0.46 | 3.14 | 6.15 | 1.0 | 788.0 | 46.0 | 15.13 | | 419.0 | 607.5 | 48.0 | 58.0 | | | | | | 44.3 | | | |
| Patient n°481 | M | 1 | 31-août-04 | 1925 | 7.56 | 15.9 | | 15.9 | | 0.48 | 2.52 | 6.31 | 0.3 | 657.0 | 64.0 | 8.27 | 67.0 | 233.0 | 790.0 | 48.0 | | 125.0 | 1.31 | 0.59 | 2.22 | | 62.4 | 66.0 | | 2.11 |
| Patient n°482 | F | 1 | 12-oct-04 | 1964 | 9.86 | 10.1 | | 10.1 | | 0.98 | 1.48 | 6.82 | 0.6 | 1086.0 | 66.0 | 14.45 | | 389.0 | 280.0 | 29.0 | 34.0 | | | | | | | | 8.30 | 10.00 |
| Patient n°483 | M | 1 | 1-sept-04 | 1963 | 7.92 | 12.5 | | 12.5 | | 0.63 | 2.03 | 6.16 | 0.4 | 908.0 | 41.0 | 20.15 | | 337.0 | 505.0 | 44.4 | 56.0 | | | | | | 33.8 | | | |

| | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|
| Patient n°484 | F | 1 | 28-sept-04 | 1919 | 9,27 | 15,2 | | 15,2 | 0,61 | 2,57 | 5,91 | 0,4 | 1034,0 | 37,0 | 25,95 | | 307,0 | 360,0 | | 31,0 | | | | | | | 14,40 | | 13,00 |
| Patient n°485 | F | 1 | 23-nov-04 | 1950 | 10,40 | 18,1 | | 18,1 | 0,57 | 2,56 | 7,07 | 0,5 | 1024,0 | 14,0 | 71,14 | 54,0 | 318,0 | | 56,0 | 44,0 | 118,0 | 1,20 | 0,66 | 1,82 | | 168,3 | 392,0 | | 8,69 |
| Patient n°486 | M | 1 | 25-oct-04 | 1944 | 12,68 | 10,3 | | 10,3 | 1,23 | 1,61 | 6,40 | 0,8 | 745,0 | 2,0 | 370,50 | | 346,0 | 635,0 | 56,0 | 49,0 | | | | | | 319,0 | | | |
| Patient n°487 | F | 1 | 14-sept-04 | 1944 | 10,33 | 14,9 | | 14,9 | 0,69 | 2,04 | 7,30 | 1,5 | 719,0 | 9,0 | 77,89 | | 334,0 | 225,0 | 55,5 | 40,0 | | | | | | 54,5 | | | |
| Patient n°487 | F | 2 | 1-févr-05 | 1944 | 8,38 | | 16,4 | 16,4 | 0,51 | 2,51 | 6,53 | 1,7 | 912,0 | 79,0 | 9,54 | | 367,0 | | 55,6 | 37,0 | | | | | | 649,0 | | | |
| Patient n°487 | F | 3 | 6-sept-05 | 1944 | 12,95 | | 21,2 | 21,2 | 0,61 | 2,67 | 7,94 | 2,1 | 722,0 | 4,0 | 178,50 | | 366,0 | | | 73,0 | | | | | | 407,0 | | | |
| Patient n°488 | M | 1 | 6-juil-04 | 1979 | 10,84 | 9,3 | | 9,3 | 1,17 | 1,09 | 8,53 | 0,3 | 881,0 | 3,0 | 291,67 | | 543,0 | | | 31,0 | | | | | | 66,5 | | | |
| Patient n°489 | M | 1 | 6-juil-04 | 1948 | 9,79 | 14,1 | | 14,1 | 0,69 | 1,72 | 8,20 | 0,4 | 761,0 | 7,0 | 106,71 | | 424,0 | | | 50,0 | | | | | | 160,1 | | | |
| Patient n°489 | M | 2 | 19-janv-05 | 1948 | 9,14 | | 15,4 | 15,4 | 0,59 | 2,15 | 7,16 | 1,3 | 649,0 | 28,0 | 21,18 | | 373,0 | | 44,9 | 52,0 | | | | | | 29,7 | | | |
| Patient n°490 | F | 1 | 6-juil-04 | 1953 | 9,12 | 12,5 | | 12,5 | 0,73 | 1,52 | 8,22 | 0,5 | 859,0 | 168,0 | 3,11 | | 479,0 | | | 42,0 | 99,0 | 1,05 | 0,62 | 1,69 | | 65,6 | 697,0 | | 18,31 |
| Patient n°490 | F | 2 | 17-janv-05 | 1953 | 10,27 | 11,6 | | 11,6 | 0,89 | 1,60 | 7,25 | 0,9 | 906,0 | 26,0 | 32,85 | | 361,0 | | 39,6 | 39,0 | | | | | | | | | 14,99 |
| Patient n°491 | F | 1 | 4-janv-05 | 1975 | 13,97 | 13,3 | | 13,3 | 1,05 | 2,19 | 6,07 | 1,0 | 796,0 | 115,0 | 4,92 | 44,0 | 350,0 | | 47,1 | 41,0 | 98,0 | 0,94 | 0,68 | 1,38 | | 23,3 | 891,0 | | 8,19 |
| Patient n°492 | F | 1 | 13-sept-04 | 1968 | 12,82 | 12,6 | | 12,6 | 1,02 | 2,26 | 5,57 | 0,8 | 612,0 | 12,0 | 49,00 | | 343,0 | 405,0 | 53,7 | 43,0 | | | | | | 56,1 | | | |
| Patient n°493 | M | 1 | 2-nov-04 | 1939 | 1,73 | 13,2 | | 13,2 | 0,13 | 2,51 | 5,26 | 0,3 | 931,0 | 157,0 | 3,93 | 69,0 | 321,0 | 625,0 | 57,0 | 47,0 | 127,0 | 1,13 | 0,68 | 1,66 | | 48,0 | 369,0 | | 13,00 |
| Patient n°494 | F | 1 | 2-nov-04 | 1942 | 7,96 | 11,7 | | 11,7 | 0,68 | 2,12 | 5,52 | 0,6 | 954,0 | 112,0 | 6,52 | 42,0 | 303,0 | 575,0 | 44,0 | 51,0 | 134,0 | 1,36 | 0,83 | 1,64 | | 48,0 | 424,0 | | 9,00 |
| Patient n°495 | F | 1 | 29-nov-04 | 1943 | 10,76 | 13,1 | | 13,1 | 0,82 | 1,73 | 7,57 | 0,5 | 1104,0 | 26,0 | 40,46 | | 290,0 | | 27,5 | 55,0 | | | | | | 371,0 | | | |
| Patient n°496 | M | 1 | 8-sept-04 | 1958 | 7,70 | 17,4 | | 17,4 | 0,44 | 2,36 | 7,37 | 0,5 | 916,0 | 35,0 | 24,17 | | 333,0 | 450,0 | 58,2 | 66,0 | | | | | | 53,3 | | | |
| Patient n°497 | F | 1 | 1-juin-04 | 1955 | 7,77 | 10,8 | | 10,8 | 0,72 | 1,38 | 7,83 | 0,9 | 972,7 | 120,8 | 6,05 | 43,0 | 412,0 | 325,0 | 36,0 | 41,0 | 93,0 | 0,88 | 0,59 | 1,49 | | 79,1 | 104,0 | | 26,10 |
| Patient n°498 | F | 1 | 19-oct-04 | 1955 | 8,60 | 14,7 | | 14,7 | 0,59 | 2,67 | | 0,4 | 779,0 | 34,0 | 20,91 | 67,0 | 364,0 | 700,0 | 30,0 | 44,0 | 85,0 | 1,10 | 0,71 | 1,55 | | 61,0 | 634,0 | 6,00 | 3,50 |
| Patient n°499 | M | 1 | 9-sept-03 | 1965 | 4,64 | 15,0 | | 15,0 | 0,31 | 2,35 | 6,38 | 212,8 | 1214,6 | 158,5 | 5,66 | 59,0 | 190,8 | | 54,0 | 55,0 | 125,0 | 0,73 | 0,60 | 1,22 | | 282,9 | 354,8 | 4,83 | 3,86 |
| Patient n°499 | M | 2 | 6-janv-04 | 1965 | 23,56 | 12,3 | | 12,3 | 1,92 | 2,03 | 6,06 | | 1207,0 | 83,5 | 12,45 | | 328,8 | 240,0 | 32,9 | 49,0 | | | | | | 41,0 | | | |
| Patient n°500 | F | 1 | 27-avr-04 | 1954 | 13,91 | 14,7 | | 14,7 | 0,95 | 2,09 | 7,03 | | 751,2 | 32,6 | 21,02 | | 421,8 | 635,0 | 41,3 | 64,0 | | | | | | 526,9 | | | |
| Patient n°500 | F | 2 | 5-mai-04 | 1954 | 13,37 | 14,4 | | 14,4 | 0,93 | 1,97 | 7,31 | | 1071,6 | 48,8 | 19,96 | 46,0 | 362,5 | 275,0 | 48,2 | 51,0 | 192,0 | | | | | 143,0 | | | |
| Patient n°501 | F | 1 | 25-août-03 | 1954 | 21,42 | 23,5 | | 23,5 | 0,91 | 2,72 | 8,64 | 70,7 | 823,5 | 44,6 | 16,47 | 48,0 | 429,3 | 185,0 | 55,6 | 60,0 | 139,0 | 1,12 | 0,71 | 1,58 | | 474,2 | 397,7 | 7,65 | 6,12 |
| Patient n°502 | F | 1 | 5-mai-04 | 1964 | 12,15 | 15,2 | | 15,2 | 0,80 | 2,04 | 7,45 | 3,4 | 971,3 | 121,6 | 5,98 | 53,0 | 384,5 | 205,0 | 41,8 | 39,0 | ### | 1,19 | 1,05 | 1,13 | | 158,4 | #### | | 7,00 |
| Patient n°502 | F | 2 | 15-juil-04 | 1964 | 20,82 | 15,9 | | 15,9 | 1,31 | 1,81 | 8,78 | | 1149,1 | 87,3 | 11,17 | | 357,1 | 360,0 | 50,4 | 42,0 | | | | | | 180,6 | 1957,8 | 13,73 | 15,10 |
| Patient n°502 | F | 2 | 22-déc-04 | 1964 | 8,77 | 15,4 | | 15,4 | 0,57 | 2,32 | 6,64 | 0,7 | 759,0 | 3,0 | 251,00 | 67,0 | 403,0 | | 36,2 | 47,0 | 100,0 | 1,02 | 0,72 | 1,42 | | 61,8 | #### | | |

| Patient | Sexe | N | Date | Année | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|
| Patient n°503 | F | 1 | 18-mars-03 | 1964 | 7.61 | 11.6 | | 11.6 | | 0.66 | 2.17 | 5.35 | 41.0 | 982.7 | 18.0 | 52.63 | 60.0 | 408.8 | 80.0 | 55.8 | 37.0 | 84.0 | 0.94 | 0.81 | 1.16 | | 73.6 | 1440.5 | | 34.42 |
| Patient n°504 | F | 1 | 16-sept-03 | 1932 | 9.21 | 18.6 | | 18.6 | | 0.50 | 2.55 | 7.29 | 60.2 | 1106.0 | 133.4 | 6.29 | 37.0 | 331.6 | | 55.8 | 58.0 | 72.0 | 1.16 | 0.74 | 1.57 | | 655.5 | 118.1 | 6.05 | 4.84 |
| Patient n°504 | F | 2 | 31-mars-04 | 1932 | 7.13 | 17.0 | | 17.0 | | 0.42 | 2.31 | 7.45 | 0.6 | 757.2 | 23.6 | 30.10 | | 360.2 | 270.0 | 45.3 | 49.0 | | | | | | 126.0 | | | |
| Patient n°505 | M | 1 | 15-sept-04 | 1942 | 7.30 | 16.7 | | 16.7 | | 0.44 | 2.19 | 7.63 | 0.7 | 211.0 | 113.0 | -0.13 | 53.0 | 297.0 | 627.5 | 72.4 | 62.0 | 141.0 | 1.47 | 0.70 | 2.10 | | 70.1 | 165.0 | | 35.00 |
| Patient n°506 | F | 1 | 16-juin-04 | 1927 | 8.33 | 10.4 | | 10.4 | | 0.80 | 1.80 | 5.78 | 0.3 | 980.0 | 90.0 | 8.89 | 34.0 | 289.0 | 1140.0 | 369.1 | 51.0 | 68.0 | 0.93 | 0.56 | 1.66 | | 43.5 | | 15.10 | 16.61 |
| Patient n°507 | M | 1 | 3-févr-04 | 1955 | 10.24 | 14.7 | | 14.7 | | 0.70 | 2.16 | 6.81 | 0.6 | 832.1 | 82.1 | 8.74 | 47.0 | 408.2 | 680.0 | 40.9 | 70.0 | 102.0 | 0.76 | 0.60 | 1.27 | | 103.0 | #### | 9.05 | 15.39 |
| Patient n°508 | M | 1 | 29-mars-05 | 1951 | 8.79 | | 11.5 | 11.5 | 0.63 | 0.76 | 1.87 | 6.15 | 0.4 | 951.0 | 78.0 | 10.19 | | | | 40.0 | | | | | | | 404.0 | | | |
| Patient n°509 | M | 1 | 7-sept-04 | 1941 | 8.47 | 10.6 | | 10.6 | | 0.80 | 1.82 | 5.80 | 0.2 | 600.0 | 13.0 | 44.15 | | 315.0 | 345.0 | 45.0 | 49.0 | | | | | | 62.5 | | | |
| Patient n°509 | M | 2 | 2-mars-05 | 1941 | 10.99 | | 12.1 | 12.1 | 0.68 | 0.91 | 1.83 | | 0.6 | 535.0 | 15.0 | 33.67 | | | | 48.0 | | | | | | 213.0 | | | | |
| Patient n°510 | F | 1 | 7-sept-04 | 1949 | 9.97 | 18.7 | | 18.7 | | 0.53 | 2.20 | 8.50 | 1.2 | 1014.0 | 17.0 | 57.65 | | 324.0 | 260.0 | 83.5 | 32.0 | | | | | | 56.5 | | | |
| Patient n°511 | F | 1 | 29-sept-04 | 1947 | 8.62 | 16.2 | | 16.2 | | 0.53 | 2.57 | 6.30 | 0.2 | 1073.0 | 19.0 | 54.47 | | 344.0 | 545.0 | 50.0 | 46.0 | | | | | 326.0 | | | | |
| Patient n°512 | M | 1 | 29-sept-04 | 1941 | 8.96 | 14.4 | | 14.4 | | 0.62 | | | 1.0 | 924.0 | 37.0 | 22.97 | | 384.0 | 460.0 | 59.0 | | | | | | 170.0 | | | | |
| Patient n°513 | F | 1 | 27-oct-04 | 1946 | 8.53 | 17.7 | | 17.7 | | 0.48 | 2.74 | 6.46 | | 883.0 | 40.0 | 20.08 | | 316.0 | 625.0 | 66.0 | 47.0 | | | | | 273.0 | | | | |
| Patient n°514 | F | 1 | 21-avr-04 | 1939 | 10.74 | 14.6 | | 14.6 | | 0.74 | 1.98 | 7.37 | 3.1 | 693.9 | 66.3 | 8.46 | | 473.6 | 210.0 | 46.4 | 45.0 | | | | | | 378.2 | | | |
| Patient n°514 | F | 2 | 5-oct-04 | 1939 | 14.88 | 13.4 | | 13.4 | | 1.11 | 0.92 | 14.56 | 27.1 | 570.0 | 32.0 | 15.81 | 56.0 | 313.0 | 620.0 | 40.0 | 14.0 | | | | | | 41.0 | | | |
| Patient n°515 | F | 1 | 7-oct-03 | 1948 | 9.14 | 18.9 | | 18.9 | | 0.48 | 3.65 | 5.18 | 2.1 | 1039.0 | 164.2 | 4.33 | | | 40.0 | 49.8 | | | 1.07 | 0.60 | 1.78 | 306.2 | 252.3 | 329.7 | 9.04 | 11.75 |
| Patient n°516 | M | 1 | 25-nov-03 | 1943 | 10.14 | 27.8 | 23.5 | 27.8 | | 0.36 | 2.52 | 11.03 | 0.2 | 1077.2 | 129.4 | 6.33 | | 105.2 | 605.0 | 41.1 | 83.0 | 121.0 | 0.83 | 0.69 | 1.20 | 358.0 | 384.2 | 334.4 | | |
| Patient n°517 | F | 1 | 13-avr-05 | 1952 | 14.41 | | 13.5 | 13.5 | 0.52 | 0.61 | 2.09 | | | 535.0 | 35.0 | 13.29 | 77.0 | | | 72.4 | | 139.0 | 0.92 | 0.56 | 1.64 | | | | | |
| Patient n°518 | F | 1 | 13-avr-05 | 1951 | 4.17 | 15.5 | | 15.5 | | 0.31 | 2.11 | | 2.1 | 861.0 | 85.0 | 8.13 | | 368.0 | | | | | | | | | | | 8.00 | 8.70 |
| Patient n°519 | F | 1 | 14-déc-04 | 1931 | 13.27 | 22.5 | | 22.5 | | 0.86 | 2.53 | | 0.2 | 988.0 | 2.0 | 492.00 | | 329.0 | 875.0 | 52.0 | 54.0 | | | | | 669.0 | | | 7.60 | 4.60 |
| Patient n°520 | F | 1 | 16-nov-04 | 1947 | 12.86 | 9.4 | | 9.4 | | 0.57 | 2.78 | 6.18 | 0.3 | 1044.0 | 12.0 | 85.00 | | 345.0 | 365.0 | 43.0 | 72.0 | | | | | | | | | |
| Patient n°521 | F | 1 | 28-avr-04 | 1932 | 12.58 | 13.5 | | 13.5 | | 1.34 | 1.52 | 5.82 | 0.6 | 924.8 | 152.3 | 4.07 | | 287.5 | 780.0 | 21.1 | 61.0 | | | | | | 26.4 | | | |
| Patient n°522 | M | 1 | 22-sept-04 | 1965 | 9.11 | 15.7 | | 15.7 | | 0.67 | 2.32 | 8.77 | 0.7 | 987.0 | 152.0 | 4.49 | | 347.0 | 190.0 | 65.0 | 55.0 | 98.0 | 0.75 | 0.62 | 1.21 | | 41.0 | | 10.54 | 18.98 |
| Patient n°523 | F | 1 | 10-déc-03 | 1960 | 16.42 | 14.9 | | 14.9 | | 1.05 | 1.79 | 8.37 | 125.5 | 801.5 | 54.9 | 12.59 | | 387.4 | 315.0 | 44.2 | 52.0 | | | | | | 184.0 | 60.9 | | |
| Patient n°523 | F | 2 | 5-mai-04 | 1960 | 12.30 | 12.8 | | 12.8 | | 0.83 | 1.78 | | | 999.9 | 42.0 | 21.80 | | 402.5 | | 30.2 | 50.0 | | | | | | 124.1 | | | |
| Patient n°524 | F | 1 | 13-déc-04 | 1958 | 13.14 | | | | | 1.03 | 1.43 | | 0.2 | 864.0 | 25.0 | 32.56 | | 322.0 | | 52.0 | 31.0 | | | | | 698.0 | | | | |
| Patient n°525 | F | 1 | 8-sept-04 | 1948 | 9.65 | 15.4 | | 15.4 | | 0.63 | 2.70 | 5.70 | 0.7 | 1013.0 | 9.0 | 110.56 | | 279.0 | 385.0 | | 52.0 | | | | | | 86.7 | | | |

EP 1 793 230 B1

|  |  |  |  |  |  |  |  |  |  |  |  |  |  |  |  |  |  |  |  |  |  |  |  |  |  |  |  |  |  |  |
|---|---|---|---|---|---|---|---|---|---|---|---|---|---|---|---|---|---|---|---|---|---|---|---|---|---|---|---|---|---|---|
| Patient n°526 | M | 1 | 10-mai-04 | 1940 | 7,47 | 13,8 |  | 13,8 |  | 0,54 | 1,88 | 7,34 |  | 2064,4 | 51,8 | 37,82 |  |  |  |  |  | 117,0 |  |  |  |  | 207,1 |  |  |  |
| Patient n°527 | F | 1 | 14-oct-03 | 1952 | 9,24 | 15,8 |  | 15,8 |  | 0,58 | 2,33 | 6,78 |  | 931,6 | 65,7 | 12,19 |  | 411,8 | 35,0 | 44,4 | 34,0 |  |  |  |  |  | 246,0 |  |  |  |
| Patient n°528 | M | 1 | 10-nov-04 | 1965 | 23,75 | 18,9 |  | 18,9 |  | 1,26 | 1,71 | 11,05 | 1,4 | 1085,0 | 108,0 | 8,05 | 57,0 | 372,0 | 680,0 | 67,0 | 59,0 | 142,0 | 1,14 | 1,09 | 1,05 |  | 25,0 | 125,0 | 12,00 | 14,00 |
| Patient n°529 | F | 1 | 30-mars-05 | 1955 | 6,12 |  | 15,0 | 15,0 | 0,59 | 0,41 | 1,82 |  | 0,4 | 1040,0 | 26,0 | 38,00 |  |  |  | 41,0 |  | 95,0 |  |  |  |  | 311,0 |  |  |  |
| Patient n°530 | F | 1 | 10-mai-04 | 1941 | 8,03 | 10,9 |  | 10,9 |  | 0,74 | 1,42 | 7,68 |  | 1929,3 | 153,4 | 10,58 | 73,0 | 435,0 | 270,0 | 23,8 | 43,0 | 93,0 |  |  |  |  | 78,2 |  |  |  |
| Patient n°531 | F | 1 | 8-juin-04 | 1937 | 10,69 | 13,5 |  | 13,5 |  | 0,79 | 2,12 | 6,37 | 0,5 | 450,0 | 1,0 | 448,00 |  | 365,0 | 735,0 | 28,9 | 62,0 |  |  |  |  |  | 104,0 |  |  |  |
| Patient n°532 | F | 1 | 13-oct-04 | 1928 | 10,36 | 17,4 |  | 17,4 |  | 0,60 | 2,56 |  | 1,0 | 884,0 | 76,0 | 9,63 |  | 314,0 | 630,0 | 63,0 | 35,0 |  |  |  |  |  | 315,0 |  |  |  |
| Patient n°533 | M | 1 | 7-juin-05 | 1946 | 4,23 |  | 22,4 | 22,4 | 1,16 | 0,19 | 2,76 | 8,12 | 0,5 | 948,0 | 113,0 | 6,39 |  | 300,0 |  | 55,0 |  | 133,0 | 0,91 | 0,73 | 1,25 | 148,0 |  |  |  |  |
| Patient n°534 | F | 1 | 13-sept-04 | 1920 | 7,64 | 34,8 |  | 34,8 |  | 0,22 | 2,80 | 12,43 | 0,9 | 832,0 | 81,0 | 8,27 |  | 280,0 | 490,0 | 83,1 | 55,0 |  |  |  |  |  | 70,3 |  |  |  |
| Patient n°535 | F | 1 | 6-oct-04 | 1947 | 12,78 | 13,1 |  | 13,1 |  | 0,98 |  |  | 1,7 | 1035,0 | 72,0 | 12,38 |  |  | 525,0 | 57,0 |  |  |  |  |  |  | 237,0 |  |  |  |
| Patient n°535 | F | 2 | 22-mars-05 | 1947 | 11,61 |  | 16,9 | 16,9 | 0,55 | 0,69 |  |  | 1,4 | 871,0 | 16,0 | 52,44 |  |  | 41,0 |  |  |  |  |  |  |  | 368,0 |  |  |  |
| Patient n°536 | F | 1 | 23-sept-03 | 1937 | 5,62 | 20,9 |  | 20,9 |  | 0,27 | 2,18 | 9,59 | 36,9 | 954,2 | 96,9 | 7,85 | 34,0 | 383,1 |  | 58,9 | 38,0 | 143,0 | 0,87 | 0,60 | 1,45 |  | 198,7 | 67,5 |  | 9,59 |
| Patient n°536 | F | 2 | 3-févr-04 | 1937 | 15,05 | 19,0 |  | 19,0 |  | 0,79 | 2,71 | 7,01 |  | 1049,5 | 117,0 | 6,97 |  | 395,4 | 160,0 | 45,8 | 40,0 |  |  |  |  |  | 131,3 |  |  |  |
| Patient n°536 | F | 3 | 22-mars-05 | 1937 | 12,20 |  | 28,5 | 28,5 | 1,64 | 0,43 | 2,52 |  | 0,7 | 923,0 | 86,0 | 8,73 |  |  | 60,0 |  |  |  |  |  |  |  | 70,0 |  |  |  |
| Patient n°537 | M | 1 | 13-avr-04 | 1967 | 7,59 | 18,4 |  | 18,4 |  | 0,41 | 2,52 | 7,30 |  | 722,0 | 35,5 | 18,33 |  | 443,4 | 600,0 | 28,9 | 72,0 |  |  |  |  |  | 145,0 |  |  |  |
| Patient n°538 | F | 1 | 26-avr-05 | 1922 | 15,78 |  | 13,8 | 13,8 | 0,80 | 1,14 | 2,38 |  | 0,7 | 807,0 | 24,0 | 31,63 |  | 220,0 |  | 71,0 |  |  | 1,19 | 0,39 | 3,05 | 647,0 |  |  |  |  |
| Patient n°539 | F | 1 | 20-sept-04 | 1931 | 7,53 | 19,4 |  | 19,4 |  | 0,39 | 1,78 |  | 0,1 | 1170,0 | 97,0 | 10,06 |  | 253,0 | 660,0 | 34,6 | 48,0 |  |  |  |  |  | 48,9 |  |  |  |
| Patient n°540 | M | 1 | 29-oct-03 | 1948 | 1,25 | 18,9 |  | 18,9 |  | 0,07 | 2,10 | 9,00 | 15,4 | 606,3 | 116,0 | 3,23 | 73,0 | 405,9 | 395,0 | 51,3 | 83,0 | 95,0 | 0,99 | 0,71 | 1,39 | 265,0 | 211,6 | 441,7 | 3,25 | 6,49 |
| Patient n°540 | M | 2 | 31-mars-04 | 1948 | 10,26 | 20,4 |  | 20,4 |  | 0,50 | 2,03 | 10,05 |  | 729,3 | 11,1 | 63,76 |  | 361,0 | 945,0 | 50,4 | 85,0 |  |  |  |  |  | 110,4 |  |  |  |
| Patient n°541 | F | 1 | 13-sept-04 | 1935 | 11,76 | 15,2 |  | 15,2 |  | 0,77 | 2,17 | 7,01 | 0,8 | 1080,0 | 101,0 | 8,69 |  | 252,0 | 355,0 | 51,0 | 39,0 |  |  |  |  |  | 71,7 |  |  |  |
| Patient n°542 | F | 1 | 22-sept-04 | 1946 | 15,12 | 12,7 |  | 12,7 |  | 1,19 | 2,09 | 6,08 | 0,9 | 651,0 | 7,0 | 91,00 |  | 302,0 | 775,0 | 46,0 | 50,0 |  |  |  |  |  | 46,5 |  |  |  |
| Patient n°543 | F | 1 | 14-févr-05 | 1948 | 9,41 |  | 18,4 | 18,4 | 0,51 | 1,84 |  | 1,6 | 855,0 | 140,0 | 4,11 |  | 332,0 |  | 57,0 | 26,0 |  |  |  |  |  | 41,0 |  |  | 0,76 | 1,07 |
| Patient n°544 | F | 1 | 26-mai-04 | 1930 | 12,20 | 20,7 |  | 20,7 |  | 0,59 | 2,41 | 8,59 |  | 1379,4 | 66,5 | 18,74 | 51,0 | 346,4 | 250,0 | 72,2 | 42,0 | 107,0 | 0,82 | 0,67 | 1,22 |  | 54,3 |  |  |  |
| Patient n°545 | F | 1 | 20-sept-04 | 1960 | 8,84 | 14,3 |  | 14,3 |  | 0,62 | 2,25 | 6,36 | 0,8 | 994,0 | 18,0 | 53,22 | 60,0 | 316,0 | 398,0 | 56,0 | 34,0 | 99,0 | 0,99 | 0,65 | 1,52 |  | 38,0 | 1120,0 | 8,70 | 16,50 |
| Patient n°546 | M | 1 | 9-sept-03 | 1967 | 11,46 | 13,7 |  | 13,7 |  | 0,84 | 1,77 | 7,74 | 8,7 | 966,9 | 80,9 | 9,95 | 45,0 | 448,2 | nd | 56,2 | 42,0 | 88,0 | 0,80 | 0,61 | 1,31 |  | 368,1 | 204,6 | 4,98 | 4,98 |
| Patient n°547 | M | 1 | 20-sept-04 | 1934 | 1,00 | 12,6 |  | 12,6 |  | 0,08 | 1,72 |  |  | 1024,0 | 93,0 | 9,01 |  | 279,0 | 410,0 | 56,4 | 46,0 |  |  |  |  |  | 50,1 |  |  |  |
| Patient n°548 | M | 1 | 20-sept-04 | 1952 | 3,31 | 17,3 |  | 17,3 |  | 0,19 | 2,08 |  | 0,3 | 805,0 | 35,0 | 21,00 |  | 333,0 | 645,0 | 78,2 | 57,0 |  |  |  |  |  | 63,5 |  |  |  |

| Patient | | N° | Date | Year | 1.42 | 14.3 | 14.3 | 0.10 | 1.92 | | | 766.0 | 20.0 | 36.30 | 73.0 | 381.0 | 43.1 | 73.0 | 96.0 | 0.56 | 0.70 | 0.80 | | 34.6 | 991.0 | 0.09 | 0.15 |
|---|---|---|---|---|---|---|---|---|---|---|---|---|---|---|---|---|---|---|---|---|---|---|---|---|---|---|---|
| Patient n°549 | M | | 1-févr-05 | 1951 | 1.42 | 14.3 | 14.3 | 0.10 | 1.92 | | | 766.0 | 20.0 | 36.30 | 73.0 | 381.0 | 43.1 | 73.0 | 96.0 | 0.56 | 0.70 | 0.80 | | 34.6 | 991.0 | 0.09 | 0.15 |
| Patient n°550 | M | 1 | 9-nov-04 | 1945 | 8.17 | 16.9 | 16.9 | 0.48 | 2.14 | | 0.2 | 940.0 | 2.0 | 468.00 | 38.0 | 327.0 | 60.0 | 68.0 | | 0.71 | 0.51 | | 187.0 | 40.0 | 111.0 | 8.60 | 10.40 |
| Patient n°551 | M | 1 | 28-sept-04 | 1949 | 7.79 | 14.7 | 14.7 | 0.53 | 2.00 | 5.43 | 0.2 | 1043.0 | 41.0 | 23.44 | 56.0 | 393.0 | 55.0 | 53.0 | 78.0 | 1.14 | 0.85 | 1.39 | | 154.0 | | 8.19 | 19.66 |
| Patient n°552 | M | 1 | 10-déc-03 | 1974 | 15.43 | 17.1 | 17.1 | 0.90 | 3.15 | | 0.2 | 913.5 | 18.4 | 47.65 | | 409.7 | 49.8 | 54.0 | 89.0 | | | 1.34 | | 204.8 | | | |
| Patient n°552 | M | 2 | 13-juil-04 | 1974 | 10.96 | 11.7 | 11.7 | 0.94 | 1.97 | 8.76 | 66.2 | 979.0 | 155.0 | 4.32 | 75.0 | 407.0 | 38.6 | 57.0 | | 0.98 | 0.68 | 1.44 | | 26.7 | 191.2 | 10.55 | 20.04 |
| Patient n°553 | F | 1 | 13-janv-04 | 1973 | 9.10 | 15.6 | 15.6 | 0.58 | 1.78 | 7.11 | 0.4 | 778.9 | 96.6 | 6.06 | | 391.9 | 29.1 | 59.0 | 93.0 | | | | | 36.0 | | | |
| Patient n°554 | F | 1 | 31-août-04 | 1967 | 11.88 | 14.8 | 14.8 | 0.80 | 2.08 | | 0.4 | 622.0 | 9.0 | 67.11 | | 345.0 | 47.2 | 36.0 | | 1.02 | 0.64 | 1.59 | | 50.0 | 564.0 | 10.00 | 1.00 |
| Patient n°555 | F | 1 | 13-oct-04 | 1937 | 16.33 | 16.0 | 16.0 | 1.02 | 2.13 | 6.21 | 0.5 | 1116.0 | 144.0 | 5.75 | 39.0 | 277.0 | 60.0 | 42.0 | 133.0 | 1.18 | 0.85 | 1.39 | 661.4 | 91.1 | 124.6 | 2.27 | 1.59 |
| Patient n°556 | F | 1 | 14-oct-03 | 1962 | 4.78 | 14.1 | 14.1 | 0.34 | 2.27 | 6.00 | 1.6 | 698.5 | 38.0 | 16.40 | 58.0 | 364.7 | 41.8 | 37.0 | 101.0 | 1.06 | 0.66 | 1.61 | | 348.1 | | 6.11 | 19.56 |
| Patient n°557 | M | 1 | 22-oct-03 | 1947 | 16.80 | 14.7 | 14.7 | 1.14 | 2.45 | 4.96 | 31.8 | 920.7 | 28.1 | 30.79 | 68.0 | 438.2 | 46.2 | 58.0 | 99.0 | 1.30 | 0.81 | 1.61 | | | 899.1 | 11.25 | 9.00 |
| Patient n°558 | F | 1 | 19-oct-04 | 1947 | 9.28 | 13.7 | 13.7 | 0.68 | 2.76 | 5.53 | 47.2 | 1007.0 | 73.0 | 11.79 | | 307.0 | 40.0 | 39.0 | 101.0 | 1.00 | 0.63 | 1.59 | 711.0 | | | | |
| Patient n°559 | F | 1 | 19-oct-04 | 1946 | 10.21 | 17.7 | 17.7 | 0.58 | 3.20 | 8.32 | 0.4 | 1166.0 | 122.0 | 7.56 | | 302.0 | 58.0 | 38.0 | 107.0 | | | | | | | 14.00 | 18.30 |
| Patient n°560 | F | 1 | 20-sept-04 | 1933 | 10.25 | 17.8 | 17.8 | 0.58 | 2.14 | 5.43 | 0.2 | 546.0 | 3.0 | 180.00 | | 272.0 | 67.5 | 63.0 | | | | | | 35.3 | | | |
| Patient n°561 | M | 1 | 20-sept-04 | 1934 | 8.78 | 13.9 | 13.9 | 0.63 | 2.56 | 8.13 | 0.8 | 733.0 | 59.0 | 10.42 | | 293.0 | 51.5 | 38.0 | | | | | | | | | |
| Patient n°562 | M | 1 | 11-oct-04 | 1944 | 5.75 | 17.0 | 17.0 | 0.34 | 2.09 | 7.37 | 0.4 | 921.0 | 22.0 | 39.86 | | 315.0 | | 56.0 | 56.0 | | | | | | | | |
| Patient n°563 | F | 1 | 11-oct-04 | 1942 | 10.13 | 15.4 | 15.4 | 0.66 | 2.09 | | 0.3 | 932.0 | 13.0 | 69.69 | | 370.0 | 38.0 | 37.0 | | | | | | | | | |
| Patient n°564 | M | 1 | 23-juin-04 | 1956 | 10.18 | 11.6 | 11.6 | 0.88 | 1.51 | 7.82 | 1.0 | 923.0 | 102.0 | 7.05 | | 371.0 | | 45.0 | | | | | 20.0 | 78.3 | | | |
| Patient n°565 | F | 1 | 21-déc-04 | 1988 | 15.61 | 9.7 | 9.7 | 1.61 | 1.08 | 6.46 | 0.4 | 715.0 | 26.0 | 25.50 | | 426.0 | 26.4 | 41.0 | | | | | 649.0 | | | | |
| Patient n°566 | F | 1 | 2-mars-05 | 1917 | 7.01 | 0.65 | 14.7 | 0.48 | 1.88 | 8.07 | 0.2 | 503.0 | 10.0 | 48.30 | | | 61.0 | | | | | | | | | | |
| Patient n°567 | F | 1 | 29-oct-03 | 1978 | 0.16 | 11.7 | 11.7 | 0.01 | 1.81 | 9.67 | 13.7 | 1118.8 | 183.2 | 4.11 | 67.0 | 479.4 | 36.2 | 49.0 | 76.0 | 0.93 | 0.62 | 1.50 | | 448.0 | 286.2 | 10.62 | 10.62 |
| Patient n°568 | M | 1 | 12-mai-04 | 1941 | 15.12 | 16.7 | 16.7 | 0.91 | 2.07 | 7.53 | 14.6 | 983.8 | 96.2 | 8.22 | 47.0 | 362.6 | 58.7 | 45.0 | 124.0 | | | | | 217.9 | | | |
| Patient n°569 | M | 1 | 12-mai-03 | 1921 | 8.66 | 17.3 | 17.3 | 0.50 | 1.79 | 6.43 | | 981.1 | 3.6 | 272.80 | 51.0 | 323.0 | 62.4 | 64.0 | 88.0 | 1.17 | 0.58 | 2.02 | | 76.9 | 76.3 | 9.78 | 16.63 |
| Patient n°570 | F | 1 | 5-mai-04 | 1932 | 14.29 | 14.9 | 14.9 | 0.96 | 1.98 | 10.04 | 1.6 | 984.7 | 134.0 | 5.35 | 30.0 | 401.4 | 33.8 | 42.0 | 126.0 | | | | | 124.9 | | | |
| Patient n°570 | F | 1 | 18-oct-04 | 1932 | 11.70 | 14.2 | 14.2 | 0.82 | 2.21 | 6.89 | 1.0 | 871.0 | 10.0 | 85.10 | | 324.0 | 56.0 | 64.0 | | 1.11 | 0.63 | 1.76 | | 77.0 | | | |
| Patient n°571 | F | 2 | 22-sept-04 | 1929 | 17.54 | 22.2 | 22.2 | 0.79 | 2.21 | 7.52 | 1.9 | 754.0 | 15.0 | 48.27 | 56.0 | 258.0 | 67.0 | 36.0 | 116.0 | | | | | 45.0 | 174.0 | 10.30 | 6.20 |
| Patient n°572 | F | 1 | 7-sept-04 | 1936 | 11.42 | 16.2 | 16.2 | 0.70 | 2.35 | | 4.2 | 1113.0 | 22.0 | 48.59 | | 245.0 | 56.0 | 51.0 | | | | | | 66.9 | | | |
| Patient n°572 | F | 2 | 25-janv-05 | 1936 | 14.99 | 19.4 | 19.4 | 0.77 | 2.58 | | | 837.0 | 162.0 | 3.17 | | 308.0 | 72.4 | 46.0 | | | | | 501.0 | | | | |

| Patient | Sexe | N | Date | Année | | | | | | | | | | | | | | | | | | | | | | | | | |
|---|---|---|---|---|---|---|---|---|---|---|---|---|---|---|---|---|---|---|---|---|---|---|---|---|---|---|---|---|---|
| Patient n°573 | F | 1 | 21-déc-04 | 1953 | 6.23 | 15.6 | | 15.6 | 0.40 | 2.02 | 7.72 | 0.4 | 740.0 | 2.0 | 368.00 | 76.0 | 380.0 | 700.0 | 38.7 | 31.0 | 63.0 | 0.83 | 0.58 | 1.43 | 570.0 | 423.1 | 307.53 | 6.77 | 6.09 |
| Patient n°574 | M | 1 | 18-juin-03 | 1938 | 10.94 | 14.2 | | 14.2 | 0.77 | 1.66 | 8.55 | 29.3 | 917.3 | 7.0 | 129.61 | 97.0 | 334.7 | 185.0 | 64.0 | 67.0 | 88.0 | 0.83 | 0.67 | 1.24 | | 36.0 | 1785.0 | 8.55 | 10.26 |
| Patient n°575 | M | 1 | 23-nov-03 | 1964 | 1.67 | 12.3 | | 12.3 | 0.14 | 1.20 | 7.59 | 46.1 | 574.5 | 77.5 | 5.41 | 45.0 | 329.9 | 475.0 | 4.9 | 57.0 | 144.0 | | | | | 201.1 | | | |
| Patient n°576 | M | 1 | 5-mai-04 | 1949 | 7.33 | 15.0 | | 15.0 | 0.49 | 2.23 | 6.73 | | 1206.6 | 122.1 | 7.89 | | 411.8 | | 42.4 | 63.0 | | | | | | | | | 14.70 |
| Patient n°577 | F | 1 | 25-janv-05 | 1951 | 10.85 | 14.3 | | 14.3 | 0.76 | 2.27 | 6.30 | 0.8 | 906.0 | 52.0 | 15.42 | | | | 49.3 | 41.0 | | | | | | | | | |
| Patient n°578 | F | 1 | 9-nov-04 | 1939 | 1.86 | 15.6 | | 15.6 | 0.12 | 1.77 | 8.81 | 0.3 | 829.0 | 20.0 | 39.45 | 57.0 | 362.0 | 635.0 | 63.0 | 53.0 | | | | | 635.0 | | | | |
| Patient n°578 | F | 2 | 4-avr-05 | 1939 | 13.27 | | 18.1 | | 0.73 | 1.60 | 11.31 | 0.2 | 907.0 | 34.0 | 24.68 | | 339.0 | | | | | | | | 291.0 | | | | |
| Patient n°579 | F | 1 | 26-oct-04 | 1933 | 13.79 | 15.4 | | 15.4 | 0.90 | 2.50 | | | 902.0 | 119.0 | 5.58 | | 307.0 | 540.0 | 62.0 | 60.0 | | | | | 332.0 | | | | |
| Patient n°580 | F | 1 | 15-sept-04 | 1952 | 11.46 | 20.1 | | 20.1 | 0.57 | 1.88 | 10.69 | 1.0 | 775.0 | 84.0 | 7.23 | | 299.0 | 317.5 | 74.6 | 36.0 | 90.0 | 1.38 | 0.74 | 1.86 | | 41.5 | | | |
| Patient n°581 | F | 1 | 18-mai-04 | 1949 | 5.85 | 13.2 | | 13.2 | 0.44 | 1.72 | 7.67 | 0.9 | 867.3 | 81.8 | 8.60 | | 395.4 | 350.0 | 33.1 | 43.0 | | | | | | 49.4 | 1517.0 | | |
| Patient n°582 | F | 1 | 13-sept-04 | 1966 | 9.21 | 17.9 | | 17.9 | 0.51 | 2.17 | 8.25 | 0.1 | 719.0 | 27.0 | 24.63 | | 333.0 | 295.0 | 68.8 | 41.0 | | | | | | 72.5 | | | |
| Patient n°583 | F | 1 | 7-sept-04 | 1947 | 1.74 | 17.7 | | 17.7 | 0.10 | 2.27 | 7.80 | 0.9 | 1080.0 | 96.0 | 9.25 | 42.0 | 321.0 | 240.0 | 63.1 | 40.0 | | | | | | 23.8 | | | |
| Patient n°584 | F | 1 | 23-nov-04 | 1936 | 7.48 | 15.0 | | 15.0 | 0.50 | 2.23 | | 0.3 | 1214.0 | 56.0 | 19.63 | | 315.0 | | 55.0 | 48.0 | | | | | 360.0 | | | | |
| Patient n°585 | F | 1 | 10-mai-04 | 1957 | 11.17 | 11.1 | | 11.1 | 1.01 | 1.55 | 7.16 | | 716.9 | 60.6 | 9.84 | | 448.6 | 255.0 | 34.0 | 48.0 | 116.0 | 1.17 | 0.67 | 1.75 | | 62.0 | | | |
| Patient n°586 | F | 1 | 12-janv-05 | 1941 | 5.86 | 11.8 | | 11.8 | 0.50 | 2.18 | | 0.7 | 778.0 | 33.0 | 21.58 | 46.0 | 355.0 | | 53.8 | 36.0 | | | | | 744.0 | | | | |
| Patient n°587 | F | 1 | 16-sept-03 | 1943 | 7.23 | 18.8 | | 18.8 | 0.38 | 2.53 | 7.43 | | 1008.4 | 103.8 | 7.71 | 51.0 | 365.3 | 135.0 | 58.7 | 67.0 | 106.0 | 1.05 | 0.69 | 1.52 | 770.4 | 218.4 | 218.4 | | 4.81 |
| Patient n°588 | M | 1 | 4-mai-04 | 1975 | 8.76 | 9.7 | | 9.7 | 0.90 | 1.62 | 5.99 | 0.5 | 1193.5 | 226.7 | 3.26 | | 415.7 | 305.0 | 21.3 | 53.0 | 93.0 | | | | | 310.9 | | | |
| Patient n°588 | M | 2 | 29-sept-04 | 1975 | 5.94 | 11.3 | | 11.3 | 0.53 | 1.80 | 6.28 | 61.0 | 1279.0 | 41.0 | 29.20 | 65.0 | 356.0 | 635.0 | 44.0 | 55.0 | | | | | 246.0 | | | |
| Patient n°589 | F | 1 | 4-mai-04 | 1947 | 12.60 | 17.1 | | 17.1 | 0.74 | 2.27 | 7.53 | | 1066.6 | 93.7 | 9.39 | | 344.9 | 330.0 | 52.4 | 59.0 | 144.0 | 1.65 | 0.71 | 2.32 | | 139.5 | 137.7 | |
| Patient n°590 | F | 1 | 12-mars-03 | 1947 | 18.00 | 15.4 | | 15.4 | 1.17 | 1.56 | 9.87 | 0.7 | 808.7 | 35.5 | 20.80 | 86.0 | 309.8 | 135.0 | 65.3 | 40.0 | 184.0 | | | | 614.0 | 57.5 | | | 1.36 |
| Patient n°590 | F | 2 | 14-oct-03 | 1947 | 12.00 | 13.3 | 0.59 | 13.3 | 0.90 | 1.17 | 11.37 | | 669.3 | 75.0 | 6.92 | | 334.6 | 140.0 | 42.2 | 36.0 | | | | | | 148.5 | | | 1.36 |
| Patient n°591 | F | 1 | 5-avr-05 | 1960 | 9.76 | 16.1 | 16.1 | 16.1 | 0.61 | 2.03 | | 46.3 | 840.0 | 63.0 | 11.33 | 35.0 | | | 48.0 | | 92.0 | 0.83 | 0.47 | 1.77 | 290.0 | | | 4.53 | |
| Patient n°592 | F | 1 | 5-mai-04 | 1955 | 12.44 | 16.4 | | 16.4 | 0.76 | 1.90 | 8.63 | | 927.1 | 87.4 | 8.61 | 66.0 | 323.7 | 275.0 | 55.6 | 47.0 | 106.0 | | | | | 125.4 | | | |
| Patient n°593 | M | 1 | 4-mai-04 | 1932 | 10.71 | 17.3 | | 17.3 | 0.62 | 1.88 | 9.20 | 1.5 | 1345.6 | 164.6 | 6.18 | | 429.6 | 280.0 | 51.8 | 56.0 | 152.0 | 0.83 | 0.92 | 0.90 | | 66.5 | | | |
| Patient n°594 | M | 1 | 22-sept-04 | 1923 | 5.40 | 22.6 | | 22.6 | 0.24 | 2.00 | 11.30 | | 963.0 | 156.0 | 4.17 | | 294.0 | 560.0 | 79.5 | 36.0 | | | | | | 69.9 | | | |
| Patient n°595 | M | 1 | 3-mai-05 | 1948 | 11.78 | 19.6 | 19.6 | | 0.60 | 1.80 | | 0.3 | 1052.0 | 179.0 | 3.88 | | | 470.0 | 68.0 | | 174.0 | | | | 76.0 | 33.0 | | | 76.00 |
| Patient n°596 | F | 1 | 20-sept-04 | 1940 | 5.14 | 16.4 | 0.23 | 16.4 | 0.31 | 2.62 | 6.26 | 0.5 | 602.0 | 16.0 | 35.63 | 44.0 | 281.0 | | 72.4 | 44.0 | | | | | | 26.1 | | | |

| | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|
| Patient n°597 | F | 1 | 5-avr-05 | 1969 | 4.81 | | 11.3 | 11.3 | 0.26 | 0.43 | 1.76 | | 0.1 | 744.0 | 29.0 | 23.66 | | | 38.0 | | 89.0 | 1.46 | 0.47 | 3.11 | 1000.0 | | | 7.50 | 12.70 | |
| Patient n°598 | F | 1 | 1-sept-04 | 1949 | 13.40 | 13.4 | | 13.4 | | 1.00 | 1.64 | 8.17 | 1.5 | 915.0 | 101.0 | 7.06 | 278.0 | 465.0 | 46.0 | 47.0 | | | | | 24.0 | | | | | |
| Patient n°599 | M | 1 | 7-sept-04 | 1930 | 11.30 | 15.9 | | 15.9 | | 0.71 | 1.94 | 8.20 | 0.5 | 922.0 | 90.0 | 8.24 | 317.0 | 810.0 | 76.0 | 69.0 | | | | | 30.2 | | | | | |
| Patient n°600 | F | 1 | 14-sept-04 | 1932 | 11.74 | 21.2 | | 21.2 | | 0.55 | 2.80 | 7.57 | 1.4 | 898.0 | 40.0 | 20.45 | 259.0 | 367.5 | 68.8 | 51.0 | 121.0 | 0.89 | 0.80 | 1.11 | 52.7 | | | | | |
| Patient n°601 | F | 1 | 21-sept-05 | 1924 | 11.99 | | 15.8 | 15.8 | 1.04 | 0.76 | 2.68 | 5.90 | 0.8 | 794.0 | 55.0 | 12.44 | 319.0 | | 85.0 | | 93.0 | | | | 63.0 | | | | | |

33

ANNEXE 2

| Abréviation | Désignation | Unité | Normes |
|---|---|---|---|
| Vit C | Vitamine C | μg/ml | 8-18 |
| Vit E | Vitamine E | μg/ml | 8-15 |
| Alpha tocoph | Alpha tocophérol | μg/ml | 8-15 |
| Gamma tocoph | Gamma tocophérol | μg/ml | 0.28 - 2.42 |
| Vit C/E | Ratio Vitamine C / Vitamine E | | 0.53 - 1.19 |
| Chol. | Cholestérol | g/l | 1.40-2.00 |
| Vit E/Chol | Ratio Vitamine E / Cholestérol | mg/g | 4.4 - 7.0 |
| βcaro | Béta-carotène | mg/l | 0.08 - 0.36 |
| GSH | Glutathion | μmol/l | 717-1110 |
| GSSG | Glutathion oxydé | μmol/l | 1.17 - 5.32 |
| Ratio G/G | Ratio Glutathion/Glutathion oxydé | | 156 - 705 |
| GPX | Glutathion Péroxydase | UI/g Hb | 30-60 |
| Prot Thiol | Protéines Thiols | μmol/l | 310 -523 |
| Cap Hydro | Capacité Antioxydante totale hydrophile | nMol eq. aAAml | 268 - 361 |
| Cap Lipo | Capacité Antioxydante totale lipophile | nMol eq. Trol/ml | 25 - 50 |
| AcideUrique | Acide urique | mg/l | 22 - 59 |
| Sel | Sélénium | μg/l | 94 - 130 |
| Cu | Cuivre | mg/l | 0.7-1.4 |
| Zn | Zinc | mg/l | 0.7- 1.2 |
| Cu/Zn | Ratio Cuivre/Zinc | | 1.00 - 1.17 |
| Perox.Lipid | Péroxydes lipidiques | μmol/l | 48 - 400 |
| LDL Oxyd | Low Density Lipid Oxydés | U/l | 26 - 117 |
| Atcp /LDL | Anti-corps contre LDL oxydés | U/l | 200 - 600 |
| 8ohdG/ creat | Ratio 8 Hydroxy-2 guanosine / créatinine | μg/g créatinine | 0-20 |
| ADN OX | Acide Désoxyribonucléinque Oxydé | μg/l | 0-26 |

ANNEXE 3

Ecart ✕   Diagnostic A ⊙   Diagnostic B ●   Diagnostic C ⋯⋯ Consensus ── Invention

**Revendications**

1. Procédé de détermination d'un score représentatif du stress oxydatif d'un patient, sur une échelle de 0 à 10, **caractérisé par** la succession des étapes suivantes :

   - dans une étape d'étalonnage initial :
   - on choisit une série de marqueurs biologiques liés au stress oxydatif et on évalue ces marqueurs biologiques par dosage, en particulier à partir de prélèvements sanguins, chez un panel de personnes saines ou présentant éventuellement différents types de pathologies et troubles cliniques connus de façon à obtenir une base de données, et
   - on effectue une analyse statistique de cette base de données en s'appuyant sur des relations connues dans le domaine médical et décrites dans la littérature de façon :
   - à établir pour chacun des marqueurs biologiques une échelle de gravité associée aux écarts constatés par rapport à des valeurs de référence,
   - à rassembler les marqueurs biologiques en au moins trois groupes de marqueurs biologiques exprimant respectivement des informations différentes pouvant être associées à des stades différents de développement du stress oxydatif et correspondant respectivement à des antioxydants exogènes, à des antioxydants endogènes et à des marqueurs de dégâts oxydatifs, et
   - à attribuer un poids d'une part à chaque groupe de marqueurs biologiques, et d'autre part à chacun des marqueurs biologiques au sein de chaque groupe, puis
   - on évalue plusieurs marqueurs biologiques du patient appartenant à plusieurs groupes de marqueurs biologiques,
   - on attribue à chacune des valeurs ainsi obtenues une note sur l'échelle de gravité, et
   - on calcule un score de stress oxydatif Spatient spécifique au patient en utilisant la formule

$$Spatient = 10x \frac{\sum\limits_{groupe1}^{nb.groupe} Poids_{groupe} \; x \left( \sum\limits_{marqueur1}^{nb.marqueurs} \left( Poids_{marqueur} \, x \, note_{patient} \right) \right)}{\sum\limits_{groupe1}^{nb.groupes} Poids_{groupe} \; x \left( \sum\limits_{marqueur1}^{nb.marqueurs} \left( Poids_{marqueur} \; x \; Max_{marqueur} \right) \right)}$$

   dans laquelle $Max_{marqueur}$ représente la note maximum du marqueur biologique dans l'échelle de gravité.

2. Procédé selon la revendication 1, **caractérisé en ce qu'** on attribue au premier groupe de marqueurs biologique correspondant à des antioxydants exogènes un poids égal à 1, au second groupe de marqueurs biologiques correspondant à des antioxydants endogènes un poids étal à 5, et au troisième groupe de marqueurs biologiques correspondant à des marqueurs de dégâts biologiques un poids égal à 10.

3. Procédé selon l'une quelconque des revendications 1 et 2, **caractérisé en ce que** le premier groupe de marqueurs biologiques renferme au moins sept marqueurs biologiques constitués par la vitamine C, la vitamine E, le cuivre, le zinc, le sélénium et les rapports vitamine C/vitamine E et cuivre/zinc.

4. Procédé selon l'une quelconque des revendications 1 à 3, **caractérisé en ce que** le second groupe de marqueurs biologiques renferme au moins quatre marqueurs biologiques constitués par le glutathion total GSH, le glutathion oxydé GSSG, les protéines thiols et le rapport GSH/GSSG.

5. Procédé selon l'une quelconque des revendications 1 à 4, **caractérisé en ce que** le troisième groupe de marqueurs biologiques renferme au moins un marqueur biologique choisi parmi les lipides basse densité oxydés LDL Oxyd. et le rapport 8 hydroxy 2guanosine/créatinine.

6. Procédé selon l'une quelconque des revendications 1 à 5, **caractérisé en ce que** la note d'un marqueur biologique sur l'échelle de gravité peut varier de 1 à 2 à de 1 à 10 selon les marqueurs.

**Claims**

1. Method of determining a score representative of the oxidative stress of a patient, on a scale of 0 to 10, **characterised by** the following successive steps:

   - in an initial calibration step:
   - a series of biological markers related to oxidative stress is selected and these biological markers are evaluated by assay, in particular from blood samples, from a panel of people who are healthy or possibly have different types of known clinical disorders and pathologies so as to obtain a database, and
   - this database is statistically analysed based on relationships known in the medical field and described in the literature so as to:
   - establish for each of the biological markers a gravity scale associated with noted deviations with respect to reference values,
   - group the biological markers into at least three groups of biological markers respectively expressing different information which can be associated with different stages of development of oxidative stress and respectively corresponding to exogenous antioxidants, endogenous antioxidants and oxidative damage markers, and
   - assign a weight on the one hand to each group of biological markers and on the other hand to each of the biological markers within each group, then
   - several biological markers of the patient belonging to several groups of biological markers are evaluated,
   - each of the thus obtained values are assigned a mark on the gravity scale, and
   - an oxidative stress score, Spatient, specific to the patient is calculated using the formula

$$\text{Spatient} = 10 \times \frac{\sum_{\text{group 1}}^{\text{no. of groups}} \text{Weight}_{\text{group}} \times \left( \sum_{\text{marker 1}}^{\text{no. of markers}} \left( \text{Weight}_{\text{marker}} \times \text{mark}_{\text{patient}} \right) \right)}{\sum_{\text{group 1}}^{\text{no. of groups}} \text{Weight}_{\text{group}} \times \left( \sum_{\text{marker 1}}^{\text{no. of markers}} \left( \text{Weight}_{\text{marker}} \times \text{Max}_{\text{marker}} \right) \right)}$$

   wherein $\text{Max}_{\text{marker}}$ represents the maximum mark of the biological marker in the gravity scale.

2. Method as claimed in Claim 1, **characterised in that** the first group of biological markers corresponding to exogenous antioxidants is assigned a weight equal to 1, the second group of biological markers corresponding to endogenous antioxidants is assigned a weight equal to 5, and the third group of biological markers corresponding to biological damage markers is assigned a weight equal to 10.

3. Method as claimed in any one of Claims 1 and 2, **characterised in that** the first group of biological markers contains at least seven biological markers formed by vitamin C, vitamin E, copper, zinc, selenium, the vitamin C/vitamin E ratio and the copper/zinc ratio.

4. Method as claimed in any one of Claims 1 to 3, **characterised in that** the second group of biological markers contains at least four biological markers formed by total glutathione GSH, oxidized glutathione GSSG, thiol proteins and the GSH/GSSG ratio.

5. Method as claimed in any one of Claims 1 to 4, **characterised in that** the third group of biological markers contains at least one biological marker selected from oxidized low density lipids Oxid. LDL and the 8 hydroxy 2 guanosine/creatinine ratio.

6. Method as claimed in any one of Claims 1 to 5, **characterised in that** the mark of a biological marker on the gravity scale can vary from 1 to 2 to from 1 to 10 depending on the markers.


**Patentansprüche**

1. Verfahren zur Bestimmung eines Zahlenwerts, der für den oxidativen Stress eines Patienten steht, auf einer Skala von 0 bis 10, **gekennzeichnet durch** die Abfolge der folgenden Schritte:

- in einem anfänglichen Kalibrierschritt:
- wird eine Reihe von biologischen Markern, die mit dem oxidativen Stress in Verbindung stehen, gewählt, und diese biologischen Marker werden einer Bewertung anhand einer quantitativen Bestimmung, insbesondere in Blutproben, bei einer Prüfgruppe von Personen unterzogen, die gesund sind oder möglicherweise verschiedenartige bekannte Erkrankungen und klinische Störungen aufweisen, um eine Datenbank zu erstellen, und
- es wird eine statistische Analyse dieser Datenbank auf Grundlage von Beziehungen durchgeführt, die im medizinischen Bereich bekannt sind und in der Literatur beschrieben sind, um:
- für jeden der biologischen Marker eine Skala des Schweregrads in Verbindung mit den Abweichungen, die gegenüber Referenzwerten festgestellt wurden, zu erstellen,
- die biologischen Marker zu mindestens drei Gruppen von biologischen Markern zusammenzufassen, welche jeweils verschiedenartige Informationen ausdrücken, die mit verschiedenen Entwicklungsstadien des oxidativen Stresses in Verbindung stehen können und die exogenen Antioxidantien, endogenen Antioxidantien beziehungsweise Markern oxidativer Schäden entsprechen, und
- einerseits jeder Gruppe von biologischen Markern und andererseits jedem der Marker innerhalb jeder Gruppe eine Gewichtung zuzuordnen, woraufhin
- mehrere biologische Marker des Patienten, die mehreren Gruppen von biologischen Markern angehören, ausgewertet werden,
- jedem der Werte, die auf diese Weise erhalten wurden, eine Note auf der Skala des Schweregrads zugeordnet wird, und
- unter Verwendung der folgenden Formel ein Zahlenwert des oxidativen Stresses Spatient, der für den Patienten spezifisch ist, berechnet wird

$$S_{patient} = 10 \times \frac{\sum\limits_{Gruppe\,1}^{Anz.Gruppen} Gewichtung_{Gruppe} \times \left( \sum\limits_{Marker\,1}^{Anz.Marker} \left( Gewichtung_{Marker} \times Note_{Patient} \right) \right)}{\sum\limits_{Gruppe\,1}^{Anz.Gruppen} Gewichtung_{Gruppe} \times \left( \sum\limits_{Marker\,1}^{Anz.Marker} \left( Gewichtung_{Marker} \times Max_{Marker} \right) \right)}$$

wobei $Max_{Marker}$ für die Höchstnote des biologischen Markers auf der Skala des Schweregrads steht.

2. Verfahren nach Anspruch 1,
**dadurch gekennzeichnet, dass** der ersten Gruppe von biologischen Markern, welche den exogenen Antioxidantien entspricht, eine Gewichtung von 1 zugeordnet wird, der zweiten Gruppe von biologischen Markern, welche den endogenen Antioxidantien entspricht, eine Gewichtung von 5, und der dritten Gruppe von biologischen Markern, welche Markern biologischer Schäden entspricht, eine Gewichtung von 10.

3. Verfahren nach einem beliebigen der Ansprüche 1 und 2,
**dadurch gekennzeichnet, dass** die erste Gruppe von biologischen Markern mindestens sieben biologische Marker umfasst, die aus Vitamin C, Vitamin E, Kupfer, Zink, Selen und den Verhältnissen Vitamin C/Nitamin E und Kupfer/Zink bestehen.

4. Verfahren nach einem beliebigen der Ansprüche 1 bis 3,
**dadurch gekennzeichnet, dass** die zweite Gruppe von biologischen Markern mindestens vier biologische Marker umfasst, die aus Gesamt-Glutathion GSH, oxidiertem Glutathion GSSG, Thiolproteinen und dem Verhältnis GSH/GSSG bestehen.

5. Verfahren nach einem beliebigen der Ansprüche 1 bis 4,
**dadurch gekennzeichnet, dass** die dritte Gruppe von biologischen Markern mindestens einen biologischen Marker umfasst, der aus den oxidierten Lipiden geringer Dichte LDL Oxid. und dem Verhältnis 8-Hydroxy-2-guanosin/Kreatinin gewählt ist.

6. Verfahren nach einem beliebigen der Ansprüche 1 bis 5,
**dadurch gekennzeichnet, dass** die Note eines biologischen Markers auf der Skala des Schweregrads in Bandbreiten variieren kann, die je nach Marker von 1 bis 2 bis zu 1 bis 10 betragen.

**EP 1 793 230 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2005020984 A